# EUROPEAN PATENT SPECIFICATION

(11) **EP 4 017 996 B1**
(45) Date of publication and mention of the grant of the patent: **09.10.2024**
(21) Application number: 20856901.2
(22) Date of filing: 21.08.2020
(51) Int. Cl.: C12Q 1/6869, C12Q 1/6806

(54) **SUPPRESSING FALSE POSITIVES (TYPE I ERROR) DURING ANALYSIS OF SAMPLE BIOLOGICAL MATERIALS**
UNTERDRÜCKUNG VON FALSCH-POSITIVEN (TYP-I-FEHLER) WÄHREND DER ANALYSE VON BIOLOGISCHEN PROBEN
SUPPRESSION DE FAUX POSITIFS (ERREUR DE TYPE I) PENDANT L'ANALYSE DE MATÉRIAUX BIOLOGIQUES D'ÉCHANTILLON

(30) Priority: 23.08.2019 US 201962890988 P
(43) Date of publication of application: 29.06.2022
(73) Proprietor: McMaster University, Hamilton, Ontario L8S 4L8 (CA)
(72) Inventor: MCARTHUR, Andrew, Hamilton, Ontario L8P 1R9 (CA); WRIGHT, Gerard, Paris, Ontario N3L 0C6 (CA); POINAR, Hendrik, Lynden, Ontario L0R 1T0 (CA); SURETTE, Michael G., Dundas, Ontario L9H 7R7 (CA); GUITOR, Allison, Kanata, Ontario K2K 3J2 (CA)
(74) Representative: Patentanwälte Bauer Vorberg Kayser
(86) International application number: PCT/CA2020/051142
(87) International publication number: WO 2021/035339

(56) References cited:
- WO-A1-2018/031486
- US-A1- 2015 218 620
- FORSTER MICHAEL ET AL: "Vy-PER: eliminating false positive detection of virus integration events in next generation sequencing data", SCIENTIFIC REPORTS, vol. 5, no. 1, 13 July 2015 (2015-07-13), XP093062091, Retrieved from the Internet <URL:https://www.nature.com/articles/srep11534> DOI: 10.1038/srep11534
- POUNDS STAN ET AL: "Robust estimation of the false discovery rate", BIOINFORMATICS, vol. 22, no. 16, 15 June 2006 (2006-06-15), GB, pages 1979 - 1987, XP093062101, ISSN: 1367-4803, Retrieved from the Internet <URL:https://academic.oup.com/bioinformatics/article-pdf/22/16/1979/48840504/bioinformatics_22_16_1979.pdf> DOI: 10.1093/bioinformatics/btl328
- GUITOR, A. ET AL.: "Capturing the Resistome: a Targeted Capture Method To Reveal Antibiotic Resistance Determinants in Metagenomes", ANTIMICROB AGENTS CHEMOTHER, vol. 64, no. 1, 20 December 2019 (2019-12-20), pages 1 - 18, XP055795510, ISSN: 1098-6596, DOI: 10.1128/AAC.01324-19
- GNIRKE, A. ET AL.: "Solution hybrid selection with ultra-long oligonucleotides for massively parallel targeted sequencing", NAT BIOTECHNOL, vol. 27, no. 2, February 2009 (2009-02-01), pages 182 - 189, XP002658414, ISSN: 1546-1696, DOI: 10.1038/nbt1523
- KUKITA, Y. ET AL.: "High-fidelity target sequencing of individual molecules identified using barcode sequences: de novo detection and absolute quantitation of mutations in plasma cell -free DNA from cancer patients", DNA RES, vol. 22, no. 4, August 2015 (2015-08-01), pages 269 - 277, XP055249604, ISSN: 1756-1663, DOI: 10.1093/dnares/dsv010
- HWANG, K-B. ET AL.: "Reducing false positive incidental findings with ensemble genotyping and logistic regression-based variant filtering methods", HUM MUTAT, vol. 35, no. 8, August 2014 (2014-08-01), pages 936 - 944, XP055795514, ISSN: 1098-1004, DOI: 10.1002/humu.22587

## Description

### TECHNICAL FIELD

The present disclosure relates to analysis of biological samples, and more particularly to suppression of false positives during such analysis.

### BACKGROUND

Antibiotic resistance (AMR) is a crisis that currently impacts human and animal health, involving the clinic, agriculture, and the environment. The World Health Organization along with public health and economic organizations across the globe recognize antibiotic resistance as one of the most pressing challenges of the 21^{st} Century (Laxminarayan *et al.,* 2013). The crisis is the result of two interrelated elements. First, resistance genes are ancient, evolving in concert with the emergence of antibiotic production, presumably hundreds of millions of years ago (Forsberg *et al.,* 2014, Davies and Davies, 2010, Barlow & Hall, 2002, Perry *et al.,* 2016, D'Costa *et al*., 2006, 2011). This challenge is amplified by the facile movement of AMR genes via horizontal gene transfer coupled with the movement of people and goods across the planet, thereby facilitating spread (Levy and Bonnie, 2004; Schwartz & Morris, 2018; Gaze *et al*., 2013). The second is the lack of new antibiotics available to counter the emergence of resistance (Brown & Wright, 2016; Silver, 2011). These two issues conspire to threaten modem medicine and food security. One of the significant gaps to address the antibiotic crisis is a lack of suitable tools to rapidly detect and identify the complete resistome (entire AMR gene contingent), in various environments and associated microbiomes.

Identifying the resistome of individual strains, microbiomes, and environmental settings (sediment, hospitals, etc.) provides critical information on the resistance gene census of a given sample e.g. infected sites, food and water supply, etc. (Surette and Wright, 2017; Allen *et al.,* 2010; Fitzpatrick and Walsh, 2016; Forsberg *et al*., 2012; Luo *et al*., 2013; Pal *et al*., 2016). This information can be used to guide antibiotic use and inform stewardship programs, track the spread and emergence of resistance, monitor the emergence of new resistance alleles associated with the use of antibiotics or other bioactive compounds, and enable molecular surveillance for public health decision making. Importantly, this strategy is highly scalable from the individual, to her/his local environments (i.e. hospital ward, barn, etc.) and even larger geographic regions (Van Schaik, 2014; Buelow *et al*., 2014; Allen *et al*., 2009; Lax and Gilbert, 2015; Nesme *et al*., 2014).

Profiling the resistomes of bacterial strains that are culturable is reasonably straightforward using whole genome sequencing or direct detection of selected genes, e.g. via polymerase chain reaction (PCR) or microarrays (Walsh and Duffy 2013; Mezger *et al.,* 2015; Zumla *et al*., 2014; Pulido *et al*., 2013). These latter strategies can also be applied to metagenomes, as was showed to be possible through the identification of resistance genes for tetracycline, penicillin, and glycopeptide antibiotics in 30,000-year old Beringian permafrost (D'Costa *et al*., 2011). A weakness of highly targeted or PCR based approaches is that they are rarely comprehensive despite the number of known resistance elements, let alone the continual emergence of variants and/or completely novel mechanisms (Boolchandani *et al.,* 2017, Boolchandani *et al.,* 2019; Crofts *et al.,* 2017). Furthermore, non-targeted resistome survey methods in metagenomes require millions of sequencing reads, or deep sequencing, and careful filtering, recognizing that the vast majority of sequences will not encode antibiotic resistance determinants (Boolchandani *et al*., 2019; Rowe and Winn, 2018).

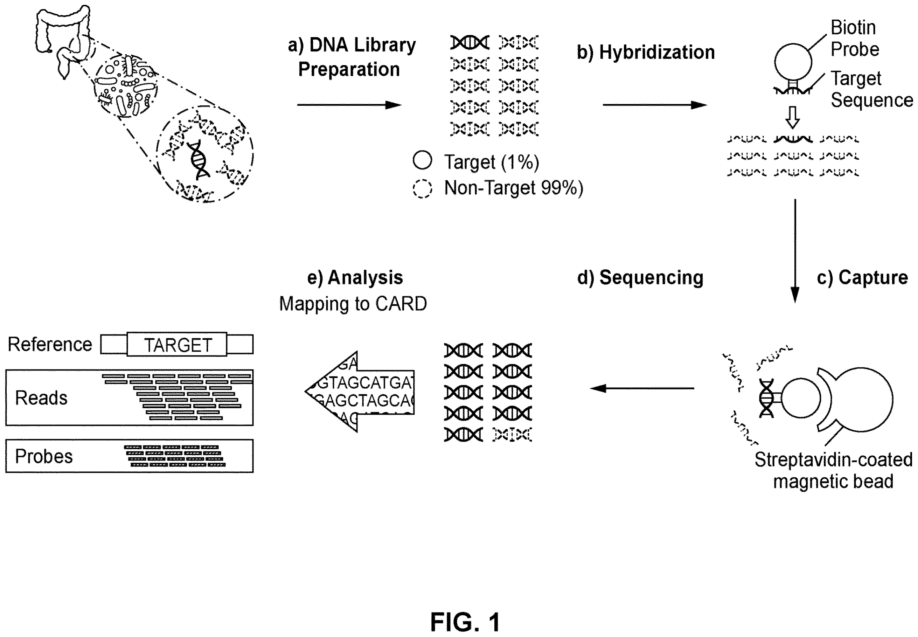

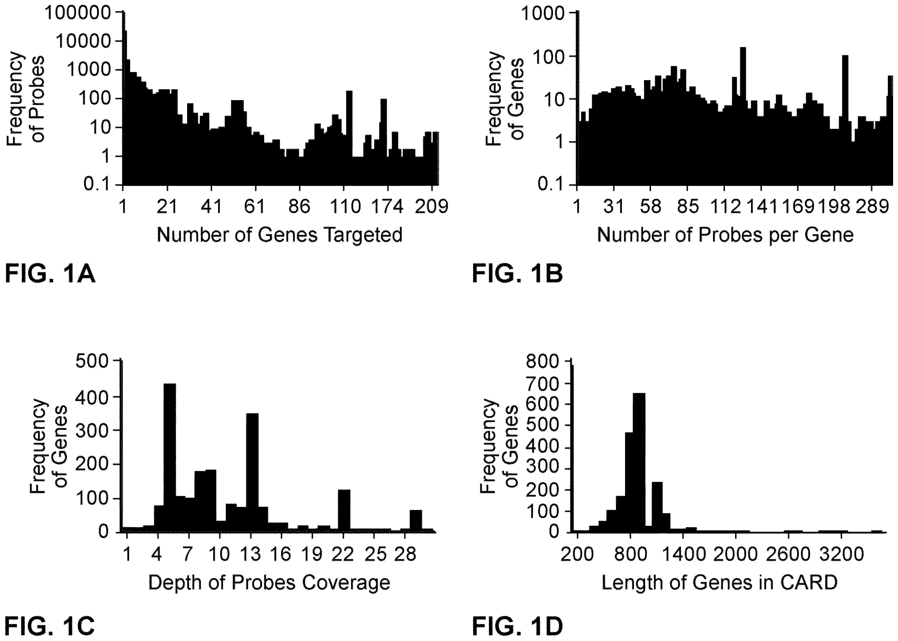

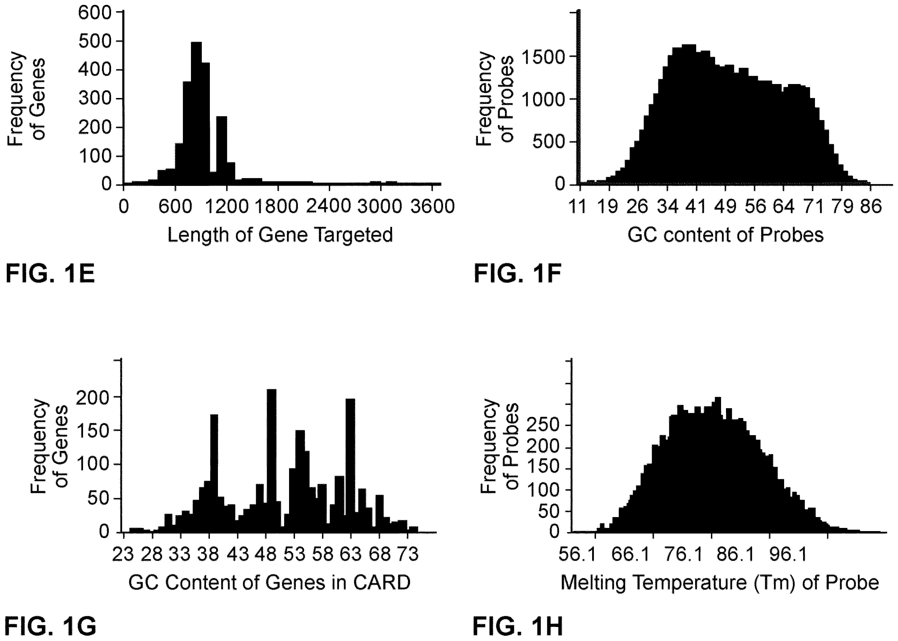

A more appropriate approach for the identification of resistomes is the use of a probe and capture strategy (Gnirke *et al*., 2009), as such methods have seen great success in enriching for targeted sequences in highly complex metagenomes. For example, this approach has been used to capture, sequence, and reconstruct human mitochondrial sequences as well as the genomes of infectious agents and extinct species from various environments including highly degraded archeological and historical samples (Wagner *et al*., 2014; Patterson Ross et al., 2018; Duggan *et al*., 2016; Devault *et al*., 2017; Enk *et al*., 2014; Depledge *et al*., 2011). In a probe and capture experiment, target RNA 'baits' are designed to be complementary (to at least 85% identity), to target DNA sequences of interest. Actual synthesized baits are biotin-labelled and are incubated with the DNA from metagenomic or genomic libraries, where they hybridize to related sequences, as shown in Figure 1. The targeted capture sequencing workflow begins with DNA isolation from a sample of interest (stool from a healthy donor in this example). In Figure 1, at step (a) DNA is fragmented through sonication and prepared as a sequencing library, and at steps (b) and (c) target sequences representing less than 1% of the total DNA are and captured through hybridization with biotinylated probes and streptavidin-coated magnetic beads. At steps (d) and (e) the purified and amplified capture library fragments are sequenced and analysed for AMR sequence content by mapping to the Comprehensive Antibiotic Resistance Database (CARD). CARD is a curated collection of characterized, peer-reviewed resistance determinants and associated antibiotics, and provides data, models, and algorithms relating to the molecular basis of antimicrobial resistance. The CARD provides curated reference sequences and SNPs organized by the Antibiotic Resistance Ontology (ARO) and AMR gene detection models. Information about CARD is available online at https://card.mcmaster.ca/. Ontologies at CARD are available on the CARD website. These data are additionally associated with detection models, in the form of curated homology cut-offs and SNP maps, for prediction of resistome from molecular sequences. These models can be downloaded or can be used for analysis of genome sequences using the Resistance Gene Identifier ("RGI") for prediction of complete resistome from genomic and metagenomic data, either online or as a stand-alone tool. All data and software associated with CARD is protected by copyright; CARD is available to academic and government users and requires licenses for commercial use; details are available at https://card.mcmaster.ca/about. For the avoidance of doubt, this patent application, and any patents to issue herefrom, do not grant any license in respect of CARD in whole or in part.

Targets are captured using streptavidin-coated magnetic bead separation, reactions pooled and sequenced on a next-generation sequencing (NGS) platform. This strategy offers excellent advantages for the sampling of resistomes in a variety of environments where resistance genes are generally rare and genetically diverse. Indeed, recently this approach has been explored for resistance gene capture by other groups (Lanza *et al*., 2018, Noyes *et al*., 2017, Allicock *et al*., 2018). However, these approaches target many other genes that are not rigorously associated with resistance, increasing the cost and the opportunity for false positive gene identification.

Forster, M. et al., Scientific Reports (2015), 5:11534 discloses a method eliminating false positive data on viral integrants as determined by paired-end sequencing.

Thus, the increasing sensitivity and lower cost of DNA sequencing holds promise for identifying AMR components at the genome level to allow precision medical and/or environmental intervention. However, this same increased sensitivity raises the risk of false positives, which may not only result in wasted effort to treat a non-existent problem, but also makes it worse. For example, a false positive identification of an AMR component may result in the unnecessary deployment of one of the limited number of antibiotics held "in reserve" because it is known to be effective against AMR. Such deployment can needlessly expose microbes to these "reserve" drugs, allowing them to develop resistance. Thus, the reduction of false positives when detecting AMR components is a crucial aspect of antibiotic stewardship.

### SUMMARY

In one aspect, the present disclosure is directed to a method for suppressing false positives (Type I Error) during analysis of sample biological materials. The method comprises, for each of at least one handling step during the analysis, obtaining at least one sample handling blank carrying a transfer substrate mixed with at least part of the sample biological materials, obtaining at least one control blank that is isolated from the sample biological materials and corresponding to the sample handling blank in that handling step, and replicating the handling applied to the at least one sample handling blank for the at least one control blank. Following completion of all handling steps, there is at least one final sample handling blank carrying the transfer substrates from the handling steps mixed with the at least part of the sample biological materials, and at least one final control blank carrying the transfer substrates from the handling steps and isolated from the sample biological materials. The method further comprises applying a hybridization probe solution containing at least one hybridization probe to each final sample handling blank to produce at least one baited final sample handling blank, and applying to each final control blank hybridization probe solution identical to that applied to each final sample handling blank to produce at least one baited final control blank. The method further comprises feeding each baited final sample handling blank into a DNA sequencer and sequencing sample bait-captured DNA carried by the baited final sample handling blank, and feeding each baited final control blank into the DNA sequencer and sequencing control bait-captured DNA carried by the baited final control blank. The method still further comprises comparing the sample bait-captured DNA to the control bait-captured DNA and discounting, from a final identified genetic sequence, genetic components that are common to the final sample handling blank and the final control blank and pass a statistical significance test.

The at least one handling step may comprise a plurality of handling steps including a collection step during which the sample biological materials are collected and at least one transfer step where the sample biological materials are transferred from a preceding sample handling blank to a subsequent sample handling blank.

The sample biological materials may be from a vertebrate, and may include at least one of blood, urine, feces, tissue, lymph fluid, spinal fluid and sputum.

The sample biological materials may be from at least one of a living organism, a cadaver of a formerly living organism, and an archaeological sample.

The sample biological materials may be from an invertebrate.

The sample biological materials may be from at least one environmental sample, which may comprise at least one of mud, soil, water, effluent, filter deposits and surface films.

In another aspect, the present disclosure is directed to a method for suppressing false positives (Type I Error) during analysis of sample biological materials. The method comprises, for at least one final sample handling blank carrying transfer substrate mixed with at least part of the sample biological materials, applying a hybridization probe solution containing at least one hybridization probe to each final sample handling blank to produce at least one baited final sample handling blank, and applying hybridization probe solution identical to that applied to each final sample handling blank to at least one final control blank, wherein the at least one final control blank carries transfer substrate identical to that applied to each sample handling blank and the at least one final control blank is isolated from the sample biological materials, to thereby produce at least one baited final control blank. The method further comprises feeding each baited final sample handling blank into a DNA sequencer and sequencing sample bait-captured DNA carried by the baited final sample handling blank, and feeding each baited final control blank into the DNA sequencer and sequencing control bait-captured DNA carried by the baited final control blank. The method still further comprises comparing the sample bait-captured DNA to the control bait-captured DNA and discounting, from a final identified genetic sequence, genetic components that are common to the final sample handling blank and the final control blank and pass a statistical significance test.

The sample biological materials may be from a vertebrate, and may include at least one of blood, urine, feces, tissue, lymph fluid, spinal fluid and sputum.

The sample biological materials may be from at least one of a living organism, a cadaver of a formerly living organism, and an archaeological sample.

The sample biological materials may be from an invertebrate.

The sample biological materials may be from at least one environmental sample, which may comprise at least one of mud, soil, water, effluent, filter deposits and surface films.

### BRIEF DESCRIPTION OF THE DRAWINGS

These and other features will become more apparent from the following description in which reference is made to the appended drawings wherein:
FIGURE 1 shows a process for rapid capture and identification of diverse antibiotic resistance genes;
FIGURE 1A shows a number of genes targeted by probes through mapping with Bowtie2;
FIGURE 1B shows a number of probes targeting genes through mapping with Bowtie2;
FIGURE 1C shows mean depth of probe coverage across individual genes in CARD;
FIGURE 1D shows length of genes in CARD;
FIGURE 1E shows length of sequence targeted by probes in genes in CARD;
FIGURE 1F shows GC content of probes;
FIGURE 1G shows GC content of genes in CARD;
FIGURE 1H shows melt temperature of final list of probes.
FIGURE 2 shows statistics for a platform for rapid capture and identification of diverse antibiotic resistance genes, including (A) an example of the process of designing probes against an antibiotic resistance gene (ndm-1), (B) a percent length coverage of genes with probes, and (C) a breakdown of resistance gene classes from CARD that are targeted by probes;
FIGURES 2A to 2D show comparative read counts normalized in subsampled individual enrichment trials through different library preparation methods;
FIGURE 3 compares enriched to shotgun results for percentage on target, percent recovery and percent coverage;
FIGURES 3A and 3B show read counts at each probe-targeted region within the Escherichia coli C0002 genome and Staphylococcus aureus C0018 genome in enriched and shotgun samples (reads were subsampled to the same sequencing depth among samples);
FIGURE 4 shows normalized read counts (reads per length (kb) of target per million reads sequenced) at each probe-targeted region within the Escherichia coli C0002 genome (part A) and Staphylococcus aureus C0018 genome (part B) in enriched and shotgun samples including individual and "mock metagenomes" of multiple strains;
FIGURES 4A, 4B and 4C show normalized read counts from C0002 control enrichments from three samples in each set to the two trials of individual enrichment;
FIGURE 5 shows normalized read counts in each 6 enriched libraries compared to their shotgun pairs;
FIGURES 5A, 5B and 5C compare enriched and shotgun ARG recovery;
FIGURE 6 shows hierarchical clustering of enriched libraries;
FIGURE 7 shows hierarchical clustering of enriched and shotgun libraries;
FIGURE 8 shows rarefaction curves for identification of antibiotic resistance genes; and
FIGURE 9 shows an illustrative method for suppressing false positives during analysis of sample biological materials in pictorial form.

### DETAILED DESCRIPTION

The present disclosure describes a targeted method for the analysis of antibiotic resistomes. The efficacy of this probeset and strategy are tested using both a panel of previously sequenced pathogenic bacteria with known resistance genotypes and phenotypes, as well as previously uncharacterized human metagenomic stool samples. The method is readily applicable to both clinical and non-clinical settings.

The probeset used herein was based on stringently curated AMR gene (ARG) sequences from the Comprehensive Antibiotic Resistance Database (CARD), tiled at four-fold coverage across ARG sequences, combined with rigorous bioinformatic analysis to suppress off-target hybridization, enabling a cost-effective and sensitive method to sample the known resistance gene landscape (Jia *et al*., 2017).

### Results

### Design and characterization of resistance gene probes

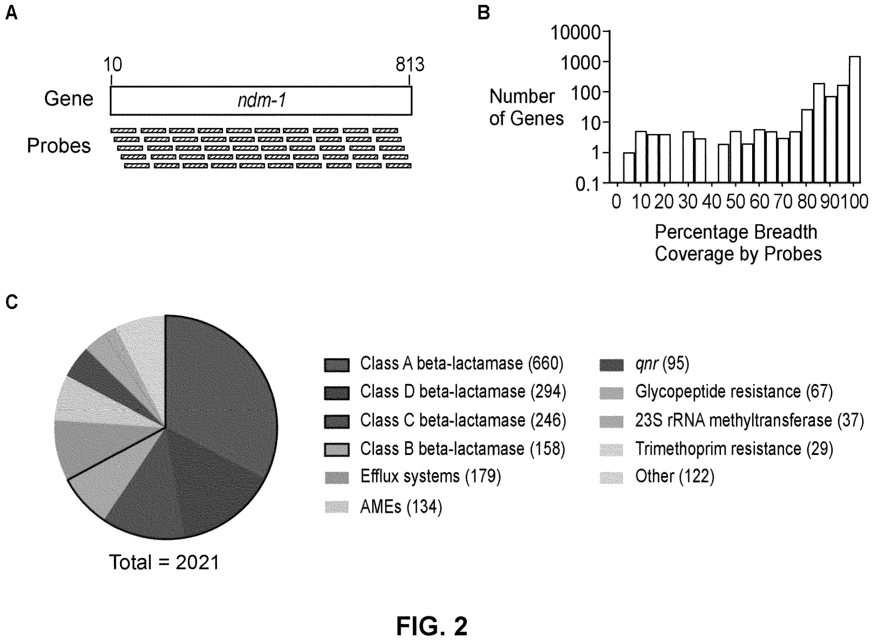

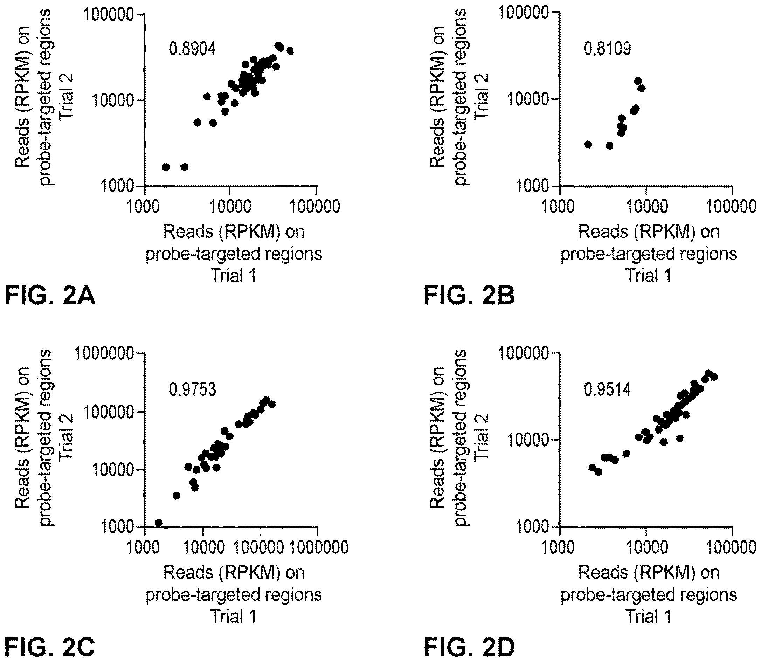

A set of 80-mer nucleotide probes were custom designed and synthesized through the myBaits platform (Arbor Biosciences, Ann Arbor, Michigan). The probes span the protein homolog model of curated ARGs from CARD and represent nucleotide sequences (2021) that are well-characterized in the literature as resistance-conferring. Many of the probes are highly specific to individual genes (100% nucleotide identity to reference ARG sequence) as shown in part (A) of Figure 2, but partial hybridization can allow for probes to target sequences that are divergent from the reference sequence. Part (A) of Figure 2 shows an example of the process of designing probes against an antibiotic resistance gene (ndm-1). In the example, probes are 80 nucleotides each and tiled at a 20-nucleotide sliding window. Resistance conferred through mutation (protein variant model in CARD) to genes encoding highly conserved proteins (including gyrA and 16S rRNA sequences) was purposefully not included in the design.

With 37,826 probes, this probeset is capable of targeting 2021 nucleotide sequences implicated in resistance across all classes of antibiotics and a wide range of resistance gene families (see part (C) Figure 2). The majority (78.03%) of genes targeted by probes mirror the breakdown in CARD, dominated by antibiotic inactivation mechanisms and by the beta-lactamase proteins, reflecting their use in the clinic (part (C) of Figure 2). The next largest category of resistance elements targeted by the probeset are efflux pumps. The majority of the probes (24,767) target a single gene and the remainder range to a maximum of 211 genes (average 5.96 genes) due to sequence conservation within gene families (see Figure 1A). For example, a single probe initially designed to target 80 nucleotides of the beta-lactamase gene *bla*_{SHV-52} is predicted to also target an additional 208 genes including other members of the SHV, LEN, and OKP-A/-B beta-lactamases due to homology between these gene sequences. Thus, in some cases there is overlap in the utility of some 80-mer probes. In addition to many beta-lactamase families, aminoglycoside-modifying enzymes (AAC(3) and AAC(6')) and quinolone resistance *qnr* genes are large families with probes designed to target upwards of 10 genes each. Remarkably, 2004 of the 2021 targeted genes (99.16%) are covered by at least 10 or more probes (see Figure 1B).

At the individual determinant level, the number of probes per gene (average 105 probes per gene, range = 1 - 309) and length coverage of a gene (average 96.20% with a range of 3.17% to 100%) varies (Figure 1B, part (B) of Figure 2). The majority of genes (1970/2021) have greater than 80% length coverage by probes (part (B) of Figure 2). Members of the beta-lactamase families (*bla*_{CTX-M}, *bla*_{TEM}, *bla*_{OXA}, *bla*_{GES}*, bla*_{S*HV*}) are among the genes with the highest probe coverage, not surprising given their preponderance in the dataset and their homology within families. 52.6% of targeted gene sequences (1063) have full-length coverage (100%) with an average depth of probe coverage of a gene of 9.47x (minimum 0.05x; maximum 28.83x) (part (B) of Figure 2; Figure 1C). Only 28 sequences from CARD have no probe coverage due to filtering of candidate probes during the design. The average length of a targeted gene in CARD is 917 bp, and the average length of all genes targeted by probes is 876 bp (see Figure 1D and Figure 1E). Overall this probeset targets ~1.77 megabases of antibiotic resistance nucleotide sequence and greater than 83% of the nucleotide sequences curated in CARD. Additional metrics assessed included the guanosine and cytosine content of probes (average 49.96% GC; range: 11 - 94%) and target genes (average: 50.98% GC; range: 23% to 77%), as well as the probe melting temperature (average: 79.62 ° C) (see Figure 1F, Figure 1G and Figure 1H). Probe design in conjunction with verification with Arbor Biosciences encouraged compatibility in the probeset and promotes efficient capture.

### ARG enrichment from bacterial genomes with a range of antibiotic resistance determinants

To characterize the sensitivity and selectivity of this probeset, a series of control experiments was conducted using a panel of previously sequenced, assembled and annotated multi-drug resistant Gram-positive and Gram-negative bacteria isolated within the Hamilton Health Sciences Network. The proportion of the genomes targeted by the probeset as determined by mapping the entire probe contingent to each genome individually ranged from 0.21 - 0.97% shown in Supplementary Table 1.

**Supplementary Table 1: Bacterial strains used in control experiments.**

| Bacterial strain | Genome size (Mb) | GC Content (%) | Predicted genes by RGI | Region predicted by RGI (%) | # Probe-targeted sites | Length of probe-targeted site (average and range) | Region with probe coverag e (%) | RGI genes with probes | Region predicted by RGI and targeted by probes (%) |
|---|---|---|---|---|---|---|---|---|---|
| *Escherichia coli* C0002 | 5.29 | 50.62 | 67 | 1.64 | 65 | 797.75 (80 - 3595) | 0.97 | 43 | 0.81 |
| *Klebsiella pneumoniae* C0006 | 5.45 | 57.23 | 30 | 0.55 | 35 | 331.54 (80 - 877) | 0.21 | 17 | 0.17 |
| *Staphylococcus aureus* C0018 | 2.92 | 32.66 | 16 | 0.55 | 13 | 112754 (140-2013) | 0.50 | 12 | 0.41 |
| *Staphylococcus aureus* C0033 | 2.92 | 32.77 | 16 | 0.64 | 14 | 1143.07 (155 - 2130) | 0.52 | 13 | 0.44 |
| *Klebsiella pneumoniae* C0050 | 5.60 | 57.05 | 34 | 0.63 | 40 | 346.18 (80 - 900) | 0.25 | 18 | 0.19 |
| *Pseudomonas aeruginosa* C0060 | 6.80 | 66.19 | 53 | 1.18 | 48 | 933.35 (97 - 3415) | 0.66 | 33 | 0.54 |
| *Escherichia coli* C0094 | 5.22 | 50.74 | 67 | 1.65 | 64 | 779.86 (80-3003) | 0.95 | 41 | 0.79 |
| *Pseudomonas aeruginosa* C0292 | 6.81 | 66.21 | 54 | 1.17 | 48 | 938.71 (97-3415) | 0.66 | 33 | 0.57 |

Clinical bacterial isolates obtained through the Wright Clinical Collection. Bacterial genomes were sequenced, and draft genome assemblies were analyzed through the Resistance Gene Identifier in CARD to predict the number of resistance genes. The total probeset was mapped against the draft assembled genome and the number of genes with probe coverage, percentage of genome covered by probes and overlap between predicted RGI genes and probe coverage were determined.

ARGs probe-to-target regions were predicted by passing draft genome assemblies through the Resistance Gene Identifier (RGI) in CARD. Strains were predicted to have between 16 and 67 ARGs of which between 13 and 65 were targeted by probes, representing 102 unique genes among the strains tested (Supplementary Table 1). Genomic DNA from four different strains was tested individually via enrichment on two different library preparations; these are referred to as Trial 1 and Trial 2 hereafter. Over 90% of reads mapped to the respective draft bacterial genomes after removing those with low mapping quality scores, as shown in Supplementary Table 2.

**Supplementary Table 2: Individual strain enrichment results.**

| Strain | Average % mapping to genome | Average % mapping to probe-targeted sites | % of RGI & targeted regions with reads | Average % coverage of RGI & targeted regions | Average reads per kb per million reads on probe-targeted region | Average depth per kb per million reads on probe-targeted region |
|---|---|---|---|---|---|---|
| *Escherichia coli* C0002 | 96.67 (±2.72) | 95.07 (±1.54) | 100 | 100 | 18975.73 (±414.91) | 6192.13 (±297.27) |
| *Staphylococcus aureus* C0018 | 97.99 (±1.98) | 94.89 (±2.31) | 100 | 100 | 67615.06 (±4360.20) | 19968.28 (±2670.37) |
| *Klebsiella pneumoniae* C0050 | 95.60 (±3.96) | 85.74 (±4.68) | 100 | 100 | 40531.43 (±2516.77) | 17315.24 (±1630.66) |
| *Pseudomonas aeruginosa* C0060 | 91.45 (±5.49) | 90.73 (±0.95) | 100 | 100 | 22725.67 (±32.97) | 6497.48 (±61.46) |

Strains were enriched individually in two trials with different library sizes. For each strain the regions predicted to be targeted by probes were determined through mapping the probeset to each individual genome). Enrichment results across two trials were determined by mapping trimmed and filtered reads to genome, calculating the percentage on-target and normalizing reads and depth per kb per million reads.

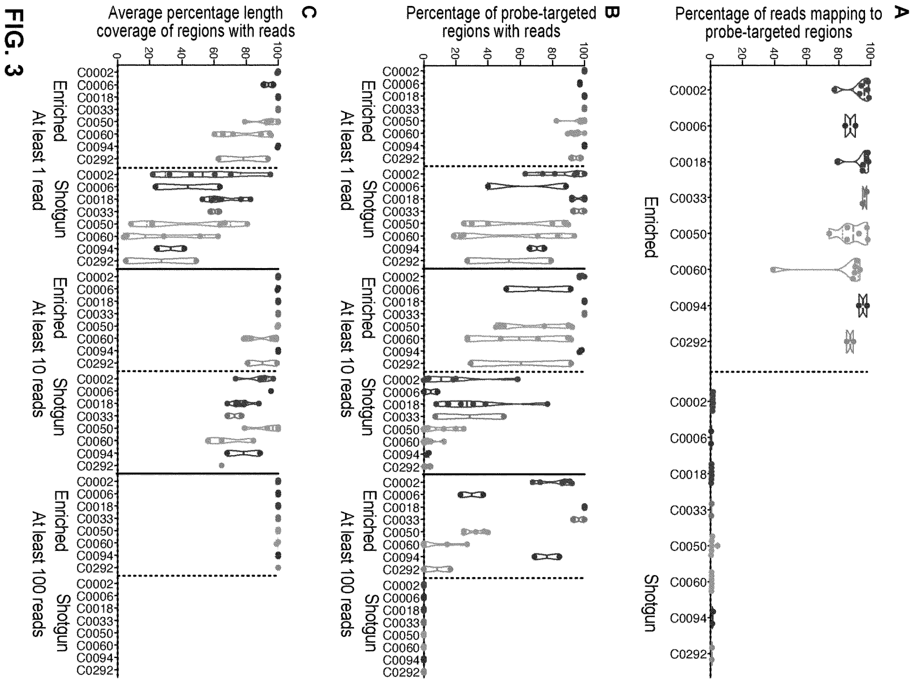

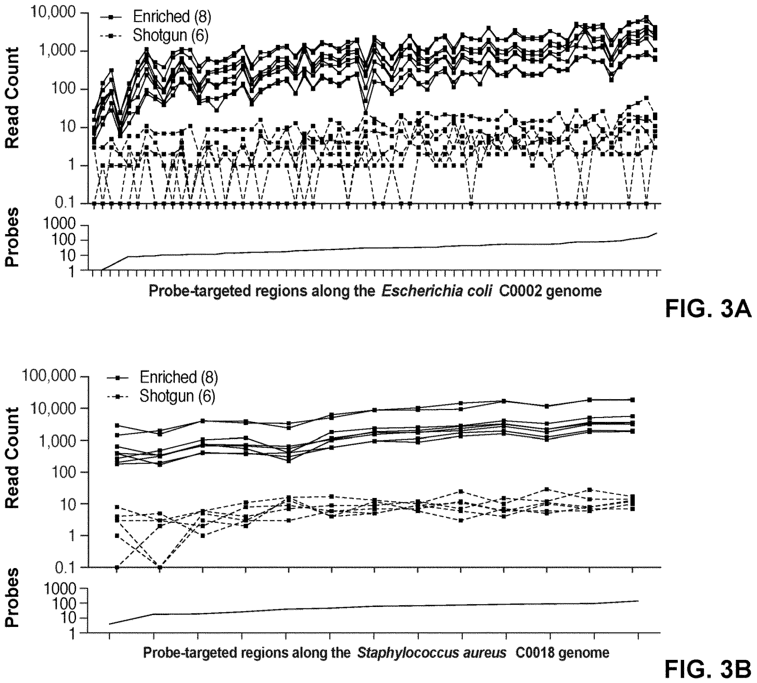

Furthermore, the majority (higher than 85% in all cases) of reads mapped to the small proportion (<1%) of the genome that was predicted to be targeted by the probeset (Supplementary Table 2); part (A) of Figure 3 shows the percentage of reads on target for each strain tested in various sample types (either individual or pooled) for both enriched and shotgun samples. In Figure 3, each point on the graph represents a replicate experiment either as a genome that was enriched individually or when pooled with other genomes (Pool 1, 2 and 3) across both trials. The horizontal line for each strain represents the mean.

### Reproducibility between library preparation methods and controls

This enrichment approach is insensitive and tractable to different library preparation methods (NEBNext Ultra II versus modified Meyer and Kircher) and varying library insert sizes (average library fragment sizes range from 396 to 1257) as shown in Supplementary Table 3 (see also Meyer and Kircher, 2010).

**Supplementary Table 3: Library and sequencing information.**

| Phase | Trial/ Set | Library | Amount of Probes (ng) | Amount of Library (ng) | Average Library Size (bp) | Clusters sequenced enriched | Clusters sequenced shotgun |
|---|---|---|---|---|---|---|---|
| Phase 1 | Trial 1 | C0002 | 100 | 100 | 988 | 66926 | |
| | | C0018 | 100 | 100 | 994 | 75860 | |
| | | C0050 | 100 | 100 | 1222 | 73941 | |
| | | C0060 | 100 | 100 | 1225 | 81810 | |
| | | Pool 1 | 100 | 100 | 1257 | 61568 | 218008 |
| | | Pool 2 | 100 | 100 | 1158 | 61658 | 159059 |
| | | Pool 3 | 100 | 100 | 1216 | 58308 | 109194 |
| | | Negative | 100 | N/A | 632 | 170565 | |
| | | Control - Blank | | | | | |
| | Trial 2 | C0002 | 100 | 100 | 435 | 99748 | |
| | | C0018 | 100 | 100 | 438 | 143804 | |
| | | C0050 | 100 | 100 | 416 | 153673 | |
| | | C0060 | 100 | 100 | 403 | 124971 | |
| | | Pool 1 | 100 | 100 | 429 | 86023 | 29241 |
| | | Pool 2 | 100 | 100 | 413 | 124170 | 33488 |
| | | Pool 3 | 100 | 100 | 427 | 127682 | 32560 |
| | | Negative | 100 | N/A | 345 | 44026 | |
| | | Control - Blank | | | | | |
| Phase 2 | Set 1 | 1 - 1 | 25 | 50 | 952 | 89768 | |
| | | 1-2 | 50 | 50 | 968 | 77117 | |
| | | 1 -3 | 100 | 50 | 919 | 65746 | |
| | | 1-4 | 50 | 100 | 1044 | 55783 | |
| | | 1 - 5 | 100 | 100 | 972 | 64761 | |
| | | 1 -6 | 200 | 100 | 940 | 71099 | 3652948 |
| | | 1 -7 | 100 | 200 | 915 | 15211 | 4405779 |
| | | 1 -8 | 200 | 200 | 1020 | 59409 | |
| | | 1 -9 | 400 | 200 | 998 | 25911 | |
| | | Negative | 50 | N/A | 276 | 2590 | |
| | | Control - Blank | | | | | |
| | Positive Controls | C0002 - 1 - 1 | 100 | 50 | 986 | 80647 | |
| | | C0002 - 1 - 2 | 50 | 50 | 939 | 116965 | |
| | | C0002 - 1 - 3 | 25 | 50 | 976 | 112881 | |
| | Set 2 | 2-1 | 25 | 50 | 955 | 158710 | |
| | | 2-2 | 50 | 50 | 887 | 100590 | |
| | | 2-3 | 100 | 50 | 891 | 102689 | |
| | | 2-4 | 50 | 100 | 902 | 120764 | |
| | | 2-5 | 100 | 100 | 956 | 141994 | 6151998 |
| | | 2-6 | 200 | 100 | 941 | 159192 | |
| | | 2-7 | 100 | 200 | 790 | 96211 | |
| | | 2-8 | 200 | 200 | 944 | 129333 | |
| | | 2-9 | 400 | 200 | 871 | 76195 | 7660355 |
| | | Negative | 50 | N/A | N/A | 3804 | |
| | | Control - Blank | | | | | |
| | Positive Controls | C0002 - 2 -1 | 100 | 33 | 993 | 139909 | |
| | | C0002 - 2 - 2 | 50 | 50 | 935 | 235429 | |
| | | C0002 - 2 - 3 | 25 | 50 | 876 | 129070 | |
| | Set 3 | 3 - 1 | 25 | 50 | 854 | 82778 | 5866495 |
| | | 3-2 | 50 | 50 | 888 | 158968 | |
| | | 3-3 | 100 | 50 | 910 | 65675 | |
| | | 3-4 | 50 | 100 | 889 | 103671 | |
| | | 3-5 | 100 | 100 | 882 | 78251 | 4213540 |
| | | 3-6 | 200 | 100 | 943 | 68331 | |
| | | 3-7 | 100 | 200 | 820 | 96722 | |
| | | 3-8 | 200 | 200 | 934 | 79036 | |
| | | 3-9 | 400 | 200 | 917 | 82375 | |
| | | Negative | 50 | N/A | N/A | 5962 | |
| | | Control - Blank | | | | | |
| | Positive Controls | C0002 - 3 -1 | 100 | 38 | 846 | 54117 | |
| | | C0002 - 3 - 2 | 50 | 32 | 881 | 96258 | |
| | | C0002 - 3 - 3 | 25 | 38 | 779 | 110746 | |

The amount in nanograms of each library and the corresponding amount of probes used for enrichment. The average size of library fragments prior to enrichment was determined through the Agilent Bioanalyzer 2100. The number of clusters (paired-end reads) that were generated for each library when sequenced by Illumina's MiSeq V2 2x250. Blanks for each trial were included and sequenced on a separate run; many of the blank libraries did not generate peaks on the Bioanalyzer nor any signal by quantitative PCR therefore their values are N/A. In Phase 2, three positive controls for enrichment were included with genomic DNA from *Escherichia coli* C0002 and varying library and probe amounts.

After subsampling reads between trials to equal depth to account for differences in sequencing between enriched libraries, there is a strong correlation between read count and read depth on targeted regions for bacterial strains enriched individually (Supplementary Table 2). For all four strains across the two Trials and different library prep methods, the correlation between read counts mapping to probe-targeted regions is high (Pearson correlation 0.8109 - 0.9753) (Figures 2A to 2D). For Figures 2A to 2D, reads from enrichment of individual genomes of *Escherichia coli* C0002 (A), *Staphylococcus aureus* C0018 (B), *Klebsiella pneumonia* C0050 (C) and *Pseudomonas aeruginosa* C0060 (D) in Trial 2 were subsampled to same depth as reads in Trial 1. The reads were mapped to the respective bacterial genome, filtered for mapping quality and then the number of reads on each RGI and probe-targeted region were counted and normalized per kb per million reads. Pearson correlation coefficients are shown. In all cases, the length percent coverage of a gene by reads is 100% (Supplementary Table 2). Finally, the Pearson correlation for average read depth on probe-targeted regions between the two trials ranges from 0.8959 to 0.9740 for the four strains (results not shown).

### Successful enrichment of ARGs in mock metagenomes

The outcome was successful capture of the majority (>80%) of antibiotic resistance genes targeted by the probeset from single-sourced bacterial genome libraries with at least 10 reads. When genomic DNA from multiple bacterial strains was pooled at varying ratios of 4 and/or 8 strains, with some strains representing less than 10% of the total `mock' metagenome, there were recovered significantly more targeted genes with at least 1, 10 or 100 reads mapping (mapping quality >=41 and length >=40) compared to shotgun sequencing (part (B) of Figure 3; Supplementary Table 4; Supplementary Table 5). Part (B) of Figure 3 shows the percent recovery of regions predicted to be targeted by probes for each strain tested in various sample types in both enriched and shotgun samples (1 versus 10 versus 100 reads per probe-targeted region).

**Supplementary Table 4: Pooling of genomic DNA to create "mock metagenomes"**

| Pool | Strain | Amount of genomic DNA pooled (ng) | Estimated % of pool | % of reads mapping from shotgun | % of reads mapping from enriched |
|---|---|---|---|---|---|
| | C0002 | 312 | 21.98 | 24.82 | 52.55 |
| Trial 1 | C0018 | 312 | 40.00 | 12.06 | 32.12 |
| Pool 1 | C0050 | 312 | 20.74 | 27.18 | 8.86 |
| | C0060 | 312 | 17.28 | 35.93 | 6.47 |
| | C0002 | 112 | 18.77 | 22.30 | 33.95 |
| Trial 2 | C0018 | 174 | 53.01 | 65.29 | 62.88 |
| Pool 1 | C0050 | 106 | 16.79 | 4.39 | 1.54 |
| | C0060 | 88 | 11.43 | 8.02 | 1.63 |
| Trial 1 | C0002 | 1250 | 66.30 | 64.73 | 71.26 |
| Pool 2 | C0018 | 180 | 17.22 | 11.96 | 19.69 |
| | C0050 | 180 | 9.07 | 11.28 | 4.75 |
| | C0060 | 180 | 7.41 | 12.03 | 4.30 |
| | C0002 | 264 | 48.04 | 57.31 | 65.39 |
| Trial 2 | C0018 | 102 | 33.92 | 35.54 | 33.24 |
| Pool 2 | C0050 | 62 | 10.75 | 1.66 | 0.44 |
| | C0060 | 51 | 7.29 | 5.49 | 0.94 |
| | C0002 | 125 | 11.01 | 13.91 | 38.50 |
| | C0006 | 125 | 10.70 | 24.75 | 2.34 |
| | C0018 | 125 | 19.88 | 6.54 | 11.62 |
| Trial 1 | C0033 | 125 | 19.88 | 11.59 | 22.81 |
| Pool 3 | C0050 | 125 | 10.40 | 12.75 | 2.73 |
| | C0060 | 125 | 8.56 | 16.40 | 2.16 |
| | C0094 | 125 | 11.01 | 6.90 | 18.78 |
| | C0292 | 125 | 8.56 | 7.15 | 1.07 |
| | C0002 | 46 | 8.65 | 9.84 | 14.80 |
| | C0006 | 83 | 8.16 | 14.44 | 1.53 |
| | C0018 | 43 | 28.17 | 11.49 | 12.49 |
| Trial 2 | C0033 | 36 | 28.15 | 34.36 | 34.58 |
| Pool 3 | C0050 | 45 | 7.68 | 0.60 | 0.13 |
| | C0060 | 83 | 5.20 | 2.02 | 0.42 |
| | C0094 | 46 | 8.78 | 25.21 | 35.67 |
| | C0292 | 36 | 5.21 | 2.04 | 0.39 |

We pooled various nanogram amounts of genomic DNA from bacteria and estimated the percentage of each strain in the respective pools based on total genome size of each strain. With reads generated through shotgun sequencing and after enrichment, we calculated the percentage of reads mapping to a particular genome by mapping to a combined reference of the genomes used in a given pool and counting the reads that mapped to each respective genome (= reads mapping to genome A / reads mapping to all genomes).

**Supplementary Table 5: Enrichment results to probe-targeted regions in pooled samples**

| Sample | Strain | %of reads in Pool | % Mapping to probe-targeted regions | %of probe-targeted regions with reads | % coverage of probe-targeted regions | Average reads per kb per million reads | Average depth per kb per million reads | Fold-enrichment in reads (average and range) |
|---|---|---|---|---|---|---|---|---|
| Trial 1 Pool 1 Enriched | C0002 | 52.75 | 93.06 | 100 | 100 | 19097.95 | 6091.42 | 810.18 (2.66 - 16590.95) |
| | C0018 | 20.05 | 94.84 | 100 | 100 | 67393.09 | 19715.42 | 135.84 (31.11 - 291.78) |
| | C0050 | 18.73 | 85.44 | 90 | 100 | 41944.82 | 16304.97 | 1341.88 (3.77 - 23020.26) |
| | C0060 | 3.40 | 90.26 | 91.67 | 98.73 | 24920.46 | 6697.48 | 994.87 (0 - 21945.61) |
| Trial 1 | C0002 | 21.61 | 1.56 | 18.46 | 90.13 | 671.09 | 153.52 | |
| Pool 1 | C0018 | 10.32 | 0.70 | 15.38 | 88.03 | 820.59 | 161.15 | |
| Shotgun | C0050 | 23.56 | 0.82 | 25.00 | 100 | 762.34 | 190.87 | |
| | C0060 | 28.70 | 0.81 | 12.50 | 84.54 | 301.55 | 44.92 | |
| Trial 2 | C0002 | 35.84 | 98.90 | 96.92 | 100 | 20081.94 | 6630.47 | 4972.95 (2.84 - 35942.31) |
| Pool 1 | C0018 | 56.55 | 98.56 | 100 | 100 | 74814.49 | 24542.74 | 144.41 (41.36 - 332.17) |
| | C0050 | 7.72 | 97.63 | 47.50 | 99.75 | 74609.44 | 24141.06 | 18991.42 (0-170582.07) |
| Enriched | C0060 | 1.31 | 93.37 | 47.92 | 83.22 | 30865.24 | 7310.50 | 17166.87 (0 - 70414.91) |
| | C0002 | 23.52 | 1.49 | 1.54 | 91.65 | 471.34 | 30.86 | |
| Trial 2 Pool 1 | C0018 | 57.30 | 0.71 | 76.92 | 79.03 | 570.56 | 98.30 | |
| | C0050 | 5.19 | 0.88 | 0 | 0 | 0 | 0 | |
| Shotgun | C0060 | 6.65 | 0.65 | 0 | 0 | 0 | 0 | |
| Trial 1 | C0002 | 68.39 | 77.35 | 96.92 | 100 | 15928.54 | 4982.54 | 57.09 (2.57 - 192.18) |
| Pool 2 | C0018 | 12.69 | 79.11 | 100 | 100 | 56570.38 | 16316.11 | 2614.81 (15.93 - 32565.71) |
| | C0050 | 12.61 | 74.13 | 75.00 | 99.93 | 41711.08 | 15702.37 | 2727.71 (0 - 39495.86) |
| Enriched | C0060 | 2.34 | 38.95 | 70.83 | 96.27 | 11523.24 | 2820.94 | 2382.94 (0 - 19387.19) |
| Trial 1 | C0002 | 58.69 | 1.34 | 58.46 | 96.92 | 321.15 | 81.43 | |
| Pool 2 | C0018 | 10.64 | 0.74 | 30.77 | 78.51 | 896.24 | 141.82 | |
| | C0050 | 11.48 | 1.33 | 20 | 100 | 1745.41 | 464.15 | |
| Shotgun | C0060 | 9.72 | 0.75 | 2.08 | 56.38 | 266.69 | 18.15 | |
| Trial 2 | C0002 | 65.64 | 98.29 | 96.92 | 100 | 19970.52 | 6708.67 | 1190.08 (7.74 - 29085.20) |
| Pool 2 | C0018 | 28.13 | 98.15 | 100 | 100 | 75034.93 | 24899.52 | 210.58 (32.41 - 596.02) |
| | C0050 | 10.26 | 98.23 | 47.50 | 100 | 77537.34 | 26906.17 | 8270.19 (0-5093725) |
| Enriched | C0060 | 0.73 | 88.86 | 27.08 | 78.56 | 37440.00 | 8936.77 | 18933.20 (0 - 106732.35) |
| Trial 2 | C0002 | 56.47 | 1.38 | 20.00 | 73.49 | 404.35 | 72.86 | |
| Pool 2 | C0018 | 29.19 | 0.57 | 23.08 | 73.76 | 698.55 | 125.73 | |
| | C0050 | 3.01 | 4.51 | 2.50 | 79.03 | 10409.44 | 2093.37 | |
| Shotgun | C0060 | 4.27 | 0.73 | 0 | 0 | 0 | 0 | |
| | C0002 | 38.74 | 94.12 | 98.46 | 100 | 19755.27 | 6312.06 | 2493.04 (3.05 - 2276727) |
| Trial 1 | C0006 | 13.66 | 84.08 | 91.43 | 100 | 51010.68 | 22066.06 | 3295.94 (0 - 61249.67) |
| | C0018 | 29.65 | 95.22 | 100 | 100 | 63154.77 | 15991.26 | 2909.12 (54.61 - 35638.08) |
| Pool 3 | C0033 | 33.17 | 94.82 | 100 | 100 | 56232.72 | 13178.66 | 156.78 (28.17 - 314.91) |
| | C0050 | 14.84 | 85.22 | 92.5 | 100 | 43478.45 | 18486.32 | 2475.78 (4.87 - 47799.65) |
| Enriched | C0060 | 2.45 | 91.97 | 89.58 | 98.78 | 26022.10 | 7430.52 | 3742.84 (3.65 - 62302.44) |
| | C0094 | 35.52 | 92.59 | 98.44 | 100 | 19949.59 | 6561.88 | 3526.16 (2.48 - 23220.26) |
| | C0292 | 2.78 | 84.96 | 91.67 | 99.29 | 28432.58 | 10574.24 | 4014.72 (0 - 54962.31) |
| | C0002 | 9.83 | 1.63 | 3.08 | 88.69 | 1449.60 | 308.97 | |
| Trial 1 | C0006 | 25.19 | 0.36 | 8.57 | 95.63 | 3450.36 | 1206.18 | |
| | C0018 | 11.94 | 0.51 | 7.69 | 68.26 | 413.96 | 50.49 | |
| Pool 3 | C0033 | 12.81 | 0.59 | 7.14 | 68.25 | 424.67 | 47.08 | |
| | C0050 | 24.09 | 0.48 | 12.5 | 93.25 | 853.91 | 300.04 | |
| Shotgun | C0060 | 17.84 | 0.90 | 4.17 | 64.69 | 222.28 | 16.28 | |
| | C0094 | 8.25 | 1.67 | 3.125 | 88.69 | 1726.91 | 368.08 | |
| | C0292 | 16.78 | 0.94 | 4.17 | 64.69 | 1141.24 | 84.87 | |
| | C0002 | 32.65 | 98.09 | 96.92 | 99.97 | 20307.57 | 6847.06 | 7369.15 (4.14 - 66339.3) |
| Trial 2 | C0006 | 7.75 | 90.49 | 51.43 | 99.50 | 86220.71 | 36708.00 | 25683.46 (0 - 271673.69) |
| | C0018 | 45.46 | 97.45 | 100 | 100 | 65485.29 | 17173.26 | 5819.09 (29.42 - 74023.04) |
| Pool 3 | C0033 | 52.11 | 97.53 | 100 | 100 | 58846.80 | 13719.18 | 698.58 (72.34 - 8084.37) |
| | C0050 | 8.22 | 92.65 | 50.00 | 99.55 | 74207.10 | 29767.85 | 21813 (0-256173.72) |
| Enriched | C0060 | 0.86 | 90.00 | 27.08 | 79.68 | 39544.66 | 8226.37 | 16172.91 (0 - 7050529) |
| | C0094 | 34.91 | 97.65 | 96.87 | 100 | 20612.44 | 7021.48 | 7479.75 (2.67 - 61794.38) |
| | C0292 | 0.89 | 89.30 | 29.17 | 80.95 | 44281.92 | 13985.84 | 18128.93 (0 - 120321.02) |
| | C0002 | 16.88 | 1.38 | 0 | 0 | 0 | 0 | |
| Trial 2 | C0006 | 15.36 | 0.47 | 0 | 0 | 0 | 0 | |
| | C0018 | 41.07 | 0.70 | 38.46 | 73.84 | 525.28 | 55.49 | |
| Pool 3 | C0033 | 44.54 | 0.79 | 50.00 | 77.22 | 703.43 | 113.13 | |
| | C0050 | 12.76 | 0.64 | 0 | 0 | 0 | 0 | |
| Shotgun | C0060 | 4.54 | 0.77 | 0 | 0 | 0 | 0 | |
| | C0094 | 21.50 | 1.23 | 1.56 | 68.13 | 404.24 | 25.04 | |
| | C0292 | 4.59 | 0.86 | 0 | 0 | 0 | 0 | |

Genomic DNA from individual strains was pooled in various ratios to produce "mock metagenomes" for enrichment. For each strain, the regions predicted be targeted by probes (determined through mapping the probeset to each individual genome) are considered the targeted region for analysis. Trimmed and filtered reads from paired enriched and shotgun pools were subsampled to same read depth. The resulting reads were mapped to the individual strain's genomes, counted on-target and normalized per kb per million reads mapping. Percentage on-target, percentage of probe-targeted regions with at least 10 reads as well as their percent coverage, average reads, and average depth were determined for each strain at the probe-targeted region level. The fold enrichment is based on all genes regardless of read counts.

In 28/32 cases, 80% or more of the reads within the enriched samples mapped to probe-targeted regions within the individual bacterial genome regardless of pooling ratios (Supplementary Table 5; part (A) of Figure 3). The one exception is Trial 1 Pool 2 (enrichment), where on-target mapping was not as effective (-70%) as the other pools for reasons that were not obvious; nevertheless, even this trial remained over 50-fold better than the unenriched samples (Supplementary Table 5). In all shotgun samples, the percentage of reads on target never exceeded 5% and in 31/32 cases was less than 2% of the total sequencing data (Supplementary Table 5, part (A) of Figure 3). Furthermore, the average percent coverage of probe-targeted regions with at least 1, 10 or 100 reads in all strains enriched individually or in pools is always higher than in the shotgun samples and ranges from 1.05- to 18.3-fold greater (part (C) of Figure 3, Supplementary Table 5). Part (C) of Figure 3 shows the average percent length coverage of probe-targeted regions with reads from strains tested individually and in pools in both enriched and shotgun samples (1 versus 10 versus 100 reads). This does not include the average percent coverage of genes in samples that did not have any captured regions (values in panel B were zero).

### Robust fold-enrichment from mock metagenomes

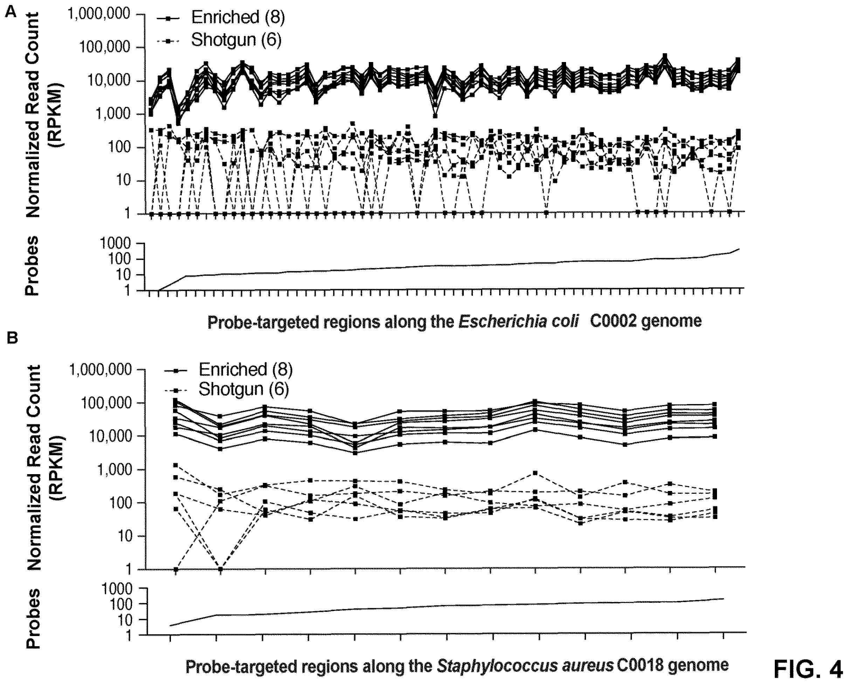

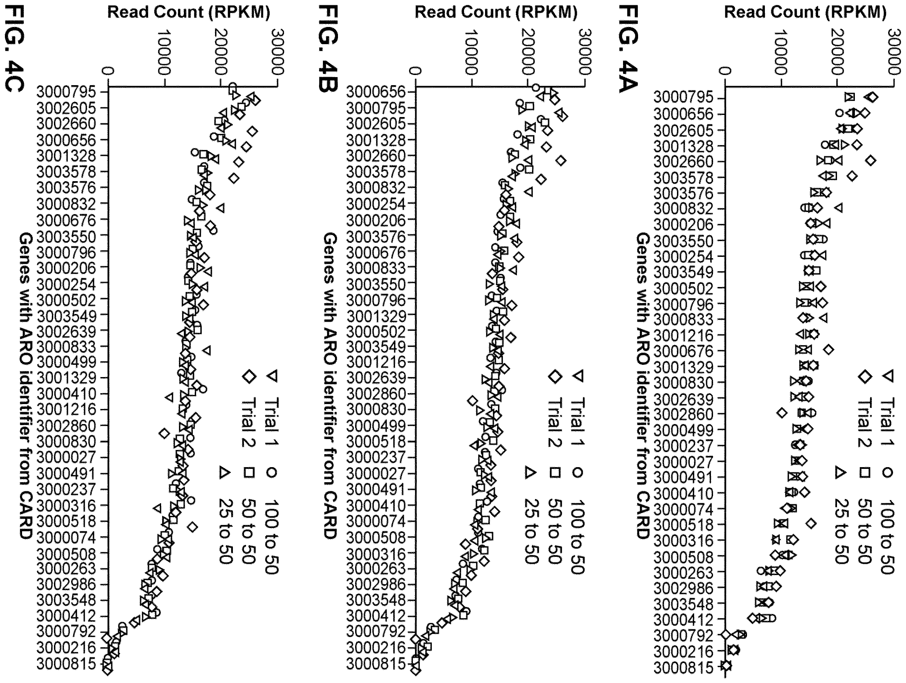

All enrichments resulted in an increased average number of read counts, a higher percentage of probe-targeted reads and higher percent coverage of these regions when compared to their shotgun controls (parts (B) and (C) of Figure 3). For all strains in all pooled libraries across both trials, the average normalized read count and depth of reads on probe-targeted ARGs from enriched libraries is over 50 times (57.09 - 25683.42) higher than from its unenriched control (Supplementary Table 5). In 31/32 cases, the fold-increase in read counts exceeded two orders of magnitude and was over four for some probe-targeted regions (Supplementary Table 5). The one case that did not conform (from Trial 1 Pool 2, see above) reflects a minor and non-reproducible variability in the quality of the capture for unknown reasons. Nonetheless, there is a clear distinction between the shotgun and enriched samples with the enriched data showing a more consistent agreement between normalized read counts per probe-targeted region. Figure 4 shows the read counts per probe-targeted region within the *Escherichia coli* C0002 strain (part A) and *Staphylococcus aureus* C0018 strain (part B) across eight enriched samples and six shotgun samples. For Figure 4, among enriched and shotgun pairs, reads were subsampled to equal depths and mapped to the individual strain's genome. Read counts were normalized by number of reads mapping per target length in kilobases per million reads. The predicted number of probes for each region along the genome are shown in the panels below. The Y axes are in the logarithmic scale.

A similar trend is observed when the raw read counts for each sample are used (Figures 3A and 3B). As shown in Figures 3A and 3B, enrichment results in higher read counts on antibiotic resistance genes compared to shotgun sequencing. Figure 3A shows raw read counts at each probe-targeted region within the *Escherichia coli* C0002 strain and Figure 3B shows raw read counts at each probe-targeted region within the *Staphylococcus aureus* C0018 strain in enriched and shotgun samples including individual and "mock metagenomes" of multiple strains. Among enriched and shotgun pairs, reads were subsampled to equal depths and mapped to the individual strain's genome. The predicted number of probes for each region along the genome are shown in the panels below. The Y axes are in the logarithmic scale.

While over 95% of the predicted genes are captured with at least 10 reads for C0002 in all the enriched samples, between 38 and 65 (all) of the probe-targeted regions have less than 10 reads in the shotgun data at the same sequencing depth (between 53,739 and 90,103 paired reads) as the enriched samples (Supplementary Tables 2, 3, 5; Figure 3).

### ARG analysis of a human GI metagenome

In order to determine the efficacy and reproducibility of the enrichment in more complex samples, enrichments were performed on replicates from metagenomic libraries with DNA isolated from a `healthy' individual's stool sample. Each library contained the same input concentration of DNA, and varying nanogram quantities of library and probes were used in nine combinations across three technical replicates (Supplementary Table 3). To determine the fold-enrichment experiments were compared with traditional shotgun sequencing; 6 of the libraries (2 in each set) were sequenced to a depth of over 3.5 million paired reads (Supplementary Table 3). Resulting reads were subsampled to the same depth using *seqtk,* normalized as per the other experiments, and then mapped to CARD using the metagenomic mapping feature (*rgi bwt*) of RGI. Also included was a series of positive control enrichments with genomic DNA from *E. coli* C0002 that was used previously for enrichment in each set. In all cases, the results identified the same genes with a consistent number of reads mapping among these replicate enrichments (when subsampled to equal depths among sets) proving reproducibility regardless of probe and library ratio (Supplementary Table 6; Figures 4A, 4B and 4C).

**Supplementary Table 6: Control enrichment with Escherichia coli C0002.**

| | Probes (ng) | Library (ng) | % reads mapping to CARD | Total number of genes | Genes with map quality >=11 | Genes with probes | Genes with length coverage with reads >=80% | Genes with probes and map quality >=11 | Genes passing all filters |
|---|---|---|---|---|---|---|---|---|---|
| C0002 - Set 1 | 25 | 50 | 63.52 | 164 | 51 | 53 | 86 | 39 | 36 |
| | 50 | 50 | 64.81 | 164 | 54 | 53 | 84 | 39 | 36 |
| | 100 | 50 | 63.75 | 154 | 53 | 53 | 80 | 40 | 36 |
| C0002 - Set2 | 25 | 50 | 61.10 | 179 | 62 | 54 | 82 | 42 | 36 |
| | 50 | 50 | 65.77 | 195 | 60 | 59 | 84 | 44 | 36 |
| | 100 | 33 | 60.31 | 170 | 59 | 57 | 87 | 42 | 36 |
| C0002 - Set 3 | 25 | 38 | 65.46 | 182 | 58 | 57 | 86 | 39 | 36 |
| | 50 | 32 | 65.77 | 172 | 58 | 53 | 88 | 40 | 36 |
| | 100 | 38 | 67.98 | 147 | 54 | 56 | 83 | 42 | 36 |

Enrichment results from the positive control of *E*. *coli* C0002 control used in Phase 2. Trimmed and deduplicated reads were mapped to CARD using RGIBWT, filtered by genes with probe coverage, an average read mapping quality >=11, and percent length coverage of a gene with reads >=80%.

Within each set, there was found an excellent correlation with previous results seen with *E*. *coli* C0002 in Trial 1 and 2 (Pearson correlations: >0.923 for all pairs in Set 1, >0.924 for Set 2, >0.901 for Set 3) (Figures 4A, 4B and 4C). Figures 4A, 4B and 4C show normalized read counts from C0002 control enrichments from three samples in each set (Figure 4A corresponds to set 1, Figure 4B corresponds to set 2 and Figure 4C corresponds to set 3) to the two trials of individual enrichment. Genes with reads were filtered based on read mapping quality greater than or equal to 80% and genes with probes mapping. Genes are ordered by sum of read counts from highest to lowest (left to right) with the ARO identifier shown along the X axis.

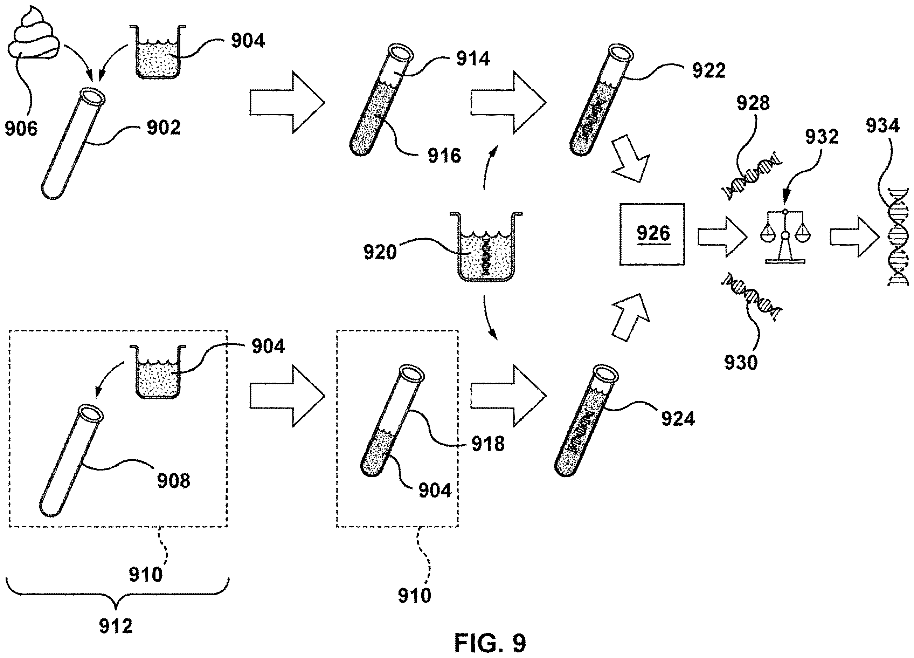

As will be described further below in the context of Figure 9, negative controls can be implemented to suppress false positives (Type I Error) during analysis. To track and measure the contamination in the lab and chemicals, a negative control of a blank DNA extraction was included and processed identically to the DNA used in Phase 1 and Phase 2 throughout library preparation, enrichment, and sequencing. A negative reagent control was also included throughout enrichment. For Phase 1 in both Trial 1 and Trial 2, a negligible amount of library DNA was found in the Blank after enrichment and very few of the sequenced reads were associated with the indexes used for the Blank library (between 2.46% and 8.96% of sequenced reads; Supplementary Table 3, Supplementary Table 7).

**Supplementary Table 7: Sequencing reads identified in the Blank samples. Enriched negative control blank libraries were sequenced on separate MiSeq 2 x 250 runs. After de-multiplexing, we pulled the reads that were associated with various index combinations used alongside the Blank Negative control throughout library preparation within the same trials and sets.**

| Sample | Samples processed alongside the blank library | Number of paired reads sequenced on run with Blank | Percentage of Blank |
|---|---|---|---|
| Blank Trial 1 | C0002 | 1575 | 0.92 |
| | C0018 | 0 | 0.00 |
| | C0050 | 435 | 0.26 |
| | C0060 | 379 | 0.22 |
| | Pool1 | 3064 | 1.80 |
| | Pool2 | 110959 | 65.05 |
| | Pool3 | 36390 | 21.33 |
| | Additional barcodes | 2487 | 1.46 |
| | Blank | 15276 | 8.96 |
| Blank Trial 2 | C0002 | 6611 | 15.02 |
| | C0018 | 11763 | 26.72 |
| | C0050 | 5194 | 11.80 |
| | C0060 | 4491 | 10.20 |
| | Pool1 | 1178 | 2.68 |
| | Pool2 | 4800 | 10.90 |
| | Pool3 | 5862 | 13.31 |
| | Additional barcodes | 3044 | 6.91 |
| | Blank | 1083 | 2.46 |
| Blank Set 1 | 1 - 1 | 456 | 17.61 |
| | 1-2 | 94 | 3.63 |
| | 1 -3 | 174 | 6.72 |
| | 1-4 | 101 | 3.90 |
| | 1-5 | 316 | 12.20 |
| | 1 -6 | 82 | 3.17 |
| | 1 -7 | 683 | 26.37 |
| | 1 -8 | 173 | 6.68 |
| | 1 -9 | 35 | 1.35 |
| | Negative Control - Blank | 28 | 1.08 |
| | C0002 - 1 - 1 | 120 | 4.63 |
| | C0002 - 1 - 2 | 37 | 1.43 |
| | C0002 - 1 - 3 | 291 | 11.24 |
| Blank Set 2 | 2-1 | 367 | 9.65 |
| | 2-2 | 22 | 0.58 |
| | 2-3 | 44 | 1.16 |
| | 2 - 4 | 119 | 3.13 |
| | 2 - 5 | 40 | 1.05 |
| | 2 - 6 | 0 | 0.00 |
| | 2 - 7 | 39 | 1.03 |
| | 2-8 | 271 | 7.12 |
| | 2 - 9 | 137 | 3.60 |
| | Negative Control - Blank | 530 | 13.93 |
| | C0002 - 2 -1 | 207 | 5.44 |
| | C0002 - 2 -2 | 34 | 0.89 |
| | C0002 - 2 - 3 | 1994 | 52.42 |
| Blank Set 3 | 3 - 1 | 224 | 3.76 |
| | 3 - 2 | 286 | 4.80 |
| | 3 - 3 | 71 | 1.19 |
| | 3 - 4 | 1653 | 27.73 |
| | 3 - 5 | 282 | 4.73 |
| | 3 - 6 | 23 | 0.39 |
| | 3 - 7 | 42 | 0.70 |
| | 3 - 8 | 128 | 2.15 |
| | 3 - 9 | 1198 | 20.09 |
| | Negative Control - Blank | 0 | 0.00 |
| | C0002 - 3 -1 | 161 | 2.70 |
| | C0002 - 3 -2 | 817 | 13.70 |
| | C0002 - 3 - 3 | 1077 | 18.06 |

After trimming and removing duplicates, more than 80% of these reads mapped to CARD with only ten genes in Trial 1 with at least 10 reads each and percent length coverage (>=10), read mapping quality (>=11) and probes mapping (Supplementary Table 8).

**Supplementary Table 8: Negative control enrichment with Blank samples. Enriched reds were divided among index combinations used during the respective Phase, Trial or Set (Supplementary Table 7). The reads belonging to each Negative Control - Blank library were trimmed and duplicates were removed then mapped to CARD through rgibwt. The number of genes with 1, at least 10 and at least 100 reads as well as genes with probes mapping, with average read mapping quality >=11 and gene length coverage with reads >=10% are shown. In Phase 2 Set 1, raw sequencing reads were used for analysis, in Set 2, deduplication was omitted, and for Set 3, there were no reads associated with the Blank indexes after sequencing.**

| Sample | Paired reads | Paired reads after trimming and deduplication | Percent of reads mapping to CARD | Total genes with reads | Genes with 10 or more reads | Genes with 100 or more reads | Genes with at least 10 reads, >10% read coverage, MQ >=11 and probes |
|---|---|---|---|---|---|---|---|
| Blank | 15276 | 2716 | 80.34 | 153 | 82 | 9 | 10: *cpxA, mefA, arlS, mdtO, mdtE, mdtN, acrD, armA, AAC(3)-IV, APH(7")-Ia,* |
| Phase 1 | | | | | | | |
| Trial 1 | | | | | | | |
| Blank | 1083 | 341 | 97.21 | 106 | 9 | 1 | 0 |
| Phase 1 | | | | | | | |
| Trial 2 | | | | | | | |
| Phase 2 | 28 | N/A | 0 | 0 | 0 | 0 | 0 |
| Set 1 | | | | | | | |
| | 530 | 412 | 76.46 | 94 | 26 | 0 | **19:** |
| Phase 2 Set 2* | | | | | | | *APH(3")-Ib, acrD, acrE, acrF, acrS, cpxA, dfrA17, emrK, emrY, eptA, evgS, mdtE, mdtF, mdtH, mdtO, mdtP, pmrF, tetQ, tolC* |
| Phase 2 Set 3 | 0 | 0 | 0 | 0 | 0 | 0 | 0 |

For Phase 2, only the Blank from Set 2 produced sufficient reads to map to CARD (76.46% reads mapping), and 19 genes were identified (Supplementary Table 8). Of these genes, two are found only in the blank sample, two are found in both shotgun and enriched libraries (*tetQ* and *acrF*), but 15 genes overlap between the blank and enriched libraries.

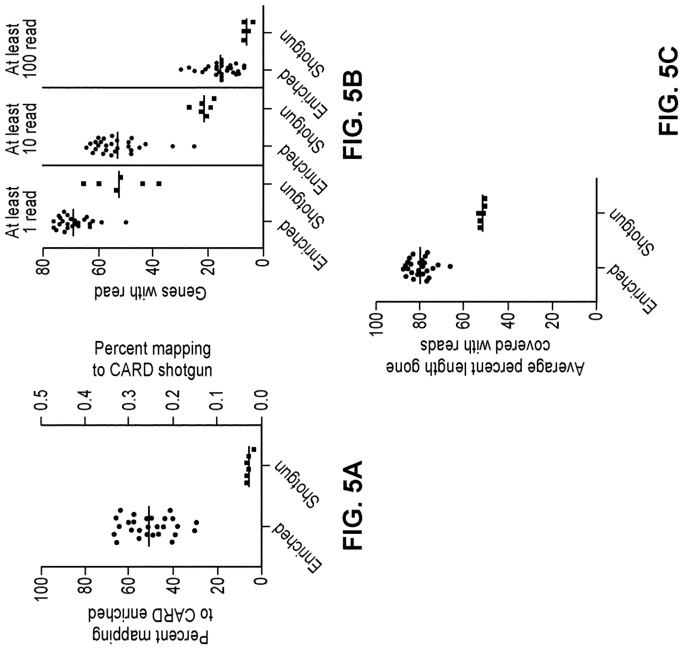

Across the enriched samples, with the full number of reads and no filters, an average of 50.69% of reads map to CARD with on average 68 genes identified with at least 10 reads, compared to 0.03% mapping in the shotgun libraries and 32 genes on average (Figures 5A and 5B; Supplementary Table 9).

**Supplementary Table 9: Phase 2 enrichment results with the full number of reads.**

| | Probes (ng) | Library (ng) | Reads mapping to CARD (%) | Total number of genes | Genes with read map quality >=11 | Genes with probes | Genes with read length coverage >=10% | Genes passing all filters |
|---|---|---|---|---|---|---|---|---|
| **Sample Set 1** | | | | | | | | |
| EN | 25 | 50 | 55.36 | 60 | 50 | 51 | 58 | 48 |
| | 50 | 50 | 65.73 | 62 | 54 | 52 | 60 | 49 |
| | 100 | 50 | 55.59 | 60 | 50 | 50 | 60 | 48 |
| | 50 | 100 | 65.63 | 56 | 47 | 46 | 55 | 43 |
| | 100 | 100 | 51.85 | 61 | 51 | 51 | 60 | 48 |
| | 200 | 100 | 58.21 | 64 | 56 | 53 | 61 | 49 |
| | 100 | 200 | 51.52 | 34 | 26 | 27 | 34 | 25 |
| | 200 | 200 | 66.57 | 60 | 50 | 48 | 59 | 45 |
| | 400 | 200 | 49.44 | 45 | 37 | 36 | 43 | 33 |
| UN | 200 | 100 | 0.030 | 26 | 19 | N/A | 24 | 18 |
| | 100 | 200 | 0.030 | 32 | 22 | N/A | 29 | 20 |

| **Sample Set 2** | | | | | | | | |
|---|---|---|---|---|---|---|---|---|
| EN | 25 | 50 | 64.07 | 78 | 67 | 64 | 76 | 61 |
| | 50 | 50 | 64.60 | 72 | 64 | 61 | 71 | 58 |
| | 100 | 50 | 57.96 | 75 | 64 | 61 | 74 | 57 |
| | 50 | 100 | 46.75 | 78 | 66 | 66 | 76 | 62 |
| | 100 | 100 | 58.99 | 79 | 69 | 64 | 77 | 61 |
| | 200 | 100 | 44.52 | 85 | 72 | 69 | 80 | 63 |
| | 100 | 200 | 60.43 | 76 | 66 | 62 | 73 | 59 |
| | 200 | 200 | 47.27 | 82 | 71 | 67 | 81 | 64 |
| | 400 | 200 | 41.22 | 70 | 59 | 58 | 69 | 55 |
| UN | 400 | 200 | 0.016 | 41 | 28 | N/A | 37 | 27 |
| | 100 | 100 | 0.032 | 34 | 24 | N/A | 32 | 23 |

| **Sample Set 3** | | | | | | | | |
|---|---|---|---|---|---|---|---|---|
| EN | 25 | 50 | 50.16 | 72 | 63 | 61 | 70 | 58 |
| | 50 | 50 | 38.19 | 79 | 66 | 64 | 76 | 60 |
| | 100 | 50 | 51.73 | 69 | 59 | 59 | 68 | 55 |
| | 50 | 100 | 29.46 | 78 | 66 | 63 | 76 | 60 |
| | 100 | 100 | 40.28 | 74 | 65 | 60 | 72 | 57 |
| | 200 | 100 | 39.06 | 67 | 57 | 57 | 67 | 53 |
| | 100 | 200 | 29.97 | 69 | 57 | 58 | 68 | 54 |
| | 200 | 200 | 40.32 | 72 | 60 | 58 | 71 | 55 |
| | 400 | 200 | 43.74 | 69 | 58 | 56 | 67 | 53 |
| UN | 100 | 100 | 0.031 | 29 | 19 | N/A | 26 | 19 |
| | 25 | 50 | 0.031 | 34 | 23 | N/A | 30 | 22 |

For the enriched samples, trimmed and deduplicated reads were mapped to CARD using RGIBWT, filtered by genes with at least 10 reads, those with probes, an average read mapping quality >=11, and length coverage of a gene with reads >=10%. For the shotgun samples, trimmed and deduplicated reads were mapped to CARD using RGIBWT, filtered by genes with an average read mapping quality >=11 and read length coverage of a gene >=10%. EN = enriched, UN = shotgun.

Significantly more genes with at least 1, 10, and 100 reads from each enriched sample were found as compared to the shotgun samples and that the average percent coverage of a gene by reads in the enriched samples is 1.5-fold higher (Figures 5B and 5C). In Figures 5A, 5B and 5C, for the enriched and shotgun samples, the full number of reads for each sample were mapped to CARD using *rgi bwt.* Figure 5A shows the percentage of reads mapping to CARD. For Figure 5B, genes were counted with at least 1, 10 and 100 reads and filtered for mapping quality (>=11), percent coverage by reads (>=10) and probes mapping (only for the enriched samples). Figure 5C shows the average percent coverage of all genes with at least 10 reads in each sample after the same filters used in Figure 5B.

Less than 0.1% of reads (at between 7 million and 15 million reads) overall in the shotgun stool samples mapped to CARD, which is consistent with the expectation that resistance genes represent a minor proportion of the total gut microbiome in healthy individuals (Supplementary Table 9). When subsampled to the same depth as their enriched pairs (between 22,324 and 149,320 reads), the results identified on average 1 (range: 0 - 2) antibiotic resistance determinant with at least 10 reads after filtering in the shotgun samples (Supplementary Table 10).

**Supplementary Table 10: Phase 2 enrichment results with subsampled reads.**

| | Probes (ng) | Library (ng) | Reads mapping to CARD (%) | Total number of genes | Genes with read map quality >=11 | Genes with probes | Genes with read length coverage >=10% | Genes passing all filters |
|---|---|---|---|---|---|---|---|---|
| **Sample Set 1** | | | | | | | | |
| EN | 25 | 50 | 55.24 | 34 | 26 | 27 | 34 | 25 |
| | 50 | 50 | 65.84 | 39 | 31 | 31 | 37 | 28 |
| | 100 | 50 | 56.11 | 46 | 37 | 37 | 45 | 34 |
| | 50 | 100 | 66.01 | 39 | 32 | 32 | 39 | 30 |
| | 100 | 100 | 51.94 | 40 | 32 | 32 | 37 | 28 |
| | 200 | 100 | 57.93 | 38 | 30 | 30 | 37 | 28 |
| | 100 | 200 | 51.52 | 34 | 26 | 27 | 34 | 25 |
| | 200 | 200 | 66.99 | 42 | 34 | 33 | 39 | 30 |
| | 400 | 200 | 49.39 | 33 | 26 | 26 | 33 | 24 |
| UN | 200 | 100 | 0.038 | 2 | 2 | N/A | 2 | 2 |
| | 100 | 200 | 0.054 | 0 | 0 | N/A | 0 | 0 |

| **Sample Set 2** | | | | | | | | |
|---|---|---|---|---|---|---|---|---|
| EN | 25 | 50 | 64.25 | 41 | 33 | 34 | 40 | 32 |
| | 50 | 50 | 64.11 | 43 | 36 | 35 | 40 | 31 |
| | 100 | 50 | 58.80 | 43 | 36 | 35 | 43 | 33 |

| | Probes (ng) | Library (ng) | Reads mapping to CARD (%) | Total number of genes | Genes with read map quality >=11 | Genes with probes | Genes with read length coverage >=10% | Genes passing all filters |
|---|---|---|---|---|---|---|---|---|
| | 50 | 100 | 46.95 | 40 | 32 | 33 | 38 | 29 |
| | 100 | 100 | 59.13 | 42 | 35 | 34 | 41 | 31 |
| | 200 | 100 | 44.64 | 45 | 35 | 34 | 41 | 31 |
| | 100 | 200 | 60.55 | 50 | 42 | 42 | 49 | 39 |
| | 200 | 200 | 47.29 | 45 | 38 | 37 | 45 | 35 |
| | 400 | 200 | 41.56 | 43 | 34 | 35 | 41 | 32 |
| UN | 400 | 200 | 0.029 | 1 | 1 | N/A | 1 | 1 |
| | 100 | 100 | 0.035 | 2 | 2 | N/A | 2 | 2 |

| **Sample Set 3** | | | | | | | | |
|---|---|---|---|---|---|---|---|---|
| EN | 25 | 50 | 50.64 | 37 | 29 | 30 | 36 | 27 |
| | 50 | 50 | 37.85 | 27 | 19 | 20 | 27 | 18 |
| | 100 | 50 | 51.41 | 36 | 27 | 28 | 33 | 24 |
| | 50 | 100 | 29.56 | 29 | 21 | 22 | 28 | 20 |
| | 100 | 100 | 40.77 | 34 | 26 | 26 | 33 | 24 |
| | 200 | 100 | 38.86 | 37 | 30 | 30 | 37 | 28 |
| | 100 | 200 | 30.08 | 31 | 23 | 24 | 30 | 21 |
| | 200 | 200 | 40.62 | 34 | 26 | 26 | 32 | 23 |
| | 400 | 200 | 44.35 | 37 | 30 | 29 | 35 | 26 |
| UN | 100 | 100 | 0.023 | 0 | 0 | N/A | 0 | 0 |
| | 25 | 50 | 0.023 | 1 | 1 | N/A | 1 | 1 |

For the enriched samples, reads were subsampled to 22,324 reads and mapped to CARD using RGIBWT. Results were filtered by genes with at least 10 reads, those with probes, an average read mapping quality >=11, and length coverage of a gene with reads >=10%. For the shotgun samples, reads were subsampled to their paired enriched sample and mapped to CARD using RGIBWT. Results were filtered by genes with an average read mapping quality >=11 and read length coverage of a gene >=10%. EN = enriched, UN = shotgun.

Conversely, when subsampled to the depth of the lowest enriched sample (22,324 reads), on average 28 ARGs in the enriched libraries post-filtering with at least 10 reads were identified (Supplementary Table 10). For further analysis of the shotgun data, the full number of reads was used and the probe-mapping filter was omitted to allow inclusion of genes that the probes do not target. Finally, as there were only a few genes with reads at 80% read length coverage in the shotgun samples, the cut-off was reduced to a 10% length coverage by reads filter for sufficient analyses.

### High fold-enrichment of ARGs from human stool

The genes and their read counts that passed the chosen filters (at least 10 reads, 10% gene length coverage by reads, mapping quality at least 11 and probes mapping) were combined within each set to compare between probe and library ratios in subsampled and full read samples through both enrichment and shotgun sequencing. With the full number of reads, 24/70 (34.28%) of genes detected overlap among all enriched libraries (n = 27), while there were identified 16 genes of a total 32 (50.00%) in all the shotgun libraries (n = 6, Supplementary Table 9, 11).

**Supplementary Table 11: Phase 2 overlapping genes with the full number of reads.**

| Samples | Total genes | Genes found in all | Genes found in 2/3 or more | Genes found in 1/3 or more | Overlap in All Samples (%) |
|---|---|---|---|---|---|
| Set 1 Enriched | 62 | 24 | 38 | 53 | 38.71 |
| Set 2 Enriched | 68 | 50 | 57 | 64 | 73.53 |
| Set 3 Enriched | 70 | 41 | 53 | 60 | 58.57 |
| All Enriched | 70 | 24 | 52 | 60 | 34.28 |
| All Shotgun | 32 | 16 | 18 | 28 | 50.00 |

We calculated the overlap of genes with at least 10 reads passing the percent length coverage by reads (>=10%), average read mapping quality (>=11) and probe mapping (except for shotgun libraries) filters.

When subsampled to the lowest enriched read coverage (22,324 reads), there are no genes that overlap between all six shotgun libraries, while 13/47 (27.66%) of genes overlap across all 27 enriched libraries (Supplementary Table 12).

**Supplementary Table 12: Phase 2 overlapping genes with subsampled reads.**

| Samples | Total genes | Genes found in all | Genes found in 2/3 or more | Genes found in 1/3 or more | Overlap in All Samples (%) |
|---|---|---|---|---|---|
| Set 1 Enriched | 38 | 16 | 26 | 32 | 42.10 |
| Set 2 Enriched | 45 | 22 | 30 | 36 | 48.89 |
| Set 3 Enriched | 37 | 13 | 20 | 26 | 35.14 |
| All Enriched | 47 | 13 | 24 | 31 | 27.66 |
| All Shotgun | 2 | 0 | 1 | 2 | 0 |

Libraries were subsampled to the same number of reads within sets and overall (22,324 reads). Shotgun libraries were subsampled to the same number of reads as the lowest enriched library overall. Resulting genes with at least 10 reads were filtered for percent coverage by reads (>=10%), average mapping quality (>=11) and probe mapping (except for the shotgun samples).

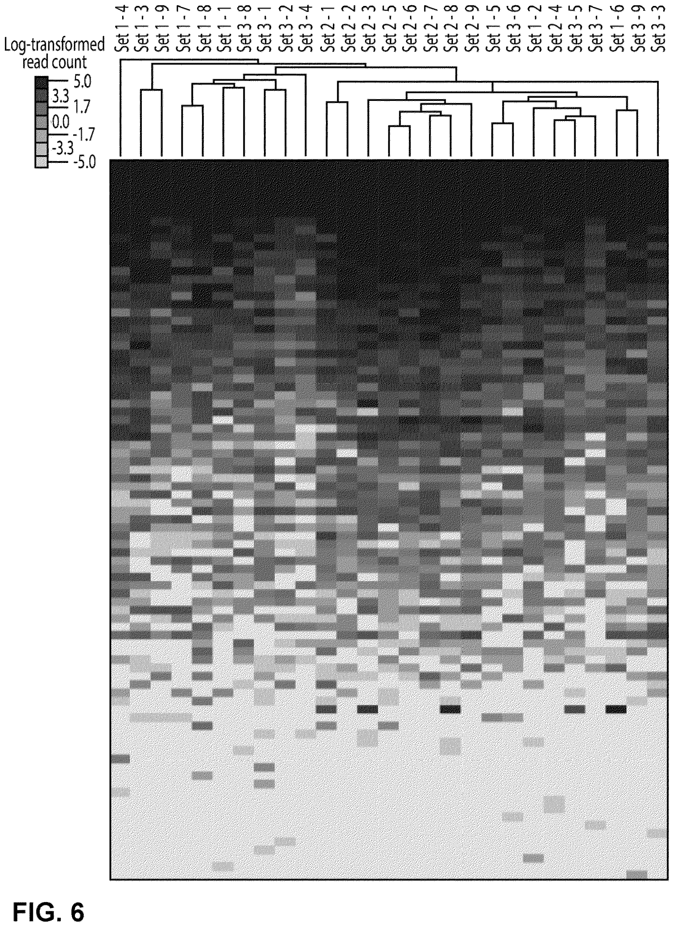

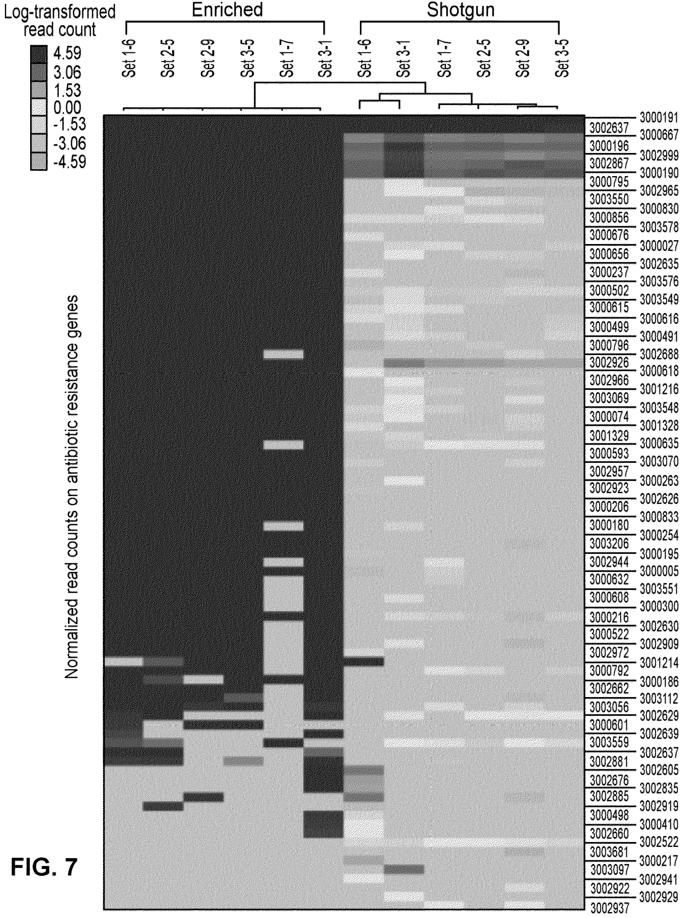

Comparing among subsampled enriched libraries (22,324 reads), the majority (31/34) of genes missing in at least one sample are those with on average less than twenty reads across the 27 libraries (Supplementary Tables 10; Figure 6). For Figure 6, enriched reads from 27 libraries were subsampled to 22,324 reads, mapped to CARD through *rgi bwt.* The reads were mapped to CARD through *rgi bwt* and filtered for genes with probes mapping, with greater than or equal to 10% length coverage by reads and an average read mapping quality >=11. Read counts were log-transformed and combined into a heatmap ordered by average read counts across the 27 enriched samples. The order of genes with higher read counts is consistent among enriched samples (Figure 6). This phenomenon with the shotgun samples is also seen at the full number of reads where there is a high agreement in read counts for genes expected or known to be present in higher abundance (i.e. gene copy number) and a more significant discrepancy between reads targeting lower abundance genes (Figure 7). For Figure 7, the full number of reads from the 6 enriched and shotgun pairs were mapped to CARD through *rgi bwt.* The results were filtered for genes with greater than or equal to 10% read length coverage and an average read mapping quality >= 11. Read counts were normalized by kb of gene and reads available for mapping, log-transformed and combined into a heatmap. Genes are ordered by sum of read counts. ARO numbers from CARD are shown on the right-hand side of the heatmap.

Thus, enrichment does not in some way bias the prevalence of rank order of AMR in these samples. Finally, both methods resulted in excellent correlation among technical replicates individually (Pearson correlation 0.871 for shotgun and 0.972 for enriched; Figures 6 and 7).

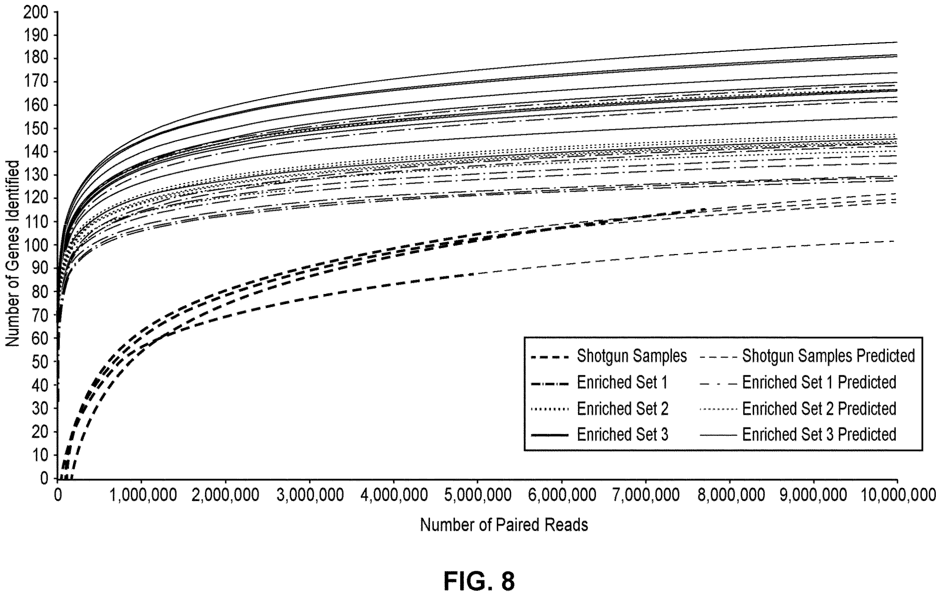

It was found that enrichment exceeded shotgun sequencing by identifying more unique antibiotic resistance genes at much lower sequencing depths. The enriched samples provided a more diverse representation of ARGs at less than 100,000 paired reads compared to over 5 million reads in the shotgun samples (Figure 8). For Figure 8, the AmrPlusPlus Rarefaction Analyzer was used with subsampling every 1% of the total reads and a gene read length of at least 10% to identify antibiotic resistance genes. The solid lines show individual sequencing experiments and the dotted lines are the logarithmic extrapolations beyond the experimental sequencing depth.

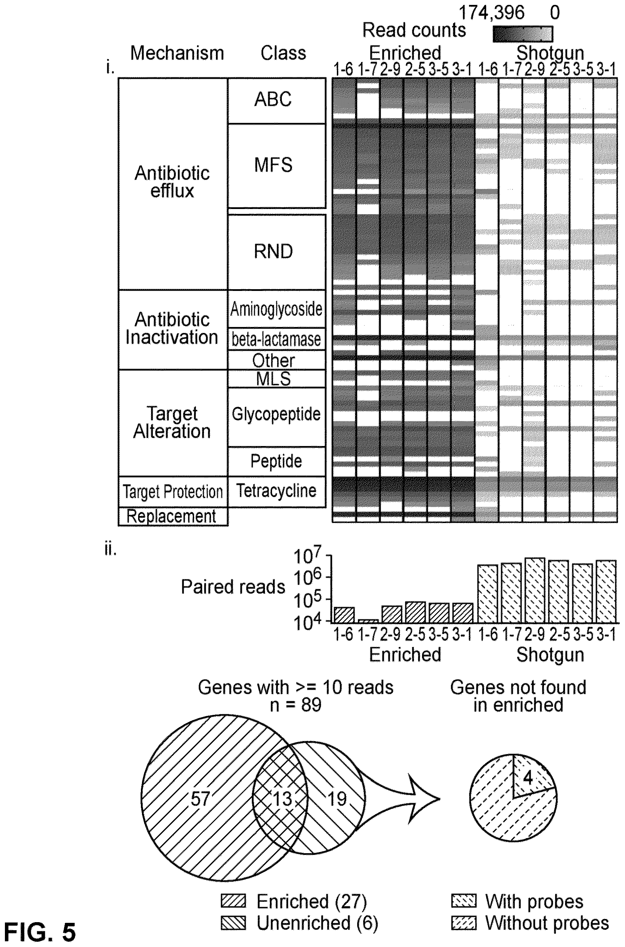

With the full number of reads in both methods (between 66- and 389-fold more in the shotgun samples than the enriched samples), the average fold-enrichment is greater than 600-fold and there are still 18 to 50 fewer genes in the shotgun samples (part (A) of Figure 5; Supplementary Table 14). For the enriched and shotgun samples, the full number of reads for each sample were mapped to CARD using *rgi bwt* and the results were filtered for genes with probes mapping, with reads with an average mapping quality >=11 and a percent length coverage of a gene by reads greater than or equal to 10%. In part (A) of Figure 5, read counts were normalized per kilobase of reference gene per million reads sequenced (RPKM) and log transformed to produce the heatmap. The rows are grouped based on resistance mechanisms as annotated in CARD (not all mechanisms and classes are shown). ABC = ATP-binding cassette antibiotic efflux pump; MFS = major facilitator superfamily antibiotic efflux pump; RND = resistance-nodulation cell division antibiotic efflux pump; MLS = macrolides, lincosamides, streptogramins. ii) The number of reads used for mapping in each sample.

In most cases, there are only a few genes found via shotgun that are missing in the enriched paired sample (between 9 and 15; 22 unique genes). Only between 1 to 5 genes in each sample is predicted to be targeted by probes for a total of 7 unique genes not identified in the enriched counterpart of each pair (Supplementary Table 14). Of these, only one, *novA* (ARO: 3002522), is missing from all enriched samples but is present in all shotgun samples with >10 reads, mapping quality >=11 and percent length coverage by reads >=10%. The other 6 genes (*macB* (ARO: 3000535), *vanRG* (ARO: 3002926), *vanSG* (ARO: 3002937), *smeE* (ARO: 3003056), *cfxA6* (ARO: 3003097), *cepA* (ARO: 3003559)) are found in only a few shotgun samples with less than 30 reads and less than 20% read length coverage on average (Supplementary Table 14; Supplementary Table 13).

| **ARO** | **Baits** | **Set 1 - 3** | **Set 1 - 4** | **Set 1 - 7** | **Set 1 - 6** | **Set 1 - 9** | **Set 1 - 8** | **Set 1 - 5** | **Set 1 - 2** | **Set 1 - 1** |
|---|---|---|---|---|---|---|---|---|---|---|
| 3000190 | Yes | 2240 | 2088 | 655 | 3095 | 1195 | 2459 | 2613 | 2472 | 2909 |
| 3000191 | Yes | 21747 | 21337 | 7489 | 30223 | 13830 | 27383 | 22368 | 25974 | 25651 |
| 3000196 | Yes | 5306 | 4929 | 1610 | 7133 | 2788 | 6253 | 5760 | 5554 | 6339 |
| 3000567 | Yes | 4375 | 3252 | 978 | 5835 | 1891 | 4454 | 4654 | 3774 | 4098 |
| 3002837 | Yes | 2403 | 2223 | 828 | 2740 | 1202 | 2523 | 2240 | 2590 | 2884 |
| 3002867 | Yes | 1093 | 1242 | 412 | 1185 | 485 | 1126 | 1232 | 1296 | 1770 |
| 3002999 | Yes | 2531 | 2026 | 743 | 3297 | 1182 | 2927 | 2612 | 2258 | 2268 |
| 3002926 | Yes | 16 | 15 | 0 | 39 | 0 | 20 | 24 | 10 | 22 |
| 3000194 | No | 0 | 0 | 0 | 0 | 0 | 0 | 0 | 0 | 0 |
| 3000375 | No | 0 | 0 | 0 | 0 | 0 | 0 | 0 | 0 | 0 |
| 3000501 | No | 0 | 0 | 0 | 0 | 0 | 0 | 0 | 0 | 0 |
| 3002522 | Yes | 0 | 0 | 0 | 0 | 0 | 0 | 0 | 0 | 0 |
| 3002597 | No | 0 | 0 | 0 | 0 | 0 | 0 | 0 | 0 | 0 |
| 3003318 | No | 0 | 0 | 0 | 0 | 0 | 0 | 0 | 0 | 0 |
| 3003730 | No | 0 | 0 | 0 | 0 | 0 | 0 | 0 | 0 | 0 |
| 3004454 | No | 0 | 0 | 0 | 0 | 0 | 0 | 0 | 0 | 0 |
| 3002965 | Yes | 50 | 25 | 0 | 41 | 24 | 74 | 48 | 52 | 57 |
| 3000535 | Yes | 26 | 0 | 0 | 107 | 0 | 43 | 56 | 0 | 29 |
| 3002647 | No | 0 | 0 | 0 | 0 | 0 | 0 | 0 | 0 | 0 |
| 3000556 | Yes | 82 | 111 | 28 | 90 | 27 | 111 | 101 | 91 | 144 |
| 3003056 | Yes | 16 | 0 | 0 | 13 | 0 | 10 | 0 | 0 | 0 |
| 3002937 | Yes | 0 | 0 | 0 | 0 | 0 | 0 | 0 | 0 | 0 |
| 3002983 | No | 0 | 0 | 0 | 0 | 0 | 0 | 0 | 0 | 0 |
| 3003559 | Yes | 0 | 0 | 0 | 0 | 0 | 0 | 0 | 0 | 0 |
| 3004032 | No | 0 | 0 | 0 | 0 | 0 | 0 | 0 | 0 | 0 |
| 3004033 | No | 0 | 0 | 0 | 0 | 0 | 0 | 0 | 0 | 0 |
| 3004074 | No | 0 | 0 | 0 | 0 | 0 | 0 | 0 | 0 | 0 |
| 3004144 | No | 0 | 0 | 0 | 0 | 0 | 0 | 0 | 0 | 0 |
| 3000502 | Yes | 190 | 107 | 28 | 181 | 51 | 140 | 141 | 127 | 130 |
| 3000793 | No | 0 | 0 | 0 | 0 | 0 | 0 | 0 | 0 | 0 |
| 3000794 | No | 0 | 0 | 0 | 0 | 0 | 0 | 0 | 0 | 0 |
| 3003097 | Yes | 0 | 0 | 0 | 0 | 0 | 0 | 0 | 0 | 0 |
| 3000027 | Yes | 49 | 51 | 20 | 40 | 26 | 51 | 39 | 37 | 38 |
| 3000237 | Yes | 39 | 36 | 28 | 57 | 26 | 102 | 53 | 53 | 94 |
| 3000491 | Yes | 83 | 66 | 24 | 173 | 36 | 111 | 143 | 83 | 88 |
| 3000615 | Yes | 57 | 27 | 13 | 55 | 27 | 68 | 33 | 36 | 43 |
| 3000616 | Yes | 28 | 11 | 13 | 50 | 11 | 36 | 53 | 69 | 73 |
| 3000795 | Yes | 92 | 64 | 12 | 173 | 38 | 78 | 125 | 56 | 96 |
| 3000796 | Yes | 144 | 102 | 22 | 223 | 76 | 110 | 94 | 102 | 131 |
| 3000830 | Yes | 93 | 40 | 12 | 97 | 49 | 56 | 66 | 54 | 49 |
| 3000833 | Yes | 46 | 55 | 11 | 49 | 28 | 42 | 27 | 18 | 19 |
| 3001216 | Yes | 23 | 55 | 11 | 73 | 19 | 35 | 35 | 66 | 23 |
| 3001328 | Yes | 44 | 28 | 11 | 22 | 17 | 42 | 37 | 19 | 32 |
| 3003549 | Yes | 75 | 91 | 20 | 104 | 36 | 93 | 112 | 79 | 118 |
| 3003550 | Yes | 73 | 44 | 34 | 118 | 53 | 83 | 74 | 74 | 57 |
| 3003576 | Yes | 59 | 76 | 16 | 112 | 30 | 65 | 65 | 68 | 91 |
| 3003578 | Yes | 68 | 25 | 11 | 71 | 30 | 42 | 46 | 53 | 47 |
| 3000074 | Yes | 42 | 15 | 0 | 36 | 15 | 29 | 48 | 43 | 19 |
| 3000499 | Yes | 68 | 37 | 0 | 76 | 24 | 40 | 66 | 56 | 48 |
| 3000518 | Yes | 31 | 10 | 0 | 47 | 17 | 28 | 21 | 16 | 10 |
| 3000656 | Yes | 23 | 28 | 0 | 36 | 26 | 18 | 16 | 11 | 37 |
| 3002635 | Yes | 59 | 31 | 15 | 65 | 0 | 35 | 29 | 51 | 46 |
| 3003548 | Yes | 57 | 40 | 0 | 33 | 15 | 18 | 24 | 17 | 11 |
| 3000254 | Yes | 40 | 17 | 0 | 25 | 18 | 26 | 14 | 38 | 37 |
| 3001329 | Yes | 24 | 38 | 0 | 36 | 0 | 12 | 30 | 60 | 12 |
| 3002986 | Yes | 27 | 33 | 0 | 35 | 0 | 42 | 20 | 15 | 17 |
| 3000216 | Yes | 13 | 13 | 0 | 35 | 0 | 25 | 14 | 16 | 0 |
| 3002688 | Yes | 0 | 13 | 0 | 14 | 0 | 25 | 24 | 14 | 21 |
| 3000195 | Yes | 15 | 0 | 0 | 15 | 0 | 0 | 19 | 19 | 15 |
| 3000300 | Yes | 22 | 0 | 0 | 0 | 14 | 17 | 11 | 12 | 13 |
| 3000676 | Yes | 0 | 0 | 0 | 18 | 0 | 11 | 19 | 18 | 0 |
| 3003070 | Yes | 25 | 19 | 0 | 22 | 0 | 0 | 19 | 0 | 0 |
| 3000180 | Yes | 15 | 15 | 0 | 0 | 0 | 0 | 0 | 12 | 31 |
| 3000593 | Yes | 0 | 0 | 0 | 10 | 0 | 12 | 0 | 23 | 15 |
| 3002626 | Yes | 17 | 0 | 0 | 0 | 11 | 0 | 0 | 11 | 0 |
| 3003069 | Yes | 27 | 0 | 0 | 13 | 0 | 0 | 12 | 0 | 0 |
| 3003206 | Yes | 13 | 0 | 0 | 15 | 0 | 16 | 0 | 0 | 31 |
| 3000206 | Yes | 0 | 0 | 0 | 0 | 0 | 14 | 12 | 0 | 13 |
| 3003551 | Yes | 0 | 22 | 0 | 0 | 0 | 0 | 13 | 16 | 0 |
| 3002923 | Yes | 0 | 0 | 0 | 0 | 0 | 0 | 13 | 0 | 13 |
| 3002944 | Yes | 15 | 0 | 0 | 17 | 0 | 0 | 12 | 0 | 0 |
| 3000005 | Yes | 0 | 15 | 0 | 28 | 0 | 0 | 0 | 12 | 0 |
| 3000522 | Yes | 29 | 15 | 0 | 11 | 0 | 0 | 10 | 0 | 10 |
| 3000263 | Yes | 0 | 0 | 0 | 0 | 0 | 0 | 0 | 10 | 10 |
| 3000832 | Yes | 0 | 15 | 0 | 0 | 0 | 0 | 0 | 0 | 0 |
| 3002972 | Yes | 0 | 0 | 0 | 0 | 0 | 0 | 0 | 10 | 14 |
| 3002630 | Yes | 0 | 0 | 0 | 0 | 0 | 0 | 0 | 15 | 0 |
| 3000508 | Yes | 0 | 0 | 0 | 21 | 0 | 0 | 0 | 0 | 0 |
| 3002882 | Yes | 0 | 0 | 0 | 0 | 0 | 16 | 0 | 0 | 0 |
| 3002957 | Yes | 0 | 0 | 0 | 0 | 0 | 0 | 0 | 10 | 14 |
| 3001214 | Yes | 0 | 0 | 0 | 0 | 0 | 0 | 0 | 0 | 0 |
| 3002909 | Yes | 0 | 0 | 0 | 0 | 0 | 0 | 0 | 0 | 0 |
| 3003112 | Yes | 0 | 0 | 0 | 0 | 0 | 0 | 0 | 0 | 0 |
| 3002881 | Yes | 0 | 0 | 0 | 0 | 0 | 0 | 0 | 0 | 0 |
| 3000792 | Yes | 10 | 0 | 0 | 0 | 0 | 0 | 0 | 0 | 0 |
| 3000186 | Yes | 0 | 0 | 0 | 0 | 0 | 0 | 0 | 0 | 0 |
| 3000801 | Yes | 0 | 0 | 0 | 0 | 0 | 0 | 0 | 0 | 0 |
| 3003052 | Yes | 0 | 0 | 0 | 0 | 0 | 0 | 0 | 0 | 0 |
| 3002629 | Yes | 0 | 0 | 0 | 0 | 0 | 0 | 0 | 0 | 0 |
| **ARO** | **Baits** | **Set 2 - 9** | **Set 2 - 3** | **Set 2 - 2** | **Set 2 - 6** | **Set 2 - 5** | **Set 2 -1** | **Set 2 - 4** | **Set 2 - 8** | **Set 2 - 7** |
| 3000190 | Yes | 3478 | 4231 | 4417 | 6684 | 6400 | 5670 | 5324 | 6567 | 4717 |
| 3000191 | Yes | 22674 | 32260 | 29099 | 46576 | 50381 | 46810 | 31557 | 36461 | 28754 |
| 3000196 | Yes | 6678 | 8515 | 8709 | 12551 | 12546 | 11884 | 9807 | 11034 | 9021 |
| 3000567 | Yes | 5956 | 7154 | 6153 | 10967 | 9134 | 7174 | 7321 | 9325 | 7143 |
| 3002837 | Yes | 2443 | 3407 | 3560 | 4857 | 5135 | 5372 | 3895 | 4083 | 3376 |
| 3002867 | Yes | 1286 | 1855 | 2000 | 2435 | 2620 | 3186 | 2469 | 2272 | 1916 |
| 3002999 | Yes | 2970 | 3701 | 3263 | 5479 | 4788 | 4264 | 3771 | 4510 | 3451 |
| 3002926 | Yes | 52 | 44 | 17 | 74 | 63 | 56 | 41 | 78 | 43 |
| 3000194 | No | 0 | 0 | 0 | 0 | 0 | 0 | 0 | 0 | 0 |
| 3000375 | No | 0 | 0 | 0 | 0 | 0 | 0 | 0 | 0 | 0 |
| 3000501 | No | 0 | 0 | 0 | 0 | 0 | 0 | 0 | 0 | 0 |
| 3002522 | Yes | 0 | 0 | 0 | 0 | 0 | 0 | 0 | 0 | 0 |
| 3002597 | No | 0 | 0 | 0 | 0 | 0 | 0 | 0 | 0 | 0 |
| 3003318 | No | 0 | 0 | 0 | 0 | 0 | 0 | 0 | 0 | 0 |
| 3003730 | No | 0 | 0 | 0 | 0 | 0 | 0 | 0 | 0 | 0 |
| 3004454 | No | 0 | 0 | 0 | 0 | 0 | 0 | 0 | 0 | 0 |
| 3002965 | Yes | 86 | 121 | 91 | 193 | 184 | 120 | 109 | 178 | 135 |
| 3000535 | Yes | 106 | 84 | 62 | 167 | 95 | 66 | 65 | 96 | 75 |
| 3002647 | No | 0 | 0 | 0 | 0 | 0 | 0 | 0 | 0 | 0 |
| 3000556 | Yes | 115 | 172 | 200 | 252 | 283 | 279 | 271 | 277 | 210 |
| 3003056 | Yes | 0 | 18 | 0 | 22 | 15 | 0 | 0 | 16 | 0 |
| 3002937 | Yes | 0 | 0 | 0 | 0 | 0 | 0 | 0 | 0 | 0 |
| 3002983 | No | 0 | 0 | 0 | 0 | 0 | 0 | 0 | 0 | 0 |
| 3003559 | Yes | 0 | 0 | 0 | 0 | 0 | 0 | 0 | 0 | 0 |
| 3004032 | No | 0 | 0 | 0 | 0 | 0 | 0 | 0 | 0 | 0 |
| 3004033 | No | 0 | 0 | 0 | 0 | 0 | 0 | 0 | 0 | 0 |
| 3004074 | No | 0 | 0 | 0 | 0 | 0 | 0 | 0 | 0 | 0 |
| 3004144 | No | 0 | 0 | 0 | 0 | 0 | 0 | 0 | 0 | 0 |
| 3000502 | Yes | 229 | 310 | 209 | 466 | 377 | 290 | 218 | 407 | 221 |
| 3000793 | No | 0 | 0 | 0 | 0 | 0 | 0 | 0 | 0 | 0 |
| 3000794 | No | 0 | 0 | 0 | 0 | 0 | 0 | 0 | 0 | 0 |
| 3003097 | Yes | 0 | 0 | 0 | 0 | 0 | 0 | 0 | 0 | 0 |
| 3000027 | Yes | 94 | 119 | 98 | 139 | 123 | 91 | 98 | 149 | 121 |
| 3000237 | Yes | 75 | 100 | 102 | 186 | 142 | 113 | 127 | 158 | 69 |
| 3000491 | Yes | 186 | 217 | 170 | 398 | 281 | 111 | 180 | 337 | 192 |
| 3000615 | Yes | 82 | 75 | 92 | 134 | 168 | 100 | 107 | 114 | 118 |
| 3000616 | Yes | 93 | 60 | 80 | 163 | 166 | 168 | 139 | 162 | 162 |
| 3000795 | Yes | 127 | 176 | 127 | 293 | 271 | 148 | 199 | 228 | 134 |
| 3000796 | Yes | 208 | 267 | 163 | 455 | 292 | 190 | 254 | 350 | 181 |
| 3000830 | Yes | 135 | 147 | 112 | 290 | 215 | 132 | 96 | 215 | 140 |
| 3000833 | Yes | 43 | 62 | 88 | 120 | 130 | 126 | 76 | 116 | 53 |
| 3001216 | Yes | 52 | 69 | 74 | 137 | 86 | 63 | 62 | 67 | 43 |
| 3001328 | Yes | 49 | 98 | 51 | 100 | 44 | 77 | 60 | 105 | 66 |
| 3003549 | Yes | 129 | 197 | 164 | 290 | 262 | 119 | 145 | 241 | 162 |
| 3003550 | Yes | 135 | 140 | 116 | 252 | 266 | 136 | 102 | 192 | 154 |
| 3003576 | Yes | 121 | 121 | 109 | 234 | 171 | 139 | 111 | 208 | 91 |
| 3003578 | Yes | 89 | 128 | 82 | 182 | 151 | 77 | 89 | 151 | 95 |
| 3000074 | Yes | 86 | 80 | 49 | 151 | 88 | 76 | 50 | 127 | 70 |
| 3000499 | Yes | 90 | 107 | 76 | 178 | 102 | 151 | 82 | 149 | 82 |
| 3000518 | Yes | 36 | 80 | 35 | 90 | 48 | 54 | 47 | 54 | 29 |
| 3000656 | Yes | 67 | 52 | 52 | 101 | 83 | 62 | 44 | 93 | 78 |
| 3002635 | Yes | 50 | 43 | 69 | 84 | 102 | 97 | 138 | 90 | 93 |
| 3003548 | Yes | 43 | 76 | 47 | 105 | 85 | 41 | 57 | 81 | 25 |
| 3000254 | Yes | 28 | 11 | 23 | 71 | 31 | 49 | 33 | 61 | 44 |
| 3001329 | Yes | 42 | 47 | 44 | 97 | 94 | 28 | 74 | 113 | 44 |
| 3002986 | Yes | 40 | 48 | 30 | 70 | 65 | 15 | 44 | 51 | 42 |
| 3000216 | Yes | 45 | 40 | 28 | 61 | 36 | 17 | 22 | 34 | 22 |
| 3002688 | Yes | 22 | 39 | 42 | 63 | 57 | 69 | 39 | 40 | 36 |
| 3000195 | Yes | 25 | 27 | 31 | 30 | 48 | 55 | 40 | 24 | 28 |
| 3000300 | Yes | 17 | 15 | 28 | 28 | 24 | 30 | 37 | 23 | 35 |
| 3000676 | Yes | 50 | 37 | 20 | 56 | 56 | 37 | 27 | 62 | 41 |
| 3003070 | Yes | 12 | 27 | 16 | 32 | 26 | 12 | 24 | 39 | 22 |
| 3000180 | Yes | 19 | 17 | 31 | 43 | 21 | 30 | 46 | 52 | 28 |
| 3000593 | Yes | 11 | 13 | 11 | 33 | 34 | 29 | 25 | 16 | 18 |
| 3002626 | Yes | 14 | 17 | 14 | 29 | 25 | 18 | 26 | 27 | 28 |
| 3003069 | Yes | 26 | 19 | 16 | 40 | 50 | 29 | 28 | 37 | 20 |
| 3003206 | Yes | 20 | 30 | 25 | 34 | 27 | 53 | 28 | 42 | 30 |
| 3000206 | Yes | 29 | 29 | 24 | 39 | 25 | 22 | 35 | 34 | 32 |
| 3003551 | Yes | 22 | 28 | 26 | 31 | 16 | 29 | 34 | 39 | 44 |
| 3002923 | Yes | 13 | 19 | 12 | 42 | 25 | 28 | 14 | 36 | 15 |
| 3002944 | Yes | 0 | 18 | 37 | 37 | 30 | 17 | 32 | 26 | 23 |
| 3000005 | Yes | 0 | 26 | 16 | 29 | 28 | 44 | 17 | 19 | 26 |
| 3000522 | Yes | 0 | 22 | 23 | 16 | 15 | 27 | 27 | 21 | 19 |
| 3000263 | Yes | 14 | 17 | 10 | 28 | 16 | 20 | 20 | 22 | 0 |
| 3000832 | Yes | 10 | 0 | 12 | 23 | 15 | 12 | 13 | 26 | 17 |
| 3002972 | Yes | 12 | 13 | 0 | 24 | 26 | 12 | 14 | 13 | 19 |
| 3002630 | Yes | 0 | 11 | 0 | 10 | 0 | 13 | 22 | 16 | 0 |
| 3000508 | Yes | 16 | 0 | 12 | 16 | 17 | 0 | 19 | 0 | 11 |
| 3002882 | Yes | 0 | 0 | 28 | 0 | 0 | 13 | 14 | 0 | 12 |
| 3002957 | Yes | 0 | 0 | 12 | 10 | 10 | 0 | 0 | 11 | 0 |
| 3001214 | Yes | 10 | 0 | 0 | 15 | 0 | 16 | 11 | 16 | 0 |
| 3002909 | Yes | 0 | 0 | 0 | 0 | 15 | 14 | 16 | 12 | 0 |
| 3003112 | Yes | 0 | 0 | 0 | 17 | 12 | 0 | 18 | 15 | 14 |
| 3002881 | Yes | 0 | 0 | 0 | 0 | 0 | 0 | 0 | 12 | 17 |
| 3000792 | Yes | 0 | 0 | 0 | 0 | 0 | 19 | 0 | 0 | 0 |
| 3000186 | Yes | 0 | 0 | 0 | 0 | 0 | 0 | 0 | 11 | 0 |
| 3000801 | Yes | 0 | 0 | 0 | 16 | 0 | 0 | 0 | 0 | 0 |
| 3003052 | Yes | 0 | 0 | 0 | 0 | 0 | 0 | 0 | 0 | 0 |
| 3002629 | Yes | 0 | 0 | 0 | 0 | 0 | 0 | 0 | 0 | 0 |
| **ARO** | **Baits** | **Set 3 - 9** | **Set3 - 6** | **Set 3 - 8** | **Set 3 - 7** | **Set 3 - 5** | **Set 3 - 3** | **Set 3 - 2** | **Set 3 - 4** | **Set 3 - 1** |
| 3000190 | Yes | 4389 | 3143 | 4035 | 4083 | 3662 | 3459 | 5115 | 3742 | 4278 |
| 3000191 | Yes | 31961 | 25807 | 27902 | 30217 | 30537 | 31375 | 57377 | 35805 | 38948 |
| 3000196 | Yes | 8770 | 7045 | 8207 | 8497 | 8055 | 7484 | 12627 | 9006 | 9549 |
| 3000567 | Yes | 7844 | 5526 | 7038 | 6490 | 6893 | 5856 | 7884 | 5888 | 5971 |
| 3002837 | Yes | 3591 | 2944 | 3308 | 3659 | 3351 | 3360 | 6483 | 4322 | 4901 |
| 3002867 | Yes | 1624 | 1429 | 1733 | 2133 | 1746 | 1579 | 3276 | 2464 | 2945 |
| 3002999 | Yes | 4441 | 3146 | 4007 | 4244 | 3884 | 3509 | 5435 | 3914 | 3948 |
| 3002926 | Yes | 49 | 50 | 29 | 19 | 29 | 45 | 21 | 18 | 21 |
| 3000194 | No | 0 | 0 | 0 | 0 | 0 | 0 | 0 | 0 | 0 |
| 3000375 | No | 0 | 0 | 0 | 0 | 0 | 0 | 0 | 0 | 0 |
| 3000501 | No | 0 | 0 | 0 | 0 | 0 | 0 | 0 | 0 | 0 |
| 3002522 | Yes | 0 | 0 | 0 | 0 | 0 | 0 | 0 | 0 | 0 |
| 3002597 | No | 0 | 0 | 0 | 0 | 0 | 0 | 0 | 0 | 0 |
| 3003318 | No | 0 | 0 | 0 | 0 | 0 | 0 | 0 | 0 | 0 |
| 3003730 | No | 0 | 0 | 0 | 0 | 0 | 0 | 0 | 0 | 0 |
| 3004454 | No | 0 | 0 | 0 | 0 | 0 | 0 | 0 | 0 | 0 |
| 3002965 | Yes | 109 | 78 | 107 | 89 | 76 | 80 | 94 | 70 | 107 |
| 3000535 | Yes | 92 | 71 | 82 | 77 | 63 | 87 | 0 | 51 | 51 |
| 3002647 | No | 0 | 0 | 0 | 0 | 0 | 0 | 0 | 0 | 0 |
| 3000556 | Yes | 145 | 110 | 130 | 117 | 119 | 112 | 216 | 170 | 211 |
| 3003056 | Yes | 0 | 0 | 0 | 0 | 20 | 0 | 0 | 0 | 0 |
| 3002937 | Yes | 0 | 0 | 0 | 0 | 0 | 0 | 0 | 0 | 0 |
| 3002983 | No | 0 | 0 | 0 | 0 | 0 | 0 | 0 | 0 | 0 |
| 3003559 | Yes | 0 | 0 | 0 | 0 | 0 | 0 | 0 | 0 | 0 |
| 3004032 | No | 0 | 0 | 0 | 0 | 0 | 0 | 0 | 0 | 0 |
| 3004033 | No | 0 | 0 | 0 | 0 | 0 | 0 | 0 | 0 | 0 |
| 3004074 | No | 0 | 0 | 0 | 0 | 0 | 0 | 0 | 0 | 0 |
| 3004144 | No | 0 | 0 | 0 | 0 | 0 | 0 | 0 | 0 | 0 |
| 3000502 | Yes | 219 | 188 | 188 | 171 | 199 | 166 | 192 | 154 | 155 |
| 3000793 | No | 0 | 0 | 0 | 0 | 0 | 0 | 0 | 0 | 0 |
| 3000794 | No | 0 | 0 | 0 | 0 | 0 | 0 | 0 | 0 | 0 |
| 3003097 | Yes | 0 | 0 | 0 | 0 | 0 | 0 | 0 | 0 | 0 |
| 3000027 | Yes | 72 | 67 | 70 | 80 | 80 | 53 | 87 | 55 | 94 |
| 3000237 | Yes | 81 | 59 | 60 | 90 | 109 | 64 | 74 | 75 | 83 |
| 3000491 | Yes | 145 | 161 | 182 | 152 | 254 | 150 | 165 | 134 | 126 |
| 3000615 | Yes | 79 | 57 | 73 | 56 | 54 | 56 | 70 | 84 | 58 |
| 3000616 | Yes | 90 | 61 | 75 | 98 | 63 | 63 | 165 | 119 | 114 |
| 3000795 | Yes | 139 | 64 | 118 | 83 | 127 | 110 | 114 | 110 | 93 |
| 3000796 | Yes | 247 | 155 | 156 | 149 | 172 | 113 | 159 | 159 | 134 |
| 3000830 | Yes | 142 | 101 | 91 | 94 | 133 | 131 | 108 | 90 | 85 |
| 3000833 | Yes | 36 | 47 | 47 | 59 | 64 | 28 | 66 | 68 | 61 |
| 3001216 | Yes | 72 | 31 | 38 | 39 | 34 | 37 | 56 | 47 | 45 |
| 3001328 | Yes | 40 | 47 | 46 | 54 | 48 | 42 | 46 | 43 | 50 |
| 3003549 | Yes | 124 | 88 | 124 | 107 | 132 | 126 | 127 | 101 | 104 |
| 3003550 | Yes | 138 | 103 | 154 | 110 | 128 | 115 | 134 | 96 | 71 |
| 3003576 | Yes | 125 | 87 | 75 | 76 | 113 | 79 | 84 | 107 | 107 |
| 3003578 | Yes | 96 | 64 | 67 | 75 | 81 | 47 | 87 | 56 | 48 |
| 3000074 | Yes | 37 | 53 | 44 | 76 | 55 | 63 | 65 | 47 | 62 |
| 3000499 | Yes | 91 | 75 | 88 | 66 | 78 | 43 | 73 | 80 | 65 |
| 3000518 | Yes | 44 | 17 | 45 | 27 | 23 | 26 | 39 | 23 | 16 |
| 3000656 | Yes | 33 | 32 | 54 | 29 | 32 | 18 | 68 | 38 | 40 |
| 3002635 | Yes | 71 | 51 | 57 | 43 | 39 | 52 | 98 | 77 | 63 |
| 3003548 | Yes | 26 | 36 | 39 | 23 | 37 | 34 | 34 | 22 | 33 |
| 3000254 | Yes | 33 | 0 | 30 | 20 | 23 | 18 | 32 | 55 | 29 |
| 3001329 | Yes | 31 | 44 | 32 | 45 | 45 | 38 | 40 | 15 | 27 |
| 3002986 | Yes | 42 | 49 | 29 | 41 | 36 | 15 | 20 | 28 | 33 |
| 3000216 | Yes | 28 | 15 | 34 | 17 | 29 | 23 | 24 | 26 | 10 |
| 3002688 | Yes | 36 | 15 | 18 | 22 | 25 | 28 | 45 | 33 | 44 |
| 3000195 | Yes | 23 | 17 | 23 | 25 | 29 | 30 | 16 | 20 | 39 |
| 3000300 | Yes | 0 | 0 | 18 | 11 | 18 | 10 | 17 | 15 | 25 |
| 3000676 | Yes | 41 | 31 | 19 | 17 | 30 | 37 | 35 | 30 | 27 |
| 3003070 | Yes | 16 | 13 | 12 | 11 | 18 | 19 | 54 | 21 | 25 |
| 3000180 | Yes | 25 | 14 | 28 | 31 | 33 | 0 | 36 | 45 | 48 |
| 3000593 | Yes | 0 | 15 | 15 | 21 | 10 | 13 | 28 | 15 | 26 |
| 3002626 | Yes | 19 | 26 | 14 | 15 | 13 | 15 | 23 | 19 | 12 |
| 3003069 | Yes | 23 | 15 | 25 | 10 | 24 | 20 | 11 | 13 | 20 |
| 3003206 | Yes | 0 | 21 | 13 | 29 | 26 | 16 | 46 | 17 | 22 |
| 3000206 | Yes | 15 | 13 | 10 | 0 | 15 | 16 | 12 | 20 | 25 |
| 3003551 | Yes | 16 | 29 | 32 | 22 | 0 | 13 | 20 | 20 | 35 |
| 3002923 | Yes | 20 | 16 | 0 | 11 | 14 | 13 | 18 | 11 | 23 |
| 3002944 | Yes | 15 | 22 | 0 | 37 | 14 | 19 | 32 | 11 | 32 |
| 3000005 | Yes | 14 | 0 | 19 | 20 | 19 | 0 | 15 | 13 | 14 |
| 3000522 | Yes | 0 | 0 | 18 | 0 | 0 | 13 | 31 | 10 | 0 |
| 3000263 | Yes | 17 | 0 | 0 | 0 | 0 | 0 | 14 | 12 | 12 |
| 3000832 | Yes | 0 | 11 | 0 | 12 | 0 | 13 | 23 | 0 | 16 |
| 3002972 | Yes | 0 | 0 | 12 | 0 | 0 | 12 | 0 | 20 | 11 |
| 3002630 | Yes | 0 | 11 | 16 | 0 | 11 | 0 | 34 | 26 | 11 |
| 3000508 | Yes | 0 | 0 | 0 | 13 | 13 | 0 | 15 | 12 | 0 |
| 3002882 | Yes | 19 | 0 | 0 | 0 | 0 | 10 | 18 | 14 | 12 |
| 3002957 | Yes | 0 | 19 | 0 | 0 | 12 | 0 | 12 | 0 | 13 |
| 3001214 | Yes | 0 | 0 | 10 | 0 | 10 | 0 | 0 | 0 | 0 |
| 3002909 | Yes | 12 | 0 | 0 | 0 | 0 | 0 | 14 | 0 | 0 |
| 3003112 | Yes | 0 | 0 | 12 | 0 | 0 | 0 | 0 | 0 | 0 |
| 3002881 | Yes | 11 | 0 | 0 | 14 | 0 | 0 | 0 | 0 | 0 |
| 3000792 | Yes | 0 | 0 | 0 | 0 | 0 | 0 | 0 | 10 | 0 |
| 3000186 | Yes | 0 | 0 | 0 | 0 | 0 | 0 | 0 | 13 | 0 |
| 3000801 | Yes | 0 | 0 | 0 | 0 | 17 | 0 | 0 | 0 | 0 |
| 3003052 | Yes | 0 | 32 | 0 | 0 | 0 | 38 | 0 | 0 | 0 |
| 3002629 | Yes | 0 | 0 | 0 | 0 | 0 | 0 | 10 | 0 | 0 |

**Supplementary Table 13: Genes identified through metagenomic analysis of enriched and shotgun samples. Combining raw read counts across all 27 enriched and 6 shotgun sample at the full number of genes with the breakdown of gene, class and mechanisms identified. Genes were filtered based on genes with at least 10 reads mapping, percent coverage greater than or equal to 10%, mapping quality greater than or equal to 11 and probes mapping (only for the enriched samples). This table is split into 4 parts with each part corresponding to a group of samples (Set 1, Set 2, Set 3 and the Shotgun samples). The first two columns are the same in all four parts.**

| **ARO** | **Set 1 - 6 Shotgun** | **Set 1 - 7 Shotgun** | **Set 2 - 9 Shotgun** | **Set 2 - 5 Shotgun** | **Set 3 - 5 Shotgun** | **Set 3 - 1 Shotgun** |
|---|---|---|---|---|---|---|
| 3000190 | 127 | 146 | 296 | 281 | 179 | 211 |
| 3000191 | 654 | 774 | 1568 | 1314 | 790 | 1150 |
| 3000196 | 116 | 151 | 238 | 221 | 133 | 227 |
| 3000567 | 44 | 59 | 96 | 90 | 66 | 72 |
| 3002837 | 94 | 114 | 208 | 174 | 84 | 152 |
| 3002867 | 32 | 32 | 86 | 50 | 38 | 48 |
| 3002999 | 46 | 50 | 60 | 66 | 44 | 76 |
| 3002926 | 10 | 22 | 30 | 28 | 16 | 24 |
| 3000194 | 546 | 635 | 1108 | 836 | 649 | 862 |
| 3000375 | 36 | 34 | 74 | 70 | 34 | 46 |
| 3000501 | 86 | 120 | 136 | 94 | 96 | 108 |
| 3002522 | 12 | 14 | 14 | 24 | 22 | 16 |
| 3002597 | 30 | 44 | 80 | 78 | 46 | 56 |
| 3003318 | 96 | 108 | 178 | 148 | 110 | 124 |
| 3003730 | 50 | 74 | 98 | 68 | 60 | 82 |
| 3004454 | 14 | 16 | 22 | 26 | 10 | 22 |
| 3002965 | 14 | 0 | 28 | 24 | 14 | 0 |
| 3000535 | 0 | 12 | 16 | 28 | 0 | 18 |
| 3002647 | 0 | 12 | 0 | 10 | 0 | 10 |
| 3000556 | 0 | 0 | 10 | 12 | 0 | 0 |
| 3003056 | 0 | 12 | 12 | 0 | 0 | 0 |
| 3002937 | 0 | 10 | 16 | 0 | 0 | 0 |
| 3002983 | 0 | 0 | 0 | 0 | 10 | 10 |
| 3003559 | 0 | 0 | 12 | 0 | 10 | 0 |
| 3004032 | 0 | 0 | 10 | 0 | 0 | 16 |
| 3004033 | 0 | 0 | 14 | 10 | 0 | 0 |
| 3004074 | 0 | 0 | 0 | 15 | 0 | 18 |
| 3004144 | 16 | 0 | 26 | 0 | 0 | 0 |
| 3000502 | 0 | 0 | 13 | 0 | 0 | 0 |
| 3000793 | 0 | 0 | 0 | 15 | 0 | 0 |
| 3000794 | 0 | 0 | 10 | 0 | 0 | 0 |
| 3003097 | 0 | 0 | 0 | 0 | 0 | 43 |
| 3000027 | 0 | 0 | 0 | 0 | 0 | 0 |
| 3000237 | 0 | 0 | 0 | 0 | 0 | 0 |
| 3000491 | 0 | 0 | 0 | 0 | 0 | 0 |
| 3000615 | 0 | 0 | 0 | 0 | 0 | 0 |
| 3000616 | 0 | 0 | 0 | 0 | 0 | 0 |
| 3000795 | 0 | 0 | 0 | 0 | 0 | 0 |
| 3000796 | 0 | 0 | 0 | 0 | 0 | 0 |
| 3000830 | 0 | 0 | 0 | 0 | 0 | 0 |
| 3000833 | 0 | 0 | 0 | 0 | 0 | 0 |
| 3001216 | 0 | 0 | 0 | 0 | 0 | 0 |
| 3001328 | 0 | 0 | 0 | 0 | 0 | 0 |
| 3003549 | 0 | 0 | 0 | 0 | 0 | 0 |
| 3003550 | 0 | 0 | 0 | 0 | 0 | 0 |
| 3003576 | 0 | 0 | 0 | 0 | 0 | 0 |
| 3003578 | 0 | 0 | 0 | 0 | 0 | 0 |
| 3000074 | 0 | 0 | 0 | 0 | 0 | 0 |
| 3000499 | 0 | 0 | 0 | 0 | 0 | 0 |
| 3000518 | 0 | 0 | 0 | 0 | 0 | 0 |
| 3000656 | 0 | 0 | 0 | 0 | 0 | 0 |
| 3002635 | 0 | 0 | 0 | 0 | 0 | 0 |
| 3003548 | 0 | 0 | 0 | 0 | 0 | 0 |
| 3000254 | 0 | 0 | 0 | 0 | 0 | 0 |
| 3001329 | 0 | 0 | 0 | 0 | 0 | 0 |
| 3002986 | 0 | 0 | 0 | 0 | 0 | 0 |
| 3000216 | 0 | 0 | 0 | 0 | 0 | 0 |
| 3002688 | 0 | 0 | 0 | 0 | 0 | 0 |
| 3000195 | 0 | 0 | 0 | 0 | 0 | 0 |
| 3000300 | 0 | 0 | 0 | 0 | 0 | 0 |
| 3000676 | 0 | 0 | 0 | 0 | 0 | 0 |
| 3003070 | 0 | 0 | 0 | 0 | 0 | 0 |
| 3000180 | 0 | 0 | 0 | 0 | 0 | 0 |
| 3000593 | 0 | 0 | 0 | 0 | 0 | 0 |
| 3002626 | 0 | 0 | 0 | 0 | 0 | 0 |
| 3003069 | 0 | 0 | 0 | 0 | 0 | 0 |
| 3003206 | 0 | 0 | 0 | 0 | 0 | 0 |
| 3000206 | 0 | 0 | 0 | 0 | 0 | 0 |
| 3003551 | 0 | 0 | 0 | 0 | 0 | 0 |
| 3002923 | 0 | 0 | 0 | 0 | 0 | 0 |
| 3002944 | 0 | 0 | 0 | 0 | 0 | 0 |
| 3000005 | 0 | 0 | 0 | 0 | 0 | 0 |
| 3000522 | 0 | 0 | 0 | 0 | 0 | 0 |
| 3000263 | 0 | 0 | 0 | 0 | 0 | 0 |
| 3000832 | 0 | 0 | 0 | 0 | 0 | 0 |
| 3002972 | 0 | 0 | 0 | 0 | 0 | 0 |
| 3002630 | 0 | 0 | 0 | 0 | 0 | 0 |
| 3000508 | 0 | 0 | 0 | 0 | 0 | 0 |
| 3002882 | 0 | 0 | 0 | 0 | 0 | 0 |
| 3002957 | 0 | 0 | 0 | 0 | 0 | 0 |
| 3001214 | 0 | 0 | 0 | 0 | 0 | 0 |
| 3002909 | 0 | 0 | 0 | 0 | 0 | 0 |
| 3003112 | 0 | 0 | 0 | 0 | 0 | 0 |
| 3002881 | 0 | 0 | 0 | 0 | 0 | 0 |
| 3000792 | 0 | 0 | 0 | 0 | 0 | 0 |
| 3000186 | 0 | 0 | 0 | 0 | 0 | 0 |
| 3000801 | 0 | 0 | 0 | 0 | 0 | 0 |
| 3003052 | 0 | 0 | 0 | 0 | 0 | 0 |
| 3002629 | 0 | 0 | 0 | 0 | 0 | 0 |

When combined, the enriched libraries cluster separately from the shotgun libraries with a stronger correlation (0.9957 compared to 0.8712 for the shotgun libraries; Figure 6).

Supplementary Table 14 compares genes with reads for shotgun and enriched stool library pairs. The full number of reads from shotgun and enriched pairs were mapped to CARD using *rgi* bwt. Results samples were filtered for gene with at least 10 reads, those probes mapping (only for the enriched samples), average read mapping quality >=11 and average read length coverage >=10%. Filtered genes and their normalized read counts (RPM) from each enriched/shotgun pair were combined to compare and determine the fold-enrichment.

| | Probes (ng) | Library (ng) | Fold- difference in reads (enriched vs shotgun) | Genes found in shotgun | Genes found in enriched | Genes overlapping | Genes with probes missing in enriched | Fold-enrichment (min - max) |
|---|---|---|---|---|---|---|---|---|
| | 200 | 100 | 389.70 | 18 | 49 | 9 | 1 | 1054.92 |
| Set 1 | | | | | | | | (0 - 10905.8) |
| | 100 | 200 | 82.24 | 20 | 25 | 7 | 5 | 1171.32 |
| | | | | | | | | (0 - 6459.8) |
| | 400 | 200 | 154.93 | 27 | 55 | 12 | 4 | 879.87 |
| Set 2 | | | | | | | | (0 - 9612.1) |
| | 100 | 100 | 80.73 | 23 | 61 | 11 | 1 | 868.16 |
| | | | | | | | | (0 - 8193.3) |
| | 100 | 100 | 66.67 | 19 | 57 | 9 | 2 | 732.16 |
| Set 3 | | | | | | | | (0 - 6962.7) |
| | 25 | 50 | 88.26 | 22 | 58 | 9 | 2 | 690.19 |
| | | | | | | | | (0 - 7319.6) |

The overlap was then compared between all 27 enriched samples and the six shotgun-sequenced libraries and included genes found through shotgun without any probes mapping. There were found a total of 89 genes with at least 10 reads between all libraries of which, 13 are overlapping between methods, 57 are unique to the enriched libraries, and 19 are unique to the shotgun libraries (part (B) of Figure 5; Supplementary Table 13). In part (B) of Figure 5, on the left, overlap of genes found with at least 10 reads, a percent coverage greater than or equal to 10% and an average mapping quality of reads greater than or equal to 11 in the 27 enriched and 6 shotgun samples. Between all samples, enriched or shotgun sequenced, there were 89 genes with reads passing these filters; 13 overlap, 57 are unique to the enriched, and 19 are unique to the shotgun samples. On the right, of the 19 genes only identified through shotgun sequencing, only 4 of these genes are predicted to be targeted by probes.

Of the 19 genes not found in any enriched library, only 4 are predicted to be targeted by probes, while the remaining were not in CARD when the probes were initially designed (8) or had probes that were removed during design and filtering (7). Of the four genes with predicted probes, *cfxA6* is present in all enriched samples but was filtered out by mapping quality; *vanSG* is only present in 2/6 shotgun samples at less than 20% gene length coverage by reads; *cepA* is found in enriched samples but at less than 10 reads; finally, there were identified *novA* in all shotgun samples but in only a few enriched samples at less than 10 reads and less than 10% read length coverage. Despite the few genes that are missing from the enriched samples, even with over 200-fold more sequencing depth, shotgun sequencing did not provide the same resolution as enrichment.

### Analysis

### Considerations in probe design

Increased interest in targeted capture approaches has resulted in the design of probesets for the detection of viruses, bacteria, and more recently, antibiotic resistance elements (Depledge *et al*., 2011; Allicock *et al*., 2018; Lanza *et al*., 2018; Noyes *et al*., 2017). Although this study is not the first to employ targeted capture for antibiotic resistance genes, focus was placed on a rigorous probe design, reduced input library and probe concentrations, and robust validation to produce a cost-effective alternative to shotgun sequencing. Finally, there are many considerations when designing a probeset including choosing an appropriate reference database and how the probe sequences are determined (Mercer *et al*., 2014; Metsky *et al.,* 2019; Enk *et al*., 2014; Phillippy, 2009; Douglas *et al.,* 2018).

In ancient genomic studies, many samples yield negligible, if any, endogenous DNA molecules to analyse often requiring extensive pre-screening (Pääbo *et al*., 2004, Damgaard *et al*., 2015). In many samples, the target sequences represent <1% of the total DNA or may be inherently difficult to extract (i.e. *Mycobacterium tuberculosis* from direct clinical samples for sequencing) and in many cases the sample itself (eg., blood, stool, soil) contains inhibitors of downstream steps in library generation (Votintseva *et al*., 2017; Rantakokko-Jalava, & Jalava, 2002; Schrader *et al*., 2012; Levy-Booth *et al*., 2007). Since microbial DNA and the target antibiotic resistance gene fragments can represent rare components in clinical and environmental samples, prior experience with ancient DNA samples guided experimental design. Given the random fragmentation that occurs through sonication and the nature of sequencing library preparation, it is difficult to predict the exact nature of all DNA molecules that will comprise the final library used in hybridization (in terms of number and length of antibiotic resistance element present on each fragment and the proportion of the library that contains resistance elements). As shown, even with a single DNA extract from an individual stool sample followed by multiple library preparations and sequencing on different days, the composition of antibiotic resistance elements recovered through shotgun sequencing of replicate libraries varies (only 50.00% of genes overlap between all samples). There was also observed some variability in enrichment with 34.28% of genes overlapping between the 27 libraries with 10 reads or more.

Others have suggested designing one probe per gene or tiling probes across a gene without overlap (1X coverage) (Noyes *et al.,* 2017). With BacCapSeq, over 4 million probes were designed to target protein-coding sequences from bacterial pathogens (including AMR from CARD and virulence factors) with an average 121-nucleotide distance between probes along their targets (Allicock *et al*., 2018). This inter-probe distance and random distribution of probes across sequences from various pathogens may reduce specificity for individual organisms and reduce on-target efficiency. Furthermore, while a well-designed probe per gene may reduce off-target sequencing, this approach risks falsely excluding genes if the specific DNA fragment targeted by that probe is not by chance included in the library or is in a very low concentration and thus simply missed due to selection and bias during DNA extraction and library preparation. In order to successfully identify a gene present in low concentration using a spaced probe tiling strategy, one may require multiple DNA extractions, library preparations, and enrichment reactions along with deeper sequencing. A tiling approach with dense and highly overlapping probes, similar to the probe design herein, increases the likelihood of capturing DNA molecules resulting in efficient enrichment and higher recovery but comes at the increased cost of production (Clark *et al*., 2011).

CARD was chosen as the reference database for the probe design and analysis due to its rigorous curation of antibiotic resistance determinants. The protein variant and protein overexpression model of the database was excluded as the genes included (*gyrA,* EF-Tu genes, efflux pump regulators, etc.) are likely to be found across many families of bacteria and were thought likely to overwhelm the probeset and sequencing effort with abundant, non-mutant antibiotic susceptible alleles. Instead, as the approach is focused on mobile genetic elements and acquired resistance genes that are often unique to individual families of bacteria, there was focus on CARD's protein homolog models targeting over 2000 antibiotic resistance genes. There was extensive filtering of candidate probes against the human genome, other eukaryote, archaeal, and weakly matching bacterial sequences to provide a probeset that is bacterial ARG specific and avoids off-target hybridization. Focusing on one highly curated database of antibiotic resistance determinants (CARD) increases the likelihood of capturing bona fide sequences that are associated with known resistance and reduces the overall cost of the probe set and sequencing effort. Noyes et al. (2017) increased the copy number of probes for large resistance genes families (beta-lactamases, etc.) where individual probes can target upwards of 200 genes, strategically increasing the concentration of those particular probes to promote equal affinity of each target gene in case there are multiple variants in a metagenome, yet the results suggest this is not necessary as enrichment did not bias the rank prevalence of AMR in the samples.

Other approaches targeting ARGs have additionally included species identifiers, plasmid markers and biocide or metal resistance (Lanza *et al*., 2018; Noyes *et al*., 2017; Allicock *et al.,* 2018). These probesets range in target capacity from 5557 genes (3.34 Mb) (Noyes *et al*., 2017) to over 78,600 genes (88.13 Mb) (Lanza *et al*., 2018) and comprise up to 4 million probes (Allicock *et al.,* 2018). The presently described approach is more conservative in probe design (1.77 Mb for 2021 genes), but this allows for more probes per gene (99.16% of genes with greater than 10 probes) and increased depth of probe coverage (9.47X average) which it is believed increases specificity and sensitivity. There was also a similar gene probe coverage to Lanza *et al.* with 97.47% of targeted genes having greater than 80% probe coverage where they have 90% of genes covered by at least 96.9% (Lanza *et al*., 2018). These alternative approaches also target a wide range of genes which can expand the amount of information obtained but increases the cost of synthesis and sequencing. As more information on environmental resistance mechanisms and new determinants emerge in resistomes, further additions to the probeset will need to be validated. In future benchmarking analysis experiments, such as those performed here, the probeset will need to be compared alongside other probe design approaches in order to inform the ideal design of a targeted-capture probeset for antibiotic resistance as has been done in other cases (Metsky *et al*., 2019; Ávila-Arcos, 2015).

### Experimental considerations in targeted capture methods

Additional metrics were assessed apart from probe design that can impact enrichment including library preparation method, input library amount, and probe to library ratio. The trials tested significantly lower inputs (25 ng to 400 ng) than recommended (up to 2 µg of DNA for metagenomic samples) setting this approach apart from other targeted capture methods of AMR genes (Noyes *et al*., 2017; Lanza *et al*., 2018). Others have looked at reducing the amount of input DNA from the recommended amount of 3000 ng to 500 ng and saw no significant differences in results (Shearer *et al*., 2012). Despite a 16-fold drop in DNA input (25 ng vs the recommended 2000 ng), there were observed no visible differences in the order of genes captured in the stool sample and normalized read counts were comparable among different library and probe amounts, suggesting that this approach is robust to substantial fluctuations yet still identifies substantially all antibiotic resistance genes in samples with low DNA yield. Thus, the enrichment is robust and amenable to different library preparation methods and DNA fragment sizes, despite what others have shown (Enk *et al*., 2014, Clark *et al*., 2011, Jones *et al.,* 2015, Ávila-Arcos, 2015).

### Standardization and controls in metagenomics

Many variables can affect the outcome of the sequencing results, including DNA extraction, library preparation, sequencing depth, enrichment methods and analysis. Factors influencing metagenome characterization include (but are not limited to) sample collection (Franzosa *et al*., 2014), DNA extraction (Mackenzie *et al*., 2015), choice of library preparation (Jones *et al.,* 2015), and excessive PCR amplification of indexed libraries (Probst *et al.,* 2015) and can lead to misinterpretation of data or loss of information, including variability in high GC sequences (Jones *et al.,* 2015). In comparative metagenomics, these variables make comparisons among samples difficult unless all methods are performed at the same time, using the same reagents and libraries sequenced to the same depth. It was attempted to reduce bias and assess enrichment by using the same DNA extract, library preparations, and enrichment in triplicate. Even among replicate libraries and shotgun sequencing runs, the differences in the number of genes identified at various sequence depths highlights the inherent variability in metagenomics (Figure 8)

Other attempts at standardization include using mock controls and spike-in controls which may allow for more accurate abundance calculations and account for variations in upstream methods (Pollock *et al*., 2018; Mercer *et al*., 2014; Jones *et al*., 2015; Eisenhofer *et al*., 2019). In the mock controls, a positive control (*E. coli* C0002) was included for enrichment to ensure the methodology and probes were performing optimally at the time of hybridization.

Advantageously, negative controls can be implemented to suppress false positives (Type I Error) during analysis. Referring to Figure 9, an illustrative method for suppressing false positives during analysis of sample biological materials is shown in pictorial form. The sample biological materials may be, for example, one or more of blood, urine, feces, tissue, lymph fluid, spinal fluid and sputum, and may come, for example from a vertebrate, such as a human being, a livestock animal such as a cow, pig, goat, horse, etc., or from a domestic companion animal, such as a cat, dog, ferret, etc., or from an invertebrate (e.g. shrimp, crab, prawn, lobster etc.). The sample biological materials may be from a living organism, a cadaver of a formerly living organism, or an archaeological sample. The sample biological materials may also be from at least one environmental sample, including, mud, soil, water, effluent (e.g. wastewater, sludge, sewage or the like), filter deposits and surface films.

The analysis comprises one or more handling steps, where the term "handling" includes initial collection of the sample biological materials, as well as transfer steps, for example from one carrier to another. For each handling step during the analysis, there is obtained at least one sample handling blank 902 carrying a transfer substrate 904 mixed with at least part of the sample biological material 906. The term "transfer substrate", as used in this context refers to a single reagent or a mixture of reagents, which may be mixed with water or another suitable substance. For example, buffers, reaction buffers, water, purification beads, or other reagents/solutions in the experiment, would be included within the meaning of "transfer substrate". The sample handling blank 902 is a reservoir or vehicle for the sample, and may be, for example, a test tube, a slide, or another suitable carrier. Additionally, for each handling step during the analysis, there is obtained at least one control blank 908 that will serve as a negative control. The control blank 908 corresponds to the sample handling blank 902 in that handling step, in that it is the same type of blank, preferably taken from the same batch of blanks (e.g. the same box of test tubes or slides) and carries the same transfer substrate 904 from same batch of transfer substrate (e.g. reagents from the same manufacturer and the same container). Importantly, the control blank 908 is isolated from the sample biological materials 906, as shown by the dashed box 910, so that the control blank 908 is not exposed to any of the sample biological materials 906. The control blank 908 is a "negative control" or a sample that is carried through the experiment without any addition of "biological materials" but including all other reagents. Any handling (e.g. agitation, centrifuge, light exposure, heating, cooling, etc.) applied to the sample handling blank(s) 902 is replicated for the control blank(s) 908 while isolation is maintained. Isolation, in this context, means that any cross-contamination of the sample biological material 906 onto the control blank 908 is avoided; isolation does not otherwise preclude side-by-side processing so as to enable identification of potential contaminants that enter the reaction from the surrounding environment. The control blank 908 is isolated from the sample biological materials 906 but not necessarily from the surrounding environment.

While Figure 9 shows only a single handling step 912, it will be appreciated that there may be additional handling steps. For example, there may be an initial a collection step during which the sample biological materials are collected on a sample handling blank, and then one or more transfer steps where the sample biological materials are transferred from a preceding sample handling blank to a subsequent sample handling blank. For example, part of a surface film may be scraped off a surface using a sterile scraper (a first sample handling blank) and then transferred to a test tube with reagent (a second sample handling blank). Each step performed with a sample handling blank is replicated with control blank. So, for example, a sterile scraper from the same batch as was used to scrape the surface film, but isolated therefrom (a first control blank) would be brought into contact with a sterile test tube from the same batch as that which received the film, containing reagent from the same batch, but isolated from the film (a second control blank).

Following completion of all handling steps, there will be at least one final sample handling blank 914 carrying an admixture 916 of the transfer substrate(s) 904 from the handling steps 912 mixed with the sample biological materials 906, and at least one final control blank 918 carrying the transfer substrate(s) 904 from the handling steps and isolated from the sample biological materials 906.

A hybridization probe solution 920 containing at least one hybridization probe is then applied to each final sample handling blank 914 to produce at least one baited final sample handling blank 922. The hybridization probe solution 920 comprises probes that hybridize to target DNA, which may be, for example AMR genes or other target DNA. The identical hybridization probe solution 920 is also applied to each final control blank 918, hybridization probe solution identical to that applied to each final sample handling blank to produce at least one baited final control blank 924. The terms "bait" and "baited" refer to a nucleotide probe that is complementary to a sequence of interest (target) and aimed at enriching that target through hybridization (complementarity of nucleotide base of target and bait/probe). The bait(s) may each be an oligonucleotide of 80 basepair lengths. All of the results above and the AMR gene enrichment are now published at https://doi.org/10.1128/AAC.01324-19.

Each baited final sample handling blank 922 is fed into a DNA sequencer 926, for example an Illumina DNA sequencer to sequence sample bait-captured DNA 928 carried by the baited final sample handling blank 922. Likewise, each baited final control blank 924 is also fed into the DNA sequencer 926 to sequence control bait-captured DNA 930 carried by the baited final control blank 924. The sample bait-captured DNA 928 is then compared 932 to the control bait-captured DNA 930 to generate a final identified genetic sequence 934. Genetic components that are common to the final sample handling blank 922 and the final control blank 904 and that pass a statistical significance test are discounted and excluded from the final identified genetic sequence 934. The statistical significance test may include, for example, deduplication, mapping quality and length cut-offs (i.e. percent length coverage and the average depth of coverage of each probe-targeted region), linear normalization based on total sequencing effort, rarefaction analysis, and comparison of total mapped read counts for different bait / sample ratios. In some embodiments, MAPQ statistical cut-offs will be used to exclude spurious alignment of DNA sequences to AMR reference sequences, i.e. bwa-mem MAPQ < 30, thus suppressing false positive results. In addition, measures of depth of read coverage and gene completeness may be used relative to AMR reference sequences, for example requiring alignment of at least 10 sequencing reads and at least 90% coverage of AMR reference sequences by mapped reads for prediction of an AMR gene for a specific sample. Lastly, detection under the above criteria of any AMR gene in a control/blank may be interpreted as laboratory contamination and that gene may be excluded from consideration in experimental samples.

Including a negative control/control blank provides an idea of background contamination that should be considered when using the bait method on experimental samples and analyzing the sequence data. For example, one could compare all samples processed to a control blank/negative control using linear normalized counts of sequencing reads based on total sequencing effort after deduplication. The reads may be mapped to a reference of probe-targeted regions. Similarities between the blank sample and experimental samples may be flagged to consider removing these results as contamination. If there is overlap between the targeted regions captured in a control blank and sample handling blank and that overlap represents ≥10% of the reads mapping to that probe-targeted region that region could be considered as a contaminant. Also, if reads from the control blank map to a probe-targeted region and in >80% of the samples processed there are also reads mapping to that same probe-targeted region it could be considered as contamination.

Thus, the present approach also introduced negative controls, including a blank DNA extraction and blank enrichment sample (water with reagents), to measure the extent of exogenous DNA contamination that is ubiquitous in all laboratory settings and reagents (Eisenhofer *et al.,* 2019; Salter *et al.,* 2014; Minich *et al.,* 2018). Only 0 - 13.93% of reads (post-enrichment) from the negative controls had the corresponding Illumina index sequence, the remainder having indexes from experimental samples, suggesting that DNA exchange among samples during enrichment or cross-contamination is the primary concern in the method (Supplementary Table 2; Supplementary Table 6). Notably, the genes identified in the Blank results not arising from cross-contamination and also found in the enriched and shotgun results are commonly associated with bacteria identified in negative controls in microbiome studies (mainly *Escherichia coli*) and encode efflux systems or other intrinsic resistance determinants (*mdtEFHOP, emrKY, cpxA, acrDEFS, pmrF, eptA, tolC).* The two genes that were unique to the Blank results (*drfa17* had 11 reads covering 85.86%; *aph(3*"*)-Ib* 16 reads with 57.46% coverage) are associated with mobile genetic elements in Enterobacteriaceae and the latter has been previously associated with laboratory reagent contamination (Sandalli *et al*., 2010; Wally *et al.,* 2019). Despite standard methods to control for contamination (i.e. filter pipettes, PCR cabinets, and sterile DNA/RNA-free consumables), there was still found to be limited contamination likely stemming from reagents and/or the surrounding laboratory environment, further highlighting the importance of negative controls in all targeted capture experiments and meticulous reporting and publishing of a laboratory based `resistome' (Supplementary Table 6; de Goffau *et al.,* 2018; Salter *et al*., 2014; Eisenhofer *et al*., 2019). The de Goffau *et al.* reference highlights the importance of reporting the reagent microbiome (contamination that is often found in reagents that are commonly used in all experiments) as in certain studies it can skew results and lead to false-positives. The Salter *et al.* reference reports frequent contamination in microbiome analyses and how studies should report results alongside `background' controls so that "erroneous conclusions are not drawn from culture-independent investigations". The Eisenhofer *et al.* reference is an opinion article highlighting criteria that should be reported on controls in microbiome research. However, although these references suggest reporting contamination or including controls, they do not suggest including blank controls as described in the present disclosure. Because enrichment/targeted capture is so sensitive to the less abundant targets which could include slight amounts of contamination it is very important to include blank controls and report these results alongside experimental results.

As can be seen from the above description, the methods described herein represent significantly more than merely using categories to organize, store and transmit information and organizing information through mathematical correlations. The methods are in fact an improvement to the technology of genetic analysis of sample biological materials, as they provide for suppression of false positives (Type I Error), which facilitates improved accuracy. Moreover, the methods are applied using physical steps carried out on physical blanks and by using a particular machine, namely a DNA sequencer. As such, the methods are confined to genetic analysis of sample biological materials and represent a technical improvement thereto.

### Analyzing enrichment data without a bacterial genome as reference

There are many available reference databases for mapping along with a variety of analytical tools (Arango-Argoty *et al.,* 2018; Asante *et al.,* 2019; Boolchandani *et al.,* 2019; Rowe and Winn, 2018; Berglund *et al.,* 2019; Hunt *et al.,* 2017; Inouye *et al.,* 2014). Similar to other targeted capture approaches for ARGs, Bowtie2 was used for mapping the sequencing reads against the reference database from which the probes were designed (Noyes *et al*., 2017; Lanza *et al*., 2018). One important factor with AMR genes is the sequence similarity between families and classes of antibiotic resistance determinants as well as with genes that do not necessarily confer resistance. The difficulty in separating uncharacterized determinants from known sequences has not been well-established. Previous attempts have used a percentage read coverage of genes filter or no filters when reporting resistance genes obtained through enrichment (Lanza *et al.,* 2018; Noyes *et al.,* 2017). Read count (1 vs 10 vs 100), read mapping quality, percent coverage by reads, and probe coverage of genes were assessed before reporting the presence or absence of resistance genes. In order to be able to make comparisons between the shotgun and enriched samples, reliance was placed on what are considered very permissive thresholds for the shotgun data (10% length coverage by reads and average read mapping quality of 11), which have not been rigorously evaluated for the correct identification or reporting of antibiotic resistance genes from metagenomic sequencing data. However, it is notable that the thresholds for the shotgun data were to obtain reasonable results at all.

Mapping quality (MAPQ) in Bowtie2 is related to the likelihood that an alignment represents the correct match of that read to the reference (Langmead and Salzberg, 2012). A mapping quality value of zero indicates that a read maps with low identity and/or that it maps to multiple locations (as the number of possible mapping locations increases the map quality decreases). In the case of the CARD reference database, there are many gene families (*bla*_{CTX-M}, *bla*_{TEM}, *bla*_{OXA}) that are very similar in nucleotide sequence identity and therefore a read belonging to one member has the potential to map to multiple genes. This feature results in an inflated number of genes with reads and consequently reduces the mapping quality for many reads. Lanza *et al.* describe this phenomenon as the mapping allele network (Lanza *et al*., 2018). The read mapping filter was kept high, with a cut-off of 41 (maximum MAPQ 41), when mapping to the respective genomes for each bacterial genome enrichment (Trial 1 and Trial 2). In the pooled mock metagenomic samples, because of the similarity between genes in two strains of the same species (i.e. Pool 3 contains two E. *coli* genomes - C0002, C0094), a mapping quality cut-off of 11 was used based on the distribution of read mapping quality. Consequently, a high mapping quality cut-off may result in inflated false-negative results, removing potential genes because the reads map to many members of AMR gene families.

The procedure included assessment and correction for duplicate removal and differences in sequencing depth. Removal of duplicates allows for more accurate assessment of fold enrichment and removes bias introduced via amplification (Metsky *et al*., 2019). The probeset is predicted to target 2021 genes from CARD, but in reality, the probes likely target many more divergent sequences. Others have shown that their probesets maintained up to 2-fold enrichment with sequences that were 70% similar to the target and that probes can be designed to tolerate up to 40 mismatches across a 120-nucleotide probe (Noyes *et al*., 2017; Metsky *et al.,* 2019). More extensive databases, including CARD's Resistome and Variants data which contains over 175,000 predicted AMR allele sequences (CARD R&V version 3.0.4), may provide additional information for variant and pathogen-of-origin identification.

### Enrichment in the gut microbiome

The enrichment of resistance genes in the human gut microbiome samples resulted in a higher average percentage on-target (50.69%) when compared to other published capture-based methods, 30.26 (20.27 - 41.83%) (Lanza *et al*., 2018), and a median of 15.8 (0.28% - 68.2%) (Noyes *et al.,* 2017). Overall, the probeset and method identified a greater diversity of antibiotic resistance genes in the human gut microbiome despite having been sequenced at 66 - 389-fold lower depth when compared to their shotgun sequenced correlate. Similar to other studies with probesets for AMR, there was found to be an average fold-enrichment of 690 - 1171 for enriched samples and an average of 96.67% of genes detected between each pair of enriched and shotgun samples were identified in the enriched library. There was identified an average of 79.76 % (58.3 - 91.67) of genes from the shotgun samples in their paired enriched library. Noyes *et al.* reported a higher overlap with genes detected by both shotgun and enrichment approaches (99.3%) and Lanza *et al.* showed a slightly lower overlap of 90.8%. Other research illustrates that enrichment maintains the frequency and rank order of genes when compared to shotgun results, similar to the enriched library results (Metsky *et al*., 2019). With a reduced depth of sequencing, it is evident that enrichment offers more valuable information in both the number of genes with reads as well as the depth and breadth of coverage of those genes (Figure 5). Only a few genes were absent in the enriched libraries when compared to the shotgun libraries. In the case of *novA,* which is 70.51% GC, perhaps the probeset or hybridization conditions were not sufficient to capture the genes by the method described herein. The variant of the *vanS* (36.7% GC) sensor from vancomycin resistance gene clusters that could not be identified was covered by less than 20 reads in the shotgun samples, suggesting a very low abundance in the metagenome. Finally, the beta-lactamase genes *cepA* and *cfxA6* had been excluded from the enriched results after filtering due to low mapping quality or less than 10 reads. The low mapping quality suggests that reads are mapping to other beta-lactamase genes in the reference database.

As enriched libraries only require a small proportion of a sequencing run, it was possible to sequence more libraries on a single run, which is much more cost-effective and time-efficient than deep shotgun sequencing. Although shotgun sequencing can provide additional information on other functions and genes of interest, targeted-capture provides a more robust, reproducible profile of a subset of genes from a metagenome at a fraction of the cost. Targeted capture provides many advantages to shotgun metagenomics when only a specific set of genes is in question across multiple samples.

### Conclusions

This study presents a focused ARG probe-capture method and analysis approach validated against pure bacterial genomes, mock metagenomic libraries, and the gut microbiota as represented by human stool. Rigorous measurement of the performance of the probe design and methods was conducted to satisfy many of the parameters routinely discussed in targeted capture (Mamanova *et al*., 2010). These metrics include sensitivity and specificity (consistently high percentage of reads on target and recovery of probe-targeted sequences), uniform recovery of ARGs across bacterial genomes, reproducibility between library preparations, reduced cost and reduced amounts of input DNA. The targeted capture is reproducible with individual DNA samples isolated from multidrug-resistant bacteria and increased the recovery of probe-targeted regions in mock metagenomes compared to shotgun sequencing, with an associated reduction in cost. It is also easily scalable, as newly discovered ARGs can be easily added to the probeset iteratively. With a small amount of DNA from a single stool sample, enrichment uncovers more information about the antibiotic resistance determinants in the gut microbiome at a significantly lower depth of sequencing when compared to the shotgun sequencing results from the same sample. This probeset provides a cost-effective and efficient approach to identify antibiotic resistance determinants in metagenomes allowing for a higher-throughput when compared to a shotgun sequencing approach. The method reveals the resistome from a variety of environments including the human gut microbiome, unearthing the realities of antibiotic resistance now ubiquituous in commensal and pathogenic milieu. The importance of suppressing false positives during analysis of sample biological materials is also emphasized.

### Methods

### Nucleotide probe design and filtering to prevent off-target hybridization

The reference for probe design was the protein homolog model of antibiotic resistance determinants (n = 2,129) from the Comprehensive Antibiotic Resistance Database (version 1.0.1 of CARD released December 14, 2015; Jia *et al.,* 2017). Using PanArray (v1.0), there were designed probes of 80 nucleotide length across all genes with a sliding window of 20 nucleotides and acceptance of 1 mismatch across probes (Phillippy, 2009). To prevent off-target hybridization between the probes and non-bacterial sequences, the candidate set of probe sequences (n = 38,980) was compared against the human reference genome and GenBank's non-redundant nucleotide database through BLAST (blastn) (Altschup *et al*., 1990; Benson *et al*., 2017). Probes with high sequence similarity (>80%) and probes with high-scoring segment pairs (HSPs) greater than 50 nucleotides of a possible 80 were discarded (n=158). The procedure identified and discarded 158 probes with human hits, 1617 probes with eukaryotic hits, 774 that were similar to viral references, and 30 that were similar to archaeal sequences. Probes with HSPs less than 50 nucleotides of a possible 80 to bacterial sequences were additionally discarded, resulting in a set of 32,066 probes. The candidate list was further filtered to omit probes that had bacterial HSPs that were <95% identity, resulting in a candidate list of 21,911 probes.

### Optimizing probe density and redundancy

Probe sequences, along with 1-100 nucleotide(s) upstream and downstream of the probe location on the target gene, were sent to Arbor Biosciences (Ann Arbor, MI) for probe design. Additional 80 nucleotide probes were created across the candidate probe and flanking sequences at four times tiling density, resulting in 226,440 probes. Sequences with 99% identity over 87.5% length were collapsed using USEARCH (usearch -cluster_fast - query_cov 0.875 -target_cov 0.875 -id 0.99 -centroids) resulting in a set of 37,826 final probes (Edgar, 2010). Filtering similar to as described above was performed against the human genome; no probes were found to be similar. Arbor Biosciences (Ann Arbor, MI) synthesized this final set of 37,826 80-nt biotinylated ssRNA probes through the custom myBaits kit.

### Probe assessment and predicted target genes

To predict the genes that can be targeted by the probes, a Bowtie2 (settings used: bowtie2 --end-to-end -N 1 '-L 32' -a) alignment was performed to compare the set of 37,826 probe sequences to the 2,238 nucleotide reference sequences of the protein homolog models in CARD (version 3.0.0 released 2018-10-11). Probes were mapped to all possible locations and the resulting alignment file was manipulated through samtools and bedtools to determine the number of instances that a probe mapped to a nucleotide sequence in CARD (samtools view -b, samtools sort, Langmead and Salzberg, 2012; Li *et al.,* 2009; Quinlan and Hall, 2010). The length coverage of each gene in CARD (i.e. fraction of the gene sequence with corresponding probes) was calculated (bedtools genomecov -ibam), and genes with zero coverage were determined (Quinlan and Hall, 2010). Furthermore, it was determined that the depth of coverage of each gene in CARD (i.e. the number of probes mapped to the gene) from the alignment (bedtools coverage -mean; Quinlan and Hall, 2010). The GC content of probe sequences and nucleotide sequences in CARD was calculated using a Python3 script from https://gist.github.com/wdecoster/8204dba7e504725e5bb249ca77bb2788. Melting temperature (Tₘ) was determined using OligoArray function melt.pl (-n RNA, -t 65 -C 1.89e⁻⁹) (Rouillard *et al.,* 2003). Finally, the mechanisms and drug classes of each resistance gene were determined using annotations found in CARD. Prism 8 for macOS (https://www.graphpad.com) was used to generate plots in Figures 1A to 1F.

### Bacterial strains, samples, and DNA extraction

Clinical bacterial isolates were obtained from the IIDR Clinical Isolate Collection which consists of strains from the core clinical laboratory at Hamilton Health Sciences Centre (Supplementary Table 1). Isolates were received from the clinical microbiology lab and grown on BHI plates at 37°C for 16 hours. A colony was inoculated into 5.5 mL LB and grown at 37°C with aeration for 16 hours, at which point genomic DNA was isolated using the Invitrogen Purelink Genomic DNA kit (Carlsbad, CA). If DNA was not isolated the same day, cell pellets were stored at -80°C. While genomic DNA from all other strains was only isolated once, DNA from a cell pellet of *Pseudomonas aeruginosa* C0060 was extracted additionally using the Invitrogen PureLink Genomic Kit (Carlsbad, CA) with a varied genomic lysis/binding buffer (30 mM EDTA, 30 mM Tris-HCl, 800 mM GuSCN, 5% Triton-X-100, 5% Tween-20, pH 8.0). The quantity of purified DNA was measured via absorbance (Thermo Fisher Nanodrop, Waltham, MA) and visualized for purity using agarose gel electrophoresis. A human stool sample was obtained from a healthy volunteer for the purpose of culturing the microbiome with consent (HiREB#5513-T). DNA was extracted the same day following a modified protocol as described in Whelan *et al.,* 2014. Briefly, samples were bead beat, centrifuged, and the supernatant further processed using the MagMax Express 96-Deep Well Magnetic Particle Processor from Applied Biosystems (Foster City, CA) with the multi-sample kit (Life Technologies #4413022). DNA was stored at -20°C until used for library preparation.

### Library preparation for isolate genome sequencing

Library preparation for genome sequencing of the clinical bacterial genomes was completed by the McMaster Genomics Facility in the Farncombe Institute at McMaster University (Hamilton, ON) using the New England Biolabs (Ipswich, MA) Nextera DNA library preparation kit. Libraries were sequenced using an Illumina HiSeq 1500 or Illumina MiSeq v3 platform using V2 (2 x 250 bp) chemistry. Paired sequencing reads were processed through Trimmomatic v0.39 to remove adaptors, checked for quality using FASTQC (http://www.bioinformatics.babraham.ac.uk/projects/fastqc/), and *de novo* assembled using SPAdes v 3.9.0 (Bolger *et al.,* 2014; Bankevich *et al.,* 2012). The Livermore Metagenomics Analysis Toolkit (LMAT) v 1.2.6 was used to identify the bacterial species and screen for contamination or mixed culture, while the Resistance Gene Identifier (RGI; version 4.2.2) from CARD was used on the SPAdes contigs to identify Perfect (100% match) and Strict (<100% match but within CARD similarity cut-offs) hits to CARD's curated antibiotic resistance genes (Ames *et al.,* 2013).

### Trials for enrichment

Two phases of experiments were performed, the first with genomic DNA from cultured multi-drug resistant bacteria (Phase 1) and the second with metagenomic DNA from a human stool sample (Phase 2). The two trials in Phase 1 differ in their library preparation methods as described below (the major difference being library fragment size by sonication). In both trials, genomic DNA from strains was tested individually (*Escherichia coli* C0002, *Pseudomonas aeruginosa* C0060, *Klebsiella pneumoniae* C0050, and *Staphylococcus aureus* C0018) (Supplementary Table 1 and 3). In addition, varying nanogram amounts (based on absorbance) of each genome were combined prior to library preparation to create "mock metagenomes" referred to as Pool 1 (C0002, C0018, C0050, C0060), Pool 2 (C0002, C0018, C0050, C0060), and Pool 3 (C0002, C0018, C0050, C0060, *Klebsiella pneumoniae* C0006, *Staphylococcus aureus* C0033, *Escherichia coli* C0094, *Pseudomonas aeruginosa* C0292). Amounts of each strain in each Pool varied between trials (Supplementary Table 4). Phase 2 consists of 3 replicates referred to as Set 1, Set 2, and Set 3 wherein DNA extract from one individual human stool sample was split evenly into each Set. From these aliquots, there were generated 9 individually indexed sequencing libraries and performed capture with varying library and probe ratios (Supplementary Table 3). In all trials and sets, a blank DNA extract was carried throughout library preparation and enrichment, while an additional negative reagent control was introduced during enrichment.

### Library preparation for enrichment sequencing

Library preparations were performed in a PCR clean hood, using bleached equipment, and UV-irradiated before use to prevent non-endogenous DNA contamination. Trial 1 used the NEBNext Ultra II DNA library preparation kit (New England Biolabs, Ipswich, MA) through the McMaster Genomics Facility. Based on absorbance and fluorometer values (QuantiFluor, Promega, Madison, WI), approximately 1 microgram of individual bacterial genomic DNA or pools of genomic DNA was sonicated to 600 base pairs (bp) and there were prepared dual-indexed libraries with a size selection for 500-600 bp inserts. A negative control consisting of a DNA extraction blank was included throughout the process. Post-library quality and quantity verification was performed using a High Sensitivity DNA Kit for the Agilent 2100 Bioanalyzer (Agilent Technologies, Santa Clara, CA) and quantitative PCR using the KAPA SYBR Fast qPCR master mix for Bio-Rad machines (Roche Canada) using primers for the distal ends of Illumina adapters and the following cycling conditions: 1) 95°C for 3 min; 2) 95°C for 10 sec; 3) 60°C for 30 sec; 5) Repeat 2-3 for 30 cycles total; 6) 60°C for 5 min 7) 8 °C hold. Illumina's PhiX control library (Illumina, San Diego, CA) was used as a standard for quantification. To increase the concentration of some libraries, samples were lyophilized and re-suspended in a smaller volume of nuclease-free water to provide approximately 100 nanograms of DNA for enrichment in an appropriate volume.

In Trial 2, the same genomic DNA, except for *P. aeruginosa* C0060 which was re-isolated, was used for library construction through a modified protocol (Supplementary material; Meyer and Kircher, 2010). Briefly, blunt end repair, adapter ligation, a library size-selection, and indexing PCR were performed on ~200 nanograms of sonicated DNA (250-300 bp) again including a negative control of a blank DNA extraction throughout the process. The McMaster Genomics Facility performed library quality control as described above.

### Library preparation from a human stool sample

One DNA extract from a donor stool sample was divided into three 50 µL aliquots of approximately 3150 nanograms each (based on fluorometer QuantiFluor results). DNA was sonicated to 600 bp and split into 9 individual library reactions (350 ng in 5.55 µL). Dual-indexes libraries (NEBNExt Ultra II library kits, New England Biolabs, Ipswich, MA) were prepared with a size-selection for 700-800 bp library fragments and 6 (Set 1), 7 (Set 2), or 8 cycles (Set 3) of amplification. The McMaster Genomics Facility performed library quality control (Agilent Bioanalyzer 2100 and quantitative PCR as described above). Positive control libraries were generated using *Escherichia coli* C0002 genomic DNA (40 ng of sonicated DNA) and a negative control with a blank DNA extract.

### Targeted capture of bacterial isolates

Enrichments were performed in a PCR clean hood, with a water bath, thermal cyclers and heat blocks located nearby. The probeset was provided by Arbor Biosciences (Ann Arbor, MI) and diluted with deionized water. For enrichment of bacterial genomes in Trial 1, there were used 100 ng of probes and 100 ng of each library following the MYBaits Manual V3 (Arbor Biosciences, Ann Arbor, MI) at a hybridization temperature of 65°C for 16 hours (see supplementary methods for more details). After hybridization and capture with Dynabeads MyOne Streptavidin C1 beads (Thermo Fisher, Waltham, MA), the resulting enriched library was amplified through 30 cycles of PCR (cycling conditions in Supplementary materials) using the KAPA HiFi HotStart polymerase with library non-specific primers (Kapa Library Amplification Primer Mix (10X), Sigma-Aldrich, St. Louis, MO). A 2 µL aliquot of this library was amplified in an additional PCR reaction for 3 cycles (same conditions as above) and then purified. The capture in Trial 2 was performed the same as Trial 1 but applied 17 cycles of amplification post-capture (PCR conditions in Supplementary details). The McMaster Genomics Facility performed library quality control as described above. Libraries were pooled in equimolar amounts and sequenced to an average of 94,117 clusters by MiSeq V2 (2x250 bp reads). Pre-enrichment libraries for the "mock metagenomes" were sequenced on a separate MiSeq V2 (2x250 bp reads) run from the enriched libraries to an average of 93,195 clusters each. From both Trial 1 and Trial 2, negative controls of blank extractions carried through library preparation and enrichment were sequenced on separate individual MiSeq 2 x 250 bp runs. After de-multiplexing, all possible index combinations were retrieved to identify potential cross-contamination of libraries as well as exogenous bacterial contamination.

### Targeted capture of the stool sample

Based on qPCR values and the average fragment sizes of each library generated from the human stool DNA extract, varying nanogram amounts of probes (25, 50, 100, 200, 400 ng) and library (50, 100, 200 ng) were combined for enrichment (Supplementary Table 2). Along with the Negative Control - Blank library, additional negative controls were introduced during enrichment using dH₂O to replace the volume normally required for library input. Capture probes were diluted with deionized and then prepared at the appropriate concentrations for each probe:library ratio. Enrichment was performed following the MYBaits Manual V4 (Arbor Biosciences, Ann Arbor, MI) at a hybridization temperature of 65°C for 24 hours. After hybridization and capture with Dynabeads (Thermo Fisher, Waltham, MA), the resulting enriched library was amplified through 14 cycles of PCR using the KAPA HiFi HotStart ReadyMix polymerase with library non-specific primers and the following conditions: 1) 98°C 45 sec; 2) 98°C 15 sec; 3) 60°C for 30 sec; 4) 72°C for 30 sec; 5) Repeat step 2 - 4 for 14 cycles total; 6) 72°C for 1 min; 7) 4°C hold (Sigma-Aldrich, St. Louis, MO). The resulting products were purified using KAPA Pure Beads at a 1X volume ratio and eluted in 10 mM Tris, pH 8.0. Purified libraries were quantified through qPCR using 10X SYBR Select Master Mix (Applied Biosystems, Foster City California) for BioRad Cfx machines, Illumina specific primers (10X primer mix from KAPA) and Illumina's PhiX Control Library as a standard. Cycling conditions were as follows: 1) 50 °C for 2 min; 2) 95 °C for 2 min; 3) 95 °C for 15 sec; 4) 60 °C for 30 sec; Repeat 3 - 4 for 40 cycles total. Enriched libraries were pooled in equimolar amounts based on qPCR values and the McMaster Metagenomic Sequencing facility performed library quality control as described above. Finally, the enriched libraries (average of 97,286 clusters) and the pre-enrichment libraries (average of 5,325,185 clusters) were sequenced by MiSeq V2 2x250 bp. The negative controls of blank extractions carried through library preparation and enrichment were sequenced on separate individual MiSeq 2 x 250 bp runs. After de-multiplexing, all possible index combinations were retrieved.

### Analysis of the bacterial isolates sequencing data

In order to identify probe-targeted regions and coordinates that overlap with predicted resistance genes based on RGI results for the individual bacterial strains, the probeset was aligned to the draft reference genome sequence using Bowtie2 version 2.3.4.1 (Langmead and Salzberg, 2012). *Skewer* version 0.2.2 (skewer -m pe -q 25 -Q 25) was used to trim sequencing reads (enriched or shotgun), bbmap version 37.93 dedupe2.sh to remove duplicates, and mapped reads to the bacterial genomes using Bowtie2 version 2.3.4.1 (--very-sensitive-local unique sites only) (Jiang *et al.,* 2014; https://sourceforge.net/projects/bbmap/; Langmead and Salzberg, 2012). Aligned reads were filtered based on mapping quality (>= 41) and length (>= 40 bp) using various tools: samtools version 1.4, bamtools version 2.4.1, and bedtools version 2.27.1 (Li *et al.,* 2009, Barnett *et al.,* 2011, Quinlan and Hall, 2010). It was determined that the number of reads mapping to the reference genome overall and the number of reads mapping within a predicted probe-targeted region using genomic coordinates and bedtools (intersectBed; Quinlan and Hall, 2010). The percent length coverage and the average depth of coverage of each probe-targeted region with at least one read was determined using bedtools coverage (-counts, -meant and default function) (Quinlan and Hall, 2010). Read counts were normalized by the number of reads mapping per kb of targeted region per total number of mapping reads to a particular genome. The number of genes with at least 1, 10 or at least 100 reads were counted and their percent length coverage by reads was determined.

### Analysis of stool sample sequencing data

The enriched and shotgun reads for the human stool sample were processed in the same way as for the bacterial isolates. Subsampling of reads was performed using seqtk version 1.2-r94 (seqtk sample -s100; https://github.com/lh3/seqtk). The *bwt* feature in RGI (beta of version 5.0.0; http://github.com/arpcard/rgi) was used to map trimmed reads using Bowtie2 version 2.3.4.1 to the CARD (version 3.0.0) generating alignments and results without any filters (Langmead and Salzberg, 2012). *The gene mapping* and *allele mapping* files were parsed to determine the number of genes in CARD with reads mapping (at least 1, at least 10, and at least 100 reads) under various filters. After plotting mapping quality for each read in every sample across the 3 sets, an average mapping quality *(mapq)* filter of 11 was chosen. A percent length coverage filter of a gene by reads of 10, 50 and 80% was assessed and the most permissive (10%) was chosen for comparison between the shotgun and enriched samples. Finally, a filter was used to check for the probes mapping to the reference sequences in most comparisons except to identify genes in the shotgun samples that would not be captured by the probeset. The same analysis process was repeated for the Negative Controls - Blank libraries after dividing the reads generated after enrichment among the index combinations used in the respective Phase, Trial or Set. In Set 1, there were very few reads associated with the Blank library after enrichment, so the raw sequencing reads were used for analysis. For the Negative Control in Set 2, deduplication was omitted, and the process could not identify any reads associated with the Blank indexes after sequencing for Set 3. Read counts were normalized using the *All Mapped Reads* column in the *gene mapping* file and the reference length in kb along with the total number of reads available for mapping (per million) (RPKM). Hierarchical clustering was performed using Gene Cluster 3.0 and Java Tree View v 1.1.6r4 (http://bonsai.hgc.jp/~mdehoon/software/cluster/software.htm) using a log transformation and clustering arrays with an uncentered correlation (Pearson) and average linkage. For rarefaction analysis, the procedure first aligned trimmed reads against CARD (version 3.0.0) using Bowtie2, followed by filtering for mapping quality >= 11 (Langmead and Salzberg, 2012). This file along with an annotation file for CARD was analyzed with the AmrPlusPlus Rarefaction Analyzer (http://megares.meglab.org/amrplusplus; Lakin *et al.,* 2016) with subsampling every 1% of total reads and a gene read length coverage of at least 10%. The average number of genes identified through after rarefaction was plotted and fit to a logarithmic curve to allow for simplified extrapolation. The heatmaps and figures were generated in Prism 8 for macOS (https://www.graphpad.com).

### Bibliography

The following references are cited herein, without any admission that any of them is relevant to the claimed invention or constitutes citable prior art:
- Allen HK, Donato J, Huimi Wang H, Cloud-Hansen KA, Davies J, Handelsman J. 2010. Call of the wild: antibiotic resistance genes in natural environments. Nature Reviews Microbiology 8: 251-259. https://doi.org/10.1038/nrmicro2312
- Allen HK, Moe LA, Rodbumrer J, Gaarder A, Handelsman J. 2009. Functional metagenomics reveals diverse β-lactamases in a remote Alaskan soil. The ISME Journal 386: 243-251. https://doi.org/10.1038/ismej.2008.86
- Allicock OM, Guo C, Uhlemann A, Whittier S, Chauhan LV, Garcia J, Price A, Morse SS, Mishra N, Briese T, Lipkin WI. 2018. BacCapSeq: a platform for diagnosis and characterization of bacterial infections. MBio 9: 1-10. https://doi.org/10.1128/mBio.02007-18
- Altschup SF, Gish W, Miller W, Myers EW, Lipman DJ. 1990. Basic Local Alignment Search Tool. J. Mol. Biol 215: 403-410.
- Ames SK, Hysom DA, Gardner SN, Lloyd GS, Gokhale MB, Allen JE. 2013. Scalable metagenomic taxonomy classification using a reference genome database. Bioinformatics 29(18): 2253-2260. https://doi.org/10.1093/bioinformatics/btt389
- Arango-Argoty G, Garner E, Pruden A, Heath LS, Vikesland P, Zhang L. 2018. DeepARG: a deep learning approach for predicting antibiotic resistance genes from metagenomic data. Microbiome 6:23. https://doi.org/10.1186/s40168-018-0401-z
- Asante J, Osei Sekyere J. 2019. Understanding antimicrobial discovery and resistance from a metagenomic and metatranscriptomic perspective: advances and applications. Environmental Microbiology Reports 00. https://doi.org/10.1111/1758-2229.12735
- Ávila-Arcos MC, Sandoval-Velasco M, Schroeder H, Carpenter ML, Malaspinas AS, Wales N, Peñaloza F, Bustamante CD, Gilbert, MTP. 2015. Comparative performance of two whole-genome capture methodologies on ancient DNA Illumina libraries. Methods in Ecology and Evolution 6(6), 725-734. https://doi.org/10.1111/2041-210X.12353
- Bankevich A, Nurk S, Antipov D, Gurevich AA, Dvorkin M, Kulikov AS, Lesin VM, Nikolenko SI, Pham S, Prjibelski AD et al. 2012. SPAdes: a new genome assembly algorithm and its applications to single-cell sequencing. Journal of Computational Biology 19(5): 455-77. https://doi.org/10.1089/cmb.2012.0021
- Barlow M, Hall BG. 2002. Predicting evolutionary potential: In vitro evolution accurately reproduces natural evolution of the TEM β-lactamase. Genetics 160(3): 823-832.
- Barnett DW, Garrison EK, Quinlan AR, Strömberg MP, Marth GT. 2011. BamTools: A C++ API and toolkit for analyzing and managing BAM files. Bioinformatics 27(12): 1691-1692. https://doi.org/10.1093/bioinformatics/btr174
- Berglund F, Osterlund T, Boulund F, Marathe NP, Larsson DGJ, Kristiansson E. 2019. Identification and reconstruction of novel antibiotic resistance genes from metagenomes. Microbiome 7(52). https://doi.org/10.1186/s40168-019-0670-1
- Bolger AM, Lohse M, Usadel B. 2014. Trimmomatic: A flexible trimmer for Illumina sequence data. Bioinformatics 30(15) : 2114-2120. https://doi.org/10.1093/bioinformatics/btu1702
- Boolchandani M, D'Souza AW, Dantas G. 2019. Sequencing-based methods and resources to study antimicrobial resistance. Nature Reviews Genetics. https://doi.org/10.1038/s41576-019-0108-4
- Boolchandani, M, Patel S, Dantas G. 2017. Functional metagenomics to study antibiotic resistance. Antibiotics: Methods and Protocols, Methods in Molecular Biology (Peter Sass (ed.)) 1520: 307-329, Springer Science+Business Media, New York, New York. https://doi.org/10.1007/978-1-4939-6634-9_19
- Brown ED, Wright GD. 2016. Antibacterial drug discovery in the resistance era. Nature 529: 336-343. doi:10.1038/nature17042
- Buelow E, Gonzalez TB, Versluis D, Oostdijk EAN, Ogilvie LA, van Mourik MSM, Oosterink E, van Passel MWJ, Smidt H, D'Andrea MM. 2014. Effects of selective digestive decontamination (SDD) on the gut resistome. Journal of Antimicrobial Chemotherapy 69(8): 2215-2223. https://doi.org/10.1093/jac/dku092
- Chafin TK, Douglas MR, Douglas ME. 2018. MrBait: universal identification and design of targeted-enrichment capture probes. Bioinformatics 34(24): 4293-4296. https://doi.org/10.1093/bioinformatics/bty548
- Clark K, Karsch-Mizrachi I, Lipman DJ, Ostell J, Sayers EW. 2017. GenBank. Nucleic Acids Research 45(D1): D37-D42. https://doi.org/10.1093/nar/gkw1070
- Clark MJ, Chen R, Lam HYK, Karczewski KJ, Chen R, Euskirchen G, Butte AJ, Snyder M. 2011. Performance comparison of exome DNA sequencing technologies. Nature Biotechnology 29(10): 908-914. https://doi.org/10.1038/nbt.1975
- Crofts TS, Gasparrini AJ, Dantas G. 2017. Next-generation approaches to understand and combat the antibiotic resistome. Nature Reviews Microbiology 15: 422-434. https://doi.org/10.1038/nrmicro.2017.28
- D'Costa VM, King CE, Kalan L, Morar M, Sung WWL, Schwarz C, Froese D, Zazula G, Calmels F, Debruyne R, et al. 2011. Antibiotic resistance is ancient. Nature 477(7365): 457-461. https://doi.org/10.1038/nature10388
- D'Costa VM, McGrann KM, Hughes DW, Wright GD. 2006. Sampling the antibiotic resistome. Science 311(5759): 374-377. https://doi.org/10.1126/science.1120800
- Damgaard PB, Margaryan A, Schroeder H, Orlando L, Willerslev E, Allentoft ME. 2015. Improving access to endogenous DNA in ancient bones and teeth. Scientific Reports 5:1184:1-12. https://doi.org/10.1038/srep11184
- Davies J, Davies D. 2010. Origins and evolution of antibiotic resistance. Microbiology and Molecular Biology Reviews 74(3): 417-433. https://doi.org/10.1128/MMBR.00016-10
- de Goffau MC, Lager S, Smith GCS, Salter SJ, Wagner J, Kronbichler A, Charnock-Jones DS, Peacock SJ, Smith GCS, Parkhill J. 2018. Recognizing the reagent microbiome. Nature Microbiology 3(8): 851-853. https://doi.org/10.1038/s41564-018-0202-y
- Depledge DP, Palser AL, Watson SJ, Yi-Chun Lai I, Gray ER, Grant P, Kanda RK, Leproust E, Kellam P, Breuer J. 2011. Specific capture and whole-genome sequencing of viruses from clinical samples. PLoS ONE 6(11):e27805. https://doi.org/10.1371/journal.pone.0027805
- Devault AM, Mortimer TD, Kitchen A, Kiesewetter H, Enk JM, Golding GB, Southon J, Kuch M, Duggan AT, Aylward W, et al. 2017. A molecular portrait of maternal sepsis from Byzantine Troy. ELife 6:e20983: 1-31. https://doi.org/10.7554/eLife.20983.001
- Duggan AT, Perdomo MF, Piombino-Mascali D, Marciniak S, Poinar D, Emery MV, Buchmann JP, Duchêne S, Jankauskas R, Humphreys M et al. 2016. 17th century variola virus reveals the recent history of smallpox. Current Biology 26: 3407-3412. https://doi.org/10.1016/j.cub.2016.10.061
- Edgar RC. 2010. Search and clustering orders of magnitude faster than BLAST. Bioinformatics 26(19): 2460-2461. https://doi.org/10.1093/bioinformatics/btq461
- Eisenhofer R, Minich JJ, Marotz C, Cooper A, Knight R, Weyrich LS. 2019. Contamination in low microbial biomass microbiome studies: Issues and recommendations. Trends in Microbiology 72(2): 105-117. https://doi.org/10.1016/j.tim.2018.11.003
- Enk JM, Devault AM, Kuch M, Murgha YE, Rouillard J.-M, Poinar HN. 2014. Ancient whole genome enrichment using baits built from modern DNA. Mol. Biol. Evol 31(5): 1292-1294. https://doi.org/10.1093/molbev/msu074
- Fitzpatrick D, Walsh F. 2016. Antibiotic resistance genes across a wide variety of metagenomes. FEMS Microbiology Ecology 92(2): 1-8. https://doi.org/10.1093/femsec/fiv168
- Forsberg KJ, Patel S, Gibson MK, Lauber CL, Knight R, Fierer N, Dantas G. 2014. Bacterial phylogeny structures soil resistomes across habitats. Nature 509: 612-616. https://doi.org/10.1038/nature13377
- Forsberg KJ, Reyes A, Wang B, Selleck EM, Sommer MOA, Dantas G. 2012. The shared antibiotic resistome of soil bacteria and human pathogens. Science 337: 1107-1111. https://doi.org/10.1126/science. 1220761
- Franzosa EA, Morgan XC, Segata N, Waldron L, Reyes J, Earl AM, Giannoukos G, Boylan MR, Ciulla D, Gevers D et al. 2014. Relating the metatranscriptome and metagenome of the human gut. Proceedings of the National Academy of Sciences 111(22): E2329-E2338. https://doi.org/10.1073/pnas.1319284111
- Gaze WH, Krone SM, Larsson DGJ, Li X-Z, Robinson JA, Simonet P, Smalla K, Timinouni M, Topp E, Wellington EM, et al. 2013. Influence of humans on evolution and mobilization of environmental antibiotic resistome. Emerging Infectious Disease Journal 19(7). https://doi.org/10.3201/eid1907.120871
- Gibson MK, Forsberg KJ, Dantas G. 2014. Improved annotation of antibiotic resistance determinants reveals microbial resistomes cluster by ecology. The ISME Journal. https://doi.org/10.1038/ismej.2014.106
- Gnirke A, Melnikov A, Maguire J, Rogov P, LeProust EM, Brockman W, Fennell T, Giannoukos G, Fisher S, Russ C, et al. 2009. Solution hybrid selection with ultra-long oligonucleotides for massively parallel targeted sequencing. Nature Biotechnology 27(2): 182-9. https://doi.org/10.1038/nbt.1523
- Hunt M, Mather AE, Sánchez-Busó L, Page AJ, Parkhill J, Keane JA, Harris SR. 2017. ARIBA: rapid antimicrobial resistance genotyping directly from sequencing reads. Microbial Genomics 3. https://doi.org/10.1099/mgen.0.000131
- Inouye M, Dashnow H, Raven L, Schultz MB, Pope BJ, Tomita T, Zobel J, Holt KE. 2014. SRST2: Rapid genomic surveillance for public health and hospital microbiology labs. Genome Medicine 6:90. https://doi.org/10.1186/s13073-014-0090-6
- Jia B, Raphenya AR, Alcock B, Waglechner N, Guo P, Tsang KK, Lago BA, Dave BM, Pereira S, Sharma AN, et al. 2017. CARD 2017: expansion and model-centric curation of the comprehensive antibiotic resistance database. Nucleic Acids Research 45: D566-D573. https://doi.org/10.1093/nar/gkw1004
- Jiang H, Lei R, Ding SW, Zhu S. 2014. Skewer: A fast and accurate adapter trimmer for next-generation sequencing paired-end reads. BMC Bioinformatics 15(1): 1-12. https://doi.org/10.1186/1471-2105-15-182
- Johnson TA, Stedtfeld RD, Wang Q, Cole JR, Hashsham SA, Looft T, Zhu Y. 2016. Clusters of antibiotic resistance genes enriched together stay together in swine agriculture. MBio 7(2): 1-11. https://doi.org/10.1128/mBio.02214-15
- Lakin SM, Dean C, Noyes NR, Dettenwanger A, Spencer Ross A, Doster E, Rovira P, Abdo Z, Jones KL, Belk KE et al. MEGARes: an antimicrobial resistance database for high throughput sequencing. 2016. Nucleic Acids Research 45:D574-D580
- Langmead B, Salzberg SL. 2012. Fast gapped-read alignment with Bowtie 2. Nature Methods 9(4): 357-359. https://doi.org/10.1038/nmeth.1923
- Lanza VF, Baquero F, Martinez JL, Ramos-Ruiz R, González-Zorn B, Andremont A, Sánchez-Valenzuela A, Ehrlich SD, Kennedy S, Ruppé E, et al. 2018. In-depth resistome analysis by targeted metagenomics. Microbiome 6(11). https: //doi.org/10.1186/s40168-017-03 87-y
- Lax S, Gilbert JA. 2015. Hospital-associated microbiota and implications for nosocomial infections. Trends in Molecular Medicine 21(7): 427-432. https://doi.org/10.1016/j.molmed.2015.03.005
- Laxminarayan R, Duse A, Wattal C, Zaidi AKM, Wertheim HFL, Sumpradit N, Vlieghe E, Hara GL, Gould IM, Goossens H, et al. 2013. Antibiotic resistance - the need for global solutions. Lancet Infect Dis 13:1057-98. https://doi.org/10.1016/S 1473-3099(13)70318-9
- Levy SB, Bonnie M. 2004. Antibacterial resistance worldwide: Causes, challenges and responses. Nature Medicine 10(12): S122-S129. https://doi.org/10.1038/nm1145
- Levy-Booth DJ, Campbell RG, Gulden RH, Hart MM, Powell JR, Klironomos JN, Pauls KP, Swanton CJ, Trevors JT, Dunfield KE. 2007. Cycling of extracellular DNA in the soil environment. Soil Biology and Biochemistry 39(12): 2977-2991. https://doi.org/https://doi.org/10.1016/j.soilbio.2007.06.020
- Li H, Handsaker B, Wysoker A, Fennell T, Ruan J, Homer N, Marth G, Abecasis G, Durbin R, 1000 Genome Project Data Processing Subgroup. 2009. The Sequence alignment/map format and SAMtools. Bioinformatics 25(16): 2078-2079. https://doi.org/10.1093/bioinformatics/btp352
- Luo Y, Yang F, Mathieu J, Mao D, Wang Q, Alvarez PJJ. 2013. Proliferation of multidrug-resistant New Delhi metallo-β-lactamase genes in municipal wastewater treatment plants in Northern China. Environ. Sci. Technol. Lett. 1: 26-30. https://doi.org/10.1021/ez400152
- Mackenzie BW, Waite DW, Taylor MW. 2015. Evaluating variation in human gut microbiota profiles due to DNA extraction method and inter-subject differences. Frontiers in Microbiology 6: 1-11. https://doi.org/10.3389/fmicb.2015.00130
- Mamanova L, Coffey AJ, Scott CE, Kozarewa I, Turner EH, Kumar A, Howard E, Shendure J, Turner DJ. 2010. Target-enrichment strategies for next-generation sequencing. Nature Methods 7(2): 111-118. https://doi.org/10.1038/NMETH.1419
- Mercer TR, Clark MB, Crawford J, Brunck ME, Gerhardt DJ, Taft RJ, Nielsen LK, Dinger ME, Mattick JS. 2014. Targeted sequencing for gene discovery and quantification using RNA CaptureSeq. Nature Protocols 9(5): 989-1009. https://doi.org/10.1038/nprot.2014.058
- Mercer TR, Gerhardt DJ, Dinger ME, Crawford J, Trapnell C, Jeddeloh JA, Mattick JS, Rinn JL. 2011. Targeted RNA sequencing reveals the deep complexity of the human transcriptome. Nature Biotechnology 30(1): 99-106. https://doi.org/10.1038/nbt.2024
- Metsky HC, Siddle KJ, Gladden-Young A, Qu J, Yang DK, Brehio P, Goldfarb A, Piantadosi A, Wohl S, Carter A, et al. 2019. Capturing sequence diversity in metagenomes with comprehensive and scalable probe design. Nature Biotechnology 37(2): 160-168. https://doi.org/10.1038/s41587-018-0006-x
- Meyer M, Kircher M. 2010. Illumina sequencing library preparation for highly multiplexed target capture and sequencing. Cold Spring Harbor Protocols 5(6). https://doi.org/10.1101/pdb.prot5448
- Mezger A, Gullberg E, Göransson J, Zorzet A, Herthnek D, Tano E, Nilsson M, Andersson DI. 2015. A General method for rapid determination of antibiotic susceptibility and species in bacterial infections. Journal of Clinical Microbiology 53(2): 425-432. https://doi.org/10.1128/JCM.02434-14
- Nesme J, C6cillon S, Delmont TO, Monier JM, Vogel TM, Simonet P. 2014. Large-scale metagenomic-based study of antibiotic resistance in the environment. Current Biology 24(10): 1096-1100. https://doi.org/10.1016/j.cub.2014.03.036
- Noyes NR, Weinroth ME, Parker JK, Dean CJ, Lakin SM, Raymond RA, Rovira P, Doster E, Abdo Z, Martin JN, et al. 2017. Enrichment allows identification of diverse, rare elements in metagenomic resistome-virulome sequencing. Microbiome 5:142. https://doi.org/10.1186/s40168-017-0361-8
- Pääbo S, Poinar H, Serre D, Jaenicke-Després V, Hebler, J, Rohland N, Kuch M, Krause J, Vigilant L, Hofreiter M. 2004. Genetic analyses from ancient DNA. Annu. Rev. Genet. 38:645-79. https://doi.org/10.1146/aanurev.genet.37.10801.143214
- Pal C, Bengtsson-Palme J, Kristiansson E, Larsson DGJ. 2016. The structure and diversity of human, animal and environmental resistomes. Microbiome 4(54): 1-15. https://doi.org/10.1186/s40168-016-0199-5
- Patterson Ross Z, Klunk J, Fornaciari G, Giuffra V, Duchêne S, Duggan AT, Poinar D, Douglas MW, Eden J-S, Holmes EC et al. 2018. The paradox of HBV evolution as revealed from a 16th century mummy. PLOS Pathogens 14(1): e1006750. https://doi.org/10.1371/journal.ppat.1006750
- Perry J, Waglechner N, Wright G. 2016. The prehistory of antibiotic resistance. Cold Spring Harb Perspect Med 6: 1-8. https://doi.org/10.1101/cshperspect.a025197
- Phillippy AM. 2009. Efficient oligonucleotide probe selection for pan-genomic tiling arrays. BMC Bioinformatics 10: 293-303. https://doi.org/10.1186/1471-2105-10-293
- Probst AJ, Weinmaier T, DeSantis TZ, Santo Domingo JW, Ashbolt N. 2015. New perspectives on microbial community distortion after whole-genome amplification. PLoS ONE 10(5): 1-16. https://doi.org/10.1371/journal.pone.0124158
- Pulido MR, Garcia-Quintanilla M, Martín-Peña R, Cisneros JM, McConnell MJ. 2013. Progress on the development of rapid methods for antimicrobial susceptibility testing. J Antimicrob Chemother 68(12): 2710-2717. https://doi.org/10.1093/jac/dkt253
- Quinlan AR, Hall IM. 2010. BEDTools: A flexible suite of utilities for comparing genomic features. Bioinformatics 26(6): 841-842. https://doi.org/10.1093/bioinformatics/btq033
- Rantakokko-Jalava K, Jalava J. 2002. Optimal DNA isolation method for detection of bacteria in clinical specimens by broad-range PCR. Journal of Clinical Microbiology 40(11): 4211-4217. https://doi.org/10.1128/JCM.40.11.4211
- Rouillard JM, Zuker M, Gulari E. 2003. OligoArray 2.0: Design of oligonucleotide probes for DNA microarrays using a thermodynamic approach. Nucleic Acids Research 31(12): 3057-3062. https://doi.org/10.1093/nar/gkg426
- Rowe WPM, Winn MD. 2018. Indexed variation graphs for efficient and accurate resistome profiling. Bioinformatics 34(21): 3601-3608. https://doi.org/10.1093/bioinformatics/bty387
- Salter SJ, Cox MJ, Turek EM, Calus ST, Cookson WO, Moffatt MF, Turner P, Parkhill J, Loman NJ, Walker AW. 2014. Reagent and laboratory contamination can critically impact sequence-based microbiome analyses. BMC Biology 12(87): 1741-7007. https://doi.org/10.1186/s12915-014-0087-z
- Sandalli C, Kurtulus Buruk C, Sancaktar M, Birol Ozgumus O. 2010. Prevalence of integrons and a new dfra17 variant in Gram-negative bacilli which cause community-acquired infections. Microbio Immunol 54: 164-169. https://doi.org/j.1348-0421.2009.00197.x
- Schrader C, Schielke A, Ellerbroek L, Johne R. 2012. PCR inhibitors - occurrence, properties and removal. Journal of Applied Microbiology 113(5): 1014-1026. https://doi.org/10.1111/j.1365-2672.2012.05384.x
- Schwartz KL, Morris SK. 2018. Travel and the spread of drug-resistant bacteria. Current Infectious Disease Reports 20(9):29. https://doi.org/10.1007/s11908-018-0634-9
- Silver LL. 2011. Challenges of antibacterial discovery. Clinical Microbiology Reviews 24(1): 71-109. https://doi.org/10.1128/CMR.00030-10
- Surette MD, Wright GD. 2017. Lessons from the environmental antibiotic resistome. Annual Review of Microbiology 71: 309-29.
- van Schaik W. 2014. The human gut resistome. Phil. Trans. R. Soc. B 370. https://doi.org/10.1098/rstb.2014.0087
- Votintseva AA, Bradley P, Pankhurst L, del Ojo Elias C, Loose M, Nilgiriwala K, Chatterjee A, Smith EG, Sanderson N, Walker TM, et al. 2017. Same-day diagnostic and surveillance data for tuberculosis via whole-genome sequencing of direct respiratory samples. J Clin Microbiol 55(5): 1285-1298. https://doi.org/10.1128/j cm.02483-16
- Wagner DM, Klunk J, Harbeck M, Devault A, Waglechner N, Sahl JW, Enk J, Birdsell DN, Kuch M, Lumibao C et al. 2014. Yersinia pestis and the Plague of Justinian 541-543 AD: a genomic analysis. Lancet Infect Dis 14: 319-26. https://doi.org/10.1016/S1473-3099(13)70323-2
- Wally N, Schneider M, Thannesberger J, Kastner MT, Bakonyi T, Indik S, Rattei T, Bedarf J, Hildebrand F, Law J, et al. 2019. Plasmid DNA contaminant in molecular reagents. Scientific Reports 9(1): 1-11. https://doi.org/10.1038/s41598-019-38733-1
- Walsh F, Duffy B. 2013. The culturable soil antibiotic resistome: a community of multi-drug resistant bacteria. PLoS ONE 8(6). https://doi.org/10.1371/journal.pone.0065567
- Whelan FJ, Verschoor CP, Steams JC, Rossi L, Luinstra K, Loeb M, Smieja M, Johnstone J, Surette MG, Bowdish DME. 2014. The Loss of topography in the microbial communities of the upper respiratory tract in the elderly. Ann Am Thorac Soc 11(4): 513-521. https://doi.org/10.1513/annalsats.201310-351oc
- Zumla A, Al-Tawfiq JA, Enne VI, Kidd M, Drosten C, Breuer J, Muller MA, Hui D, Maeurer M, Bates M, et al. 2014. Rapid point of care diagnostic tests for viral and bacterial respiratory tract infections - needs, advances, and future prospects. Lancet Infect Dis 14(11): 1123-1135. https://doi.org/10.1016/S1473-3099(14)70827-8

### Data Access

Raw sequencing reads (FASTQ) for IIDR Clinical Isolate Collection bacterial isolate genome assembly were deposited in NCBI BioProject PRJNA532924. All metagenomic sequencing results, enriched or shotgun, were deposited in NCBI BioProject PRJNA540073. The probeset sequences and annotations are available at the CARD website (http://card.mcmaster.ca).

One or more currently preferred embodiments have been described by way of example. It will be apparent to persons skilled in the art that a number of variations and modifications can be made without departing from the scope of the claims.

## Claims

1. A method for suppressing false positives (Type I Error) during analysis of sample biological materials, the method comprising:
for each of at least one handling step during the analysis:
obtaining at least one sample handling blank carrying a transfer substrate mixed with at least part of the sample biological materials;
obtaining at least one control blank that is isolated from the sample biological materials and corresponding to the sample handling blank in that handling step; and
replicating the handling applied to the at least one sample handling blank for the at least one control blank;
whereby, following completion of all handling steps, there is:
at least one final sample handling blank carrying the transfer substrates from the handling steps mixed with the at least part of the sample biological materials; and
at least one final control blank carrying the transfer substrates from the handling steps and isolated from the sample biological materials;
then:
applying a hybridization probe solution containing at least one hybridization probe to each final sample handling blank to produce at least one baited final sample handling blank; and
applying to each final control blank, hybridization probe solution identical to that applied to each final sample handling blank to produce at least one baited final control blank;
then:
feeding each baited final sample handling blank into a DNA sequencer and sequencing sample bait-captured DNA carried by the baited final sample handling blank; and
feeding each baited final control blank into the DNA sequencer and sequencing control bait-captured DNA carried by the baited final control blank;
then comparing the sample bait-captured DNA to the control bait-captured DNA and discounting, from a final identified genetic sequence, genetic components that:
are common to the final sample handling blank and the final control blank; and
pass a statistical significance test.

2. The method of claim 1, wherein the at least one handling step comprises a plurality of handling steps including:
a collection step during which the sample biological materials are collected; and
at least one transfer step where the sample biological materials are transferred from a preceding sample handling blank to a subsequent sample handling blank.

3. The method of claim 1, wherein the sample biological materials are from a vertebrate.

4. The method of claim 3, wherein the sample biological materials include at least one of blood, urine, feces, tissue, lymph fluid, spinal fluid and sputum.

5. The method of claim 1, wherein the sample biological materials are from at least one of a living organism, a cadaver of a formerly living organism, and an archaeological sample.

6. The method of claim 1, wherein the sample biological materials are from an invertebrate.

7. The method of claim 1, wherein the sample biological materials are from at least one environmental sample.

8. The method of claim 7, wherein the at least one environmental sample comprises at least one of mud, soil, water, effluent, filter deposits and surface films.

9. A method for suppressing false positives (Type I Error) during analysis of sample biological materials, the method comprising:
for at least one final sample handling blank carrying transfer substrate mixed with at least part of the sample biological materials:
applying a hybridization probe solution containing at least one hybridization probe to each final sample handling blank to produce at least one baited final sample handling blank; and
applying hybridization probe solution identical to that applied to each final sample handling blank to at least one final control blank, wherein the at least one final control blank carries transfer substrate identical to that applied to each sample handling blank and the at least one final control blank is isolated from the sample biological materials, to thereby produce at least one baited final control blank;
then:
feeding each baited final sample handling blank into a DNA sequencer and sequencing sample bait-captured DNA carried by the baited final sample handling blank; and
feeding each baited final control blank into the DNA sequencer and sequencing control bait-captured DNA carried by the baited final control blank;
then comparing the sample bait-captured DNA to the control bait-captured DNA and discounting, from a final identified genetic sequence, genetic components that:
are common to the final sample handling blank and the final control blank; and
pass a statistical significance test.

10. The method of claim 9, wherein the sample biological materials are from a vertebrate.

11. The method of claim 10, wherein the sample biological materials include at least one of blood, urine, feces, tissue, lymph fluid, spinal fluid and sputum.

12. The method of claim 9, wherein the sample biological materials are from at least one of a living organism, a cadaver of a formerly living organism, and an archaeological sample.

13. The method of claim 9, wherein the sample biological materials are from an invertebrate.

14. The method of claim 9, wherein the sample biological materials are from at least one environmental sample.

15. The method of claim 14, wherein the at least one environmental sample comprises at least one of mud, soil, water, effluent, filter deposits and surface films.

## Patentansprüche

1. Verfahren zum Unterdrücken Falschpositiver (Fehler 1. Art) während der Analyse von biologischem Probenmaterial, wobei das Verfahren umfasst:
für jeden von mindestens einem Handhabungsschritt während der Analyse:
Erhalten von mindestens einer Probenhandhabungsblindprobe, die ein Transfersubstrat trägt, gemischt mit mindestens einem Teil des biologischen Probenmaterials;
Erhalten von mindestens einer Kontrollblindprobe, die aus dem biologischen Probenmaterial isoliert wird und der Probenhandhabungsblindprobe in diesem Handhabungsschritt entspricht; und
Replizieren der Handhabung, die auf die mindestens eine Probenhandhabungsblindprobe angewendet wurde, für die mindestens eine Kontrollblindprobe;
wobei nach Abschluss aller Handhabungsschritte vorliegt:
mindestens eine endgültige Probenhandhabungsblindprobe, die die Transfersubstrate aus den Handhabungsschritten trägt, gemischt mit dem mindestens einen Teil des biologischen Probenmaterials; und
mindestens eine endgültige Kontrollblindprobe, die die Transfersubstrate aus den Handhabungsschritten trägt und aus dem biologischen Probenmaterial isoliert ist;
dann:
Aufbringen einer Hybridisierungssondenlösung, die mindestens eine Hybridisierungssonde enthält, auf jede endgültige Probenhandhabungsblindprobe, um mindestens eine mit Köder versehene endgültige Probenhandhabungsblindprobe herzustellen; und
Aufbringen einer Hybridisierungssondenlösung, die mit der auf jede endgültige Probenhandhabungsblindprobe aufgebrachten identisch ist, auf jede endgültige Kontrollblindprobe, um mindestens eine mit Köder versehene endgültige Kontrollblindprobe herzustellen;
dann:
Zuführen jeder mit Köder versehenen endgültigen Probenhandhabungsblindprobe in einen DNA-Sequenzierer und Sequenzieren einer ködergebundenen Proben-DNA, die von der mit Köder versehenen endgültigen Probenhandhabungsblindprobe getragen wird; und
Zuführen jeder mit Köder versehenen endgültigen Kontrollblindprobe in den DNA-Sequenzer und
Sequenzieren einer ködergebundenen Kontroll-DNA, die von der mit Köder versehenen endgültigen Kontrollblindprobe getragen wird;
dann Vergleichen der ködergebundenen Proben-DNA mit der ködergebundenen Kontroll-DNA und Herausrechnen aus einer endgültigen identifizierten genetischen Sequenz diejenigen genetischen Komponenten, die:
in der endgültigen Probenhandhabungsblindprobe und der endgültigen Kontrollblindprobe gleich sind; und
einen Test zur statistischen Signifikanz bestehen.

2. Verfahren nach Anspruch 1, wobei der mindestens eine Handhabungsschritt eine Vielzahl von Handhabungsschritten umfasst, einschließlich:
eines Sammelschritts, bei dem das biologische Probenmaterial gesammelt wird; und
mindestens einen Transferschritt, bei dem das biologische Probenmaterial von einer vorhergehenden Probenhandhabungsblindprobe auf eine nachfolgende Probenhandhabungsblindprobe übertragen wird.

3. Verfahren nach Anspruch 1, wobei das biologische Probenmaterial von einem Wirbeltier stammt.

4. Verfahren nach Anspruch 3, wobei das biologische Probenmaterial mindestens eines aus Blut, Urin, Fäzes, Gewebe, Lymphflüssigkeit, Rückenmarksflüssigkeit und Sputum umfasst.

5. Verfahren nach Anspruch 1, wobei das biologische Probenmaterial von mindestens einem von einem lebenden Organismus, dem Kadaver eines ehemals lebenden Organismus und einer archäologischen Probe stammt.

6. Verfahren nach Anspruch 1, wobei das biologische Probenmaterial von einem Wirbellosen stammt.

7. Verfahren nach Anspruch 1, wobei das biologische Probenmaterial aus mindestens einer Umweltprobe stammt.

8. Verfahren nach Anspruch 7, wobei die mindestens eine Umweltprobe mindestens eines von Schlamm, Erde, Wasser, Abwasser, Filterablagerungen und Oberflächenfilmen umfasst.

9. Verfahren zum Unterdrücken Falschpositiver (Fehler 1. Art) während der Analyse von biologischem Probenmaterial, wobei das Verfahren umfasst:
für mindestens eine endgültige Probenhandhabungsblindprobe, die ein Transfersubstrat trägt, gemischt mit mindestens einem Teil des biologischen Probenmaterials;
Aufbringen einer Hybridisierungssondenlösung, die mindestens eine Hybridisierungssonde enthält, auf jede endgültige Probenhandhabungsblindprobe, um mindestens eine mit Köder versehene endgültige Probenhandhabungsblindprobe herzustellen; und
Aufbringen einer Hybridisierungssondenlösung, die mit der auf jede endgültige Probenhandhabungsblindprobe aufgebrachten identisch ist, auf mindestens eine endgültige Kontrollblindprobe, wobei die mindestens eine endgültige Kontrollblindprobe ein Transfersubstrat trägt, das mit dem auf jede Probenhandhabungsblindprobe aufgebrachten identisch ist, und die mindestens eine endgültige Kontrollblindprobe wird von dem biologischen Probenmaterial isoliert, um dadurch mindestens einen mit Köder versehene endgültige Kontrollblindprobe herzustellen;
dann:
Zuführen jeder mit Köder versehenen endgültigen Probenhandhabungsblindprobe in einen DNA-Sequenzierer und Sequenzieren einer ködergebundenen Proben-DNA, die von der mit Köder versehenen endgültigen Probenhandhabungsblindprobe getragen wird; und
Zuführen jeder mit Köder versehenen endgültigen Kontrollblindprobe in den DNA-Sequenzer und Sequenzieren einer ködergebundenen Kontroll-DNA, die von der mit Köder versehenen endgültigen Kontrollblindprobe getragen wird;
dann Vergleichen der ködergebundenen Proben-DNA mit der ködergebundenen Kontroll-DNA und Herausrechnen aus einer endgültigen identifizierten genetischen Sequenz diejenigen genetischen Komponenten, die:
in der endgültigen Probenhandhabungsblindprobe und der endgültigen Kontrollblindprobe gleich sind; und
einen Test zur statistischen Signifikanz bestehen.

10. Verfahren nach Anspruch 9, wobei das biologische Probenmaterial von einem Wirbeltier stammt.

11. Verfahren nach Anspruch 10, wobei das biologische Probenmaterial mindestens eines aus Blut, Urin, Fäzes, Gewebe, Lymphflüssigkeit, Rückenmarksflüssigkeit und Sputum umfasst.

12. Verfahren nach Anspruch 9, wobei das biologische Probenmaterial von mindestens einem von einem lebenden Organismus, dem Kadaver eines ehemals lebenden Organismus und einer archäologischen Probe stammt.

13. Verfahren nach Anspruch 9, wobei das biologische Probenmaterial von einem Wirbellosen stammt.

14. Verfahren nach Anspruch 9, wobei das biologische Probenmaterial aus mindestens einer Umweltprobe stammt.

15. Verfahren nach Anspruch 14, wobei die mindestens eine Umweltprobe mindestens eines von Schlamm, Erde, Wasser, Abwasser, Filterablagerungen und Oberflächenfilmen umfasst.

## Revendications

1. Procédé de suppression de faux positifs (erreur de type 1) durant l'analyse de matières biologiques d'échantillon, le procédé comprenant les étapes consistant à :
pour chacune d'au moins une étape de manipulation durant l'analyse :
obtenir au moins un témoin de manipulation d'échantillon transportant un substrat de transfert mélangé avec au moins une partie des matières biologiques d'échantillon ;
obtenir au moins un témoin de contrôle qui est isolé des matières biologiques d'échantillon et correspondant au témoin de manipulation d'échantillon dans cette étape de manipulation ; et
répliquer la manipulation appliquée à l'au moins un témoin de manipulation d'échantillon pour l'au moins un témoin de contrôle ;
moyennant quoi, après la fin de toutes les étapes de manipulation, il y a :
au moins un témoin de manipulation d'échantillon final transportant les substrats de transfert des étapes de manipulation mélangés avec l'au moins une partie des matières biologiques d'échantillon ; et
au moins un témoin de contrôle final transportant les substrats de transfert des étapes de manipulation et isolé des matières biologiques d'échantillon ;
puis :
appliquer une solution de sonde d'hybridation contenant au moins une sonde d'hybridation à chaque témoin de manipulation d'échantillon final pour produire au moins un témoin de manipulation d'échantillon final appâté ; et
appliquer à chaque témoin de contrôle final, une solution de sonde d'hybridation identique à celle appliquée à chaque témoin de manipulation d'échantillon final pour produire au moins un témoin de contrôle final appâté ;
puis :
alimenter chaque témoin de manipulation d'échantillon final appâté dans un séquenceur d'ADN et séquencer l'ADN capturé par appât d'échantillon porté par le témoin de manipulation d'échantillon final appâté ; et
alimenter chaque témoin de contrôle final appâté dans le séquenceur d'ADN et séquencer l'ADN capturé par appât de contrôle porté par le témoin de contrôle final appâté ;
puis comparer l'ADN capturé par appât d'échantillon à l'ADN capturé par appât de contrôle et escomptant, à partir d'une séquence génétique identifiée finale, les composants génétiques qui :
sont communs au témoin de manipulation d'échantillon final et au témoin de contrôle final ; et
passer un test de signification statistique.

2. Procédé selon la revendication 1, dans lequel l'au moins une étape de manipulation comprend une pluralité d'étapes de manipulation comprenant :
une étape de collecte durant laquelle les matières biologiques d'échantillon sont collectées ; et
au moins une étape de transfert où les matériaux biologiques d'échantillon sont transférés d'un témoin de manipulation d'échantillon précédent à un témoin de manipulation d'échantillon subséquent.

3. Procédé selon la revendication 1, dans lequel les matières biologiques d'échantillon proviennent d'un vertébré.

4. Procédé selon la revendication 3, dans lequel les matières biologiques d'échantillons comprennent au moins un du sang, de l'urine, des fèces, d'un tissu, du fluide lymphatique, du fluide spinal et d'un crachat.

5. Procédé selon la revendication 1, dans lequel les matières biologiques d'échantillon sont d'au moins un d'un organisme vivant, d'un cadavre d'un organisme antérieurement vivant et d'un échantillon archéologique.

6. Procédé selon la revendication 1, dans lequel les matières biologiques d'échantillon proviennent d'un invertébré.

7. Procédé selon la revendication 1, dans lequel les matières biologiques d'échantillon proviennent d'au moins un échantillon environnemental.

8. Procédé selon la revendication 7, dans lequel l'au moins un échantillon environnemental comprend au moins un de la boue, du sol, de l'eau, d'un effluent, de dépôts de filtre et de films de surface.

9. Procédé de suppression de faux positifs (erreur de type 1) durant l'analyse de matières biologiques d'échantillon, le procédé comprenant les étapes consistant à :
pour au moins un témoin de manipulation d'échantillon final transporter le substrat de transfert mélangé avec au moins une partie des matières biologiques d'échantillon ;
appliquer une solution de sonde d'hybridation contenant au moins une sonde d'hybridation à chaque témoin de manipulation d'échantillon final pour produire au moins un témoin de manipulation d'échantillon final appâté ; et
appliquer la solution de sonde d'hybridation identique à celle appliquée à chaque témoin de manipulation d'échantillon final à au moins un témoin de contrôle final, l'au moins un témoin de contrôle final transportant le substrat de transfert identique à celui appliqué à chaque témoin de manipulation d'échantillon et l'au moins un témoin de contrôle final étant isolé des matières biologiques d'échantillon, pour ainsi produire au moins un témoin de contrôle final appâté ;
puis :
alimenter chaque témoin de manipulation d'échantillon final appâté dans un séquenceur d'ADN et séquencer l'ADN capturé par appât d'échantillon porté par le témoin de manipulation d'échantillon final appâté ; et
alimenter chaque témoin de contrôle final appâté dans le séquenceur d'ADN et séquencer l'ADN capturé par appât de contrôle porté par le témoin de contrôle final appâté ;
puis comparer l'ADN capturé par appât d'échantillon à l'ADN capturé par appât de contrôle et escomptant, à partir d'une séquence génétique identifiée finale, les composants génétiques qui :
sont communs au témoin de manipulation d'échantillon final et au témoin de contrôle final ; et
passer un test de signification statistique.

10. Procédé selon la revendication 9, dans lequel les matières biologiques d'échantillon proviennent d'un vertébré.

11. Procédé selon la revendication 10, dans lequel les matières biologiques d'échantillon comprennent au moins un du sang, de l'urine, des fèces, d'un tissu, du fluide lymphatique, du fluide spinal et d'un crachat.

12. Procédé selon la revendication 9, dans lequel les matières biologiques d'échantillon proviennent d'au moins un d'un organisme vivant, d'un cadavre d'un organisme antérieurement vivant et d'un échantillon archéologique.

13. Procédé selon la revendication 9, dans lequel les matières biologiques d'échantillon proviennent d'un invertébré.

14. Procédé selon la revendication 9, dans lequel les matières biologiques d'échantillon proviennent d'au moins un échantillon environnemental.

15. Procédé selon la revendication 14, dans lequel l'au moins un échantillon environnemental comprend au moins un de la boue, du sol, de l'eau, d'un effluent, de dépôts de filtre et de films de surface.
